# FASCICULE DE BREVET EUROPEEN

(11) **EP 1 544 296 B1**
(45) Date de publication et mention de la délivrance du brevet: **15.04.2009**
(21) Numéro de dépôt: 04292987.7
(22) Date de dépôt: 14.12.2004
(51) Int. Cl.: C12N 15/10, C12Q 1/68

(54) **Procédé de mutagenèse dirigée massive**
Verfahren zur ortspezifischen Massenmutagenese
Method for massive directed mutagenesis

(30) Priorité: 18.12.2003 FR 0314892
(43) Date de publication de la demande: 22.06.2005
(73) Titulaire: Biomethodes, 91000 Evry (FR)
(72) Inventeur: Sylvestre, Julien, 75002 Paris (FR); Delcourt, Marc, 75013 Paris (FR)
(74) Mandataire: Gallois, Valérie

(56) Documents cités:
- WO-A-02/16606
- WEILER J ET AL: "Combining the preparation of oligonucleotide arrays and synthesis of high-quality primers." ANALYTICAL BIOCHEMISTRY. UNITED STATES 15 DEC 1996, vol. 243, no. 2, 15 décembre 1996 (1996-12-15), pages 218-227, XP000684351 ISSN: 0003-2697
- BEIER MARKUS ET AL: "Analysis of DNA-microarrays produced by inverse in situ oligonucleotide synthesis." JOURNAL OF BIOTECHNOLOGY. NETHERLANDS 14 MAR 2002, vol. 94, no. 1, 14 mars 2002 (2002-03-14), pages 15-22, XP002281008 ISSN: 0168-1656
- LASHKARI D A ET AL: "An automated multiplex oligonucleotide synthesizer: development of high-throughput, low-cost DNA synthesis." PROCEEDINGS OF THE NATIONAL ACADEMY OF SCIENCES OF THE UNITED STATES OF AMERICA. UNITED STATES 15 AUG 1995, vol. 92, no. 17, 15 août 1995 (1995-08-15), pages 7912-7915, XP000611248 ISSN: 0027-8424
- HAAS S A ET AL: "Genome-scale design of PCR primers and long oligomers for DNA microarrays" NUCLEIC ACIDS RESEARCH, OXFORD UNIVERSITY PRESS, SURREY, GB, vol. 31, no. 19, 1 octobre 2003 (2003-10-01), pages 5576-5581, XP002260424 ISSN: 0305-1048
- RAMSAY G: "DNA CHIPS: STATE-OF-THE ART" NATURE BIOTECHNOLOGY, NATURE PUBLISHING, US, vol. 16, 1998, pages 40-44, XP000198575 ISSN: 1087-0156
- PON RICHARD T ET AL: "Tandem oligonucleotide synthesis on solid-phase supports for the production of multiple oligonucleotides." THE JOURNAL OF ORGANIC CHEMISTRY. UNITED STATES 8 FEB 2002, vol. 67, no. 3, 8 février 2002 (2002-02-08), pages 856-864, XP002281009 ISSN: 0022-3263

## Description

La présente invention concerne le domaine de la biologie moléculaire et plus particulièrement celui de la mutagenèse. Elle a pour objet un procédé de mutagenèse dirigée à haut débit, c'est-à-dire la constitution de nombreux mutants dirigés en un temps, un coût et un nombre d'étapes réduits. L'invention concerne également les polynucléotides doubles brins obtenus et les peptides, polypeptides ou protéines obtenus présentant une ou plusieurs propriétés améliorées ainsi que les utilisations du procédé.

La mutagenèse est une technique visant à modifier artificiellement la séquence nucléotidique d'un fragment d'ADN, dans le but de modifier l'activité biologique qui en découle.

Le terme de mutagenèse peut être associé à trois modifications distinctes d'un fragment d'ADN :
- la délétion, qui consiste à éliminer un ou plusieurs nucléotides du fragment d'ADN d'intérêt ;
- l'insertion, qui consiste à en rajouter ;
- la substitution, qui consiste à remplacer une ou plusieurs bases par un même nombre de bases de nature différente.

La mutagenèse joue un rôle central dans le domaine de l'amélioration des protéines, et principalement des enzymes et des protéines thérapeutiques.

L'amélioration des enzymes est d'un intérêt économique majeur : en effet, de très nombreuses enzymes industrielles sont utilisées dans différents procédés, - comme la synthèse d'antibiotiques ou de vitamines, la fabrication de bière, le traitement des textiles -, ou dans des produits aussi divers que les lessives ou l'alimentation pour le bétail (Turner et al.Trends Biotechnol. 2003 Nov;21 (11):474-8) L'amélioration des enzymes permet de réduire les coûts des procédés correspondants, ou permet d'en mettre en oeuvre de nouveaux.

Les paramètres à améliorer sont variés : Par exemple, et sans que cela soit exhaustif, on peut par évolution moléculaire obtenir une enzyme qui a un turn-over extrêmement élevé (Griffiths AD et al., EMBO J. 2003, 22(1):24-35); obtenir une enzyme qui a une thermostabilité accrue (Baik SH et al., Appl Microbiol Biotechnol. 2003, 61(4):329-35) ; optimiser une protéine thérapeutique (Vasserot AP et al., Drug Discov Today. 2003, 8(3):118-26) ; obtenir un peptide qui se lie avec une forte affinité à un ligand donné (Lamla T et al., J Mol Biol. 2003, 329(2):381-8) ; créer *in vitro* un anticorps contre à peu près n'importe quel ligand, pour une utilisation en diagnostic (Azzazy HM et al., Clin. Biochem. 2002, 35(6):425-45) ; créer un ribozyme avec une nouvelle activité catalytique (McGinness KE et al., Chem Biol. 2002, 9(5):585-96 ; Sun L. et al., Chem. Biol. 2002, 9(5):619-28). Les paramètres à améliorer peuvent être multiples : par exemple on peut obtenir, par évolution moléculaire, une enzyme à la fois résistante à la chaleur et à l'oxydation (Oh KH. et al., Protein Eng. 2002, 15(8):689-95) ou élargir la gamme de pH dans laquelle l'enzyme est efficace tout en augmentant son activité (Bessler C. et al. Protein Sci. 2003 Oct;12(10):2141-9). Enfin les enzymes permettent aujourd'hui de remplacer certains procédés chimiques lourds et polluants par des procédés beaucoup plus respectueux de l'environnement (on parle de chimie verte).

Dans le domaine des protéines thérapeutiques, l'obtention de protéines mutantes ayant des propriétés nouvelles est également d'un intérêt économique et thérapeutique majeur : l'isolement d'EPO mutant ayant une durée de vie prolongée ou d'insuline à effet retard constituent des exemples de succès dans l'obtention de nouvelles générations de protéines thérapeutiques par mutagenèse. Parmi les protéines thérapeutiques qu'il serait intéressant d'améliorer, on peut notamment citer les hormones, les cytokines, les interférons, les vaccins, et les anticorps.

Dans ce contexte de la recherche de mutants ayant acquis une nouvelle propriété ou amélioré une propriété existante, la mutagenèse constitue une première étape et permet de créer de la diversité. Dans une seconde étape, cette diversité est ensuite criblée en utilisant un test fonctionnel, afin d'isoler une molécule mutante codant pour une protéine améliorée. Cet événement est généralement rare, et un nombre important de molécules mutantes doivent être analysées avant d'obtenir une molécule améliorée.

Différentes approches de mutagenèse peuvent être mises en oeuvre dans ce contexte :

Une approche rationnelle, qui consiste à utiliser un raisonnement physicochimique et/ou des données structurales et/ou de la modélisation bioinformatique pour générer un petit nombre d'hypothèses, auquel correspondra un petit nombre de mutants à générer. Il est attendu que ces quelques mutants aient chacun une forte probabilité de correspondre à une protéine améliorée. Assez souvent, cependant, la relative rareté des données cristallographiques sur les protéines et la faible qualité des prédictions bioinformatiques rendent cette approche hasardeuse.

Une approche par « évolution moléculaire », qui est sensée reproduire, en accéléré et *in vitro,* l'évolution naturelle des gènes. Un grand nombre de variants sont générés au hasard. Ces mutants sont ensuite triés individuellement (criblage à haut débit) ou en masse (méthodes de sélection). Le plus souvent, le nombre de mutants à cribler est extrêmement important (typiquement: 10⁶-10¹²) puisque la très grande majorité des mutants ne sont pas améliorés et puisque si l'on souhaite explorer un espace de séquences raisonnablement intéressant, il faut une banque d'effectif élevé. En l'absence d'un système de sélection de masse, cette approche se révèle souvent fastidieuse.

Entre ces deux extrêmes, des stratégies mixtes peuvent exister : un grand nombre de mutants contenant une part de hasard et une part de rationalité peuvent être conçus et générés. Il est alors attendu que la fréquence des mutants améliorés dans ces banques semi-rationnelles soit plus forte que si la diversité est générée sur une base de hasard uniquement ; les efforts de criblage seront-donc réduits.

Les applications de l'évolution moléculaire ne se limitent pas à découvrir des protéines aux propriétés nouvelles ou améliorées. On peut aussi faire évoluer au laboratoire des acides nucléiques. Outre l'intérêt en recherche fondamentale (Mc Giness KE et al. Chem Biol. 2003 Jan;10(1):5-14), certains de ces ARN ou de ces ADN (notamment les ribozymes) peuvent avoir un intérêt biotechnologique, diagnostic ou thérapeutique. Des approches par longs oligonucléotides dégénérés ou par mutagenèse aléatoire ont permis de produire de premiers résultats prometteurs, notamment dans le domaine de l' "évolution continue" (Mc Ginness KE et al. Chem Biol. 2002 May;9(5):585-96 ; Tsukiji S : Nat Struct Biol. 2003 Sep;10(9):713-7; Ricca BI et al. J Mol Biol. 2003 Jul 25;330(5):1015-25 ; Khan AU et al. J Biomed Sci: 2003 Sep-Oct;10(5):457-67). Une méthode à haut-débit et spécifique permettant de faire évoluer (muter) puis de sélectionner une ou plusieurs molécules de ce type serait potentiellement complémentaire.

La mutagenèse a également un intérêt et une utilisation opposés : créer des mutations associées à une diminution de l'activité biologique. Cette démarche s'inscrit le plus souvent dans le cadre de recherchés en amont sur les relations entre la structure et la fonction des protéines, et a plus particulièrement pour objectif l'identification des résidus directement impliqués dans l'activité de la protéine étudiée. Cette approche n'est le plus souvent pas associée à une application industrielle immédiate.

Si la modification d'un acide aminé entraîne la perte de l'activité biologique, il est probable que cet acide aminé intervienne dans la constitution du site actif supportant cette activité biologique. Cette conclusion doit cependant être considérée avec beaucoup de circonspection : il est alternativement possible que cet acide aminé n'intervienne pas directement dans le site actif de l'activité biologique, mais qu'il prenne part à des activités annexes (comme l'adressage intracellulaire de la protéine par exemple), ou encore que la modification introduite à son niveau déstabilise l'ensemble de la protéine, l'effet de la substitution introduite étant alors indirect, et non direct.

Il est alors important de savoir reconnaître les motifs supportant les activités d'adressage, de localisation membranaire, de liaison de cofacteurs...

D'autre part, il est essentiel que les modifications introduites induisent le moins de déstabilisation possible de la structure secondaire de la protéine. C'est pour cela que, le plus souvent, c'est l'Alanine qui est introduite en substitution des acides aminés d'origine. Ce petit acide aminé est connu pour conserver la majorité des structures secondaires protéiques (Hélice alpha ou feuillet béta), pour ne pas entraîner d'importante modification stérique ni électrique, et donc pour minimiser les déstabilisations globales des protéines.

Les travaux menés dans ce domaine impliquent la création d'un grand nombre de mutants ponctuels comportant chacun la substitution d'un acide aminé par une Alanine. Ces molécules mutantes sont ensuite destinées à être étudiées individuellement au moyen de tests fonctionnels, pour mesurer l'effet de la substitution introduite. Plusieurs centaines d'articles ont été publiés qui utilisent cette technique. Le principe de l'alanine scanning, l'intérêt et les limites de cette stratégie sont présentes notamment dans les revues de DeLano WL. (Curr Opin Struct Biol. 2002 Feb;12(1):14-20) et de Morrisson KL et al. (Curr Opin Chem Biol. 2001 Jun;5(3):302-7) ; la base de données ASEdb centralise de nombreux résultats d'alanine scan (Thom KS et al. Bioinformatics. 2001 Mar;17(3):284-5). Dans certain cas, les acides aminés sont remplacés systématiquement par des cystéines et non par des alanines (Tamura N et al. Curr Opin Chem Biol. 2003 Oct;7(5):570-9 ; Winkler HH et al. Biochemistry. 2003 Nov 4;42(43):12562-9). Plus généralement, on peut envisager tout type de substitution systématique par un acide aminé donné. Dans la même optique d'utiliser l'évolution moléculaire non directement pour améliorer des protéines, mais dans le but de recherche pour générer des données permettant d'analyser les relations structure-fonction dans la protéine, Christ D et al. ont récemment décrit une approche faisant appel a de la mutagenèse semi-aléatoire (Proc Natl Acad Sci U S A. 2003 Oct 22).

En résumé, la mutagenèse est un outil qui permet d'obtenir des molécules améliorées ayant un intérêt économique tout particulièrement dans le domaine de la biocatalyse (enzymes industrielles) et dans celui de la médecine (protéines thérapeutiques). La mutagenèse est également une approche qui permet de caractériser les protéines dans un but de recherche, en identifiant les acides aminés d'une protéine directement liées à sa fonction.

Bien que l'essentiel de l'intérêt économique se situe dans le domaine des protéines, la mutagenèse et l'évolution moléculaire d'ADN ou d'ARN, en particulier D'BARN doués de propriétés catalytiques (ribozymes) peut être intéressante. Une mutagenèse site spécifique haut-débit est également intéressante dans ce contexte.

Différentes techniques de mutagenèse ont été développées au cours des dernières décennies, et peuvent être utilisées dans ces différents contextes.

Les techniques de mutagenèse peuvent être séparées en cinq grands groupes :
la mutagenèse aléatoire ;
la mutagenèse par mélange d'ADN (la recombinaison) ;
la mutagenèse dirigée ;
la mutagenèse à saturation ;
la mutagenèse semi-aléatoire.

La mutagenèse aléatoire vise à introduire dans un fragment d'ADN des substitutions de nature et de position non contrôlées.

Historiquement, la mutagenèse aléatoire était réalisée au moyen de procédés chimiques altérant la structure de l'ADN (Richie DA Genet Res. 1965 Nov;6(3):474-8 et Bridges BA Mutat Res. 1966 Aug 3(4):273-9).

Une seconde approche pour générer des mutants aléatoires consiste à transformer le plasmide contenant le gène d'intérêt dans des souches de bactéries dites « mutatrices » (Giraud A et al. Curr Opin Microbiol. 2001 Oct;4(5):582-5), déficientes en certains gènes impliqués dans la fidélité de réplication de l'ADN (irving RA et al. Methods Mol Biol. 2002;178:295-302). Cette approche n'est plus guère utilisée aujourd'hui, notamment en raison des problèmes liés à l'instabilité génétique de ce type de souches.

Plus récemment, de très nombreux documents ont décrit des techniques de mutagenèse aléatoire basées soit sur l'utilisation d'une polymérase modifiée ayant structurellement une faible fidélité de réplication, soit sur l'utilisation d'une polymérase non modifée, mais dans des conditions particulières d'amplification menant à un fort taux de mutation.(la PCR mutagene ou 'error-prone PCR' est revue dans Cirino PC et al. Methods Mol Biol. 2003;231:3-9 ; Leung, D.W., et al. (1989) Technique 1: 11-15 ; Cadwell, R.C. and Joyce, G.F. (1992) PCR Methods Appl. 2: 28-33.). Dans un cas comme dans l'autre, l'enzyme introduit des mutations au fil des cycles ; à la fin de la réaction, de nombreuses copies de la molécule initiale sont obtenues, chacune de ces molécules comportant une ou plusieurs mutations différentes. Ces molécules sont présentes sous forme d'une banque, c'est-à-dire d'un mélange de molécules de nature différente. Le nombre moyen de mutations par molécule peut être maîtrisé en jouant sur les différents paramètres de la réaction de mutagenèse.

La mutagenèse aléatoire présente des limitations importantes. Ainsi, les mutations introduites par la polymérase ne concernent pas le plus souvent plusieurs nucléotides contigus, mais un seul. En utilisant l'approche de mutagenèse aléatoire, seule une partie des 64 codons possibles peuvent être obtenus à partir de ces simples substitutions et, en moyenne, seuls 5 acides aminés sur les 19 possibles peuvent être obtenus à partir du codon de départ. De surcroît, chaque base n'est pas substituée de manière équiprobable par chacune des autres bases, ce qui introduit des biais dans les populations de molécules d'ADN créées par rapport à des populations idéales ou tout A, par exemple, aurait la même probabilité d'être remplacé par un T, un C ou un G.

En outre, une des limitations de la mutagenèse aléatoire vient de la nécessité de cloner dans un vecteur linéarisé le fragment d'ADN obtenu lors de la réalisation de la PCR mutagène. Cette étape de clonage se révèle souvent le facteur limitant lorsqu'on cherche à obtenir un grand nombre de molécules mutantes. En effet, l'étape de ligation restreint à 10⁶ environ l'effectif de la banque.

La mutagenèse par mélange d'ADN s'inspire du processus de recombinaison à l'oeuvre dans l'évolution Darwinienne, notamment lors de la reproduction sexuée. La mutagenèse par mélange d'ADN consiste à recombiner des séquences partiellement homologues, isolées à partir de différents organismes. Par exemple, si l'on travaille sur une enzyme, la première étape d'une approche par mélange d'ADN reviendra à isoler un grand nombre de gènes homologues à cette enzyme, soit à partir de collections de souches, soit à partir de gènes directement isolés à partir d'échantillons naturels (par une approche maintenant décrite sous le terme de « metagénome »). Différentes approches existent alors pour mélanger les domaines de ces gènes homologues et générer une banque de molécules d'ADN « chimériques », c'est-à-dire constituées de plusieurs domaines ayant des provenances différentes ( (Brevet Maxygen US 6 132 970 ; Stemmer WP et al. Nature. 1994 Aug 4;370(6488):389-91; Aguinaldo AM, et al.-Methods Mol Biol. 2002;192:235-9 ; Zhao H et al. Nat Biotechnol. 1998 Mar;16(3):258-61.) ; Shao, Z et al. Nucleic Acids Res. 26 (2): 681-683 ; Kawarasaki Y et al. Nucleic Acids Res. 2003 Nov 1;31(21):e126 ; brevet Diversa US 5 965 408 ; Brevet Proteus WO 00 09 679 ; Brevet Alligator WO 02 48 351). Il est attendu que ces molécules contiennent ainsi des caractéristiques nouvelles, et notamment des propriétés cumulées de deux ou plusieurs gènes parentaux. Ainsi, par exemple, si l'on dispose au départ de deux gènes homologues d'une même enzyme, l'un connu pour être très actif, et l'autre pour être thermostable (ce dernier ayant par exemple été isolé à partir d'un organisme thermophile), on peut espérer que certaines des molécules obtenues par mélange de ces deux gènes, - contenant alors certains domaines du premier et certains autres du second -, auront des propriétés cumulées de forte activité et de thermostabilité (une telle additivité n'est pas évidente et pas toujours assurée mais est en pratique assez fréquemment observée). Dans certains cas, non seulement des propriétés cumulées, mais également des propriétés nouvelles (par exemple une activité supérieure à celle de tous les gènes naturels de départ), ont été obtenues par mélange de gènes.

Cette approche par recombinaison d'ADN est basée sur l'idée générale que la combinaison nouvelle de mutations naturelles,- ayant donc été pré-criblées par la nature pour le maintien de l'activité de l'enzyme -, a plus de chance d'être porteuse d'amélioration que l'introduction de mutations générées au hasard. En même temps que l'on restreint la génération de diversité à un espace de séquence 'raisonnable' car 'préselectionné', on est cependant limité par les séquences d'origine, qu'il faut connaître, par la nécessite d'avoir des gènes ayant un niveau d'homologie suffisant, et par l'impossibilité de générer des séquences autres que des combinaisons des séquences initiales.

Ces approches de mélange d'ADN se sont révélées être particulièrement efficaces dans le domaine de l'amélioration des enzymes. Par contre, cette approche n'est pas adaptée au domaine des protéines thérapeutiques, d'une part parce que les polymorphismes humains sont relativement limités, et d'autre part parce qu'il n'est guère concevable, pour des raisons d'immunogénicité notamment, de penser que le mélange d'ADN à partir de protéines de différentes espèces puisse apporter un bénéfice notable en thérapeutique humaine.

La mutagenèse dirigée vise à introduire dans un gène recombinant une ou quelques mutations de nature et de position connues. Un oligonucléotide est utilisé pour introduire cette ou ces mutations. Cet oligonucléotide est classiquement constitué de vingt à trente bases homologues à la région ciblée et contenant en son centre la ou les mutations projetées.

Cet oligonucléotide est utilisé pour amorcer une réaction de réplication (ou d'amplification, c'est-à-dire de multiples réplications) en utilisant le fragment d'ADN comme matrice. La séquence nouvellement synthétisée contient alors la modification recherchée.

Les premières techniques de mutagenèse dirigée étaient basées sur l'amplification d'un fragment d'ADN linéaire, qui devait ensuite être introduit dans un plasmide par clonage en utilisant des enzymes de restriction.

Plus récemment, l'oligonucléotide mutant a été utilisé pour amorcer la réplication circulaire du plasmide contenant le fragment d'ADN d'intérêt. Le nombre de manipulations à réaliser est ainsi minimisé. Cependant, une étape de sélection est nécessaire pour isoler les molécules ayant effectivement intégré la mutation des molécules d'ADN de départ. Cette étape de sélection des molécules mutantes peut reposer sur l'utilisation d'organismes particuliers, comme la souche bactérienne ung- (Kunkel TA, Bebenek K, McClary J. Methods Enzymol. 1991;204:125-39.), ou le phage M13. Elle peut aussi être basée sur l'introduction simultanée d'une seconde mutation, co-ségrégeant avec la première et sélectionnable sur un critère de résistance aux antibiotiques (EP 0938552), ou de modification d'un site de restriction unique (Catalogue Clontech 2000, page 45).

Ces approches sont maintenant obsolètes, et l'approche aujourd'hui la plus répandue est basée sur les-différences de méthylation entre l'ADN synthétisé dans des bactéries (méthylé) et l'ADN synthétisé *in vitro* (non méthylé). Un système de criblage basé sur ce critère a été mis au point et est maintenant généralisé : Il s'agit d'utiliser l'enzyme Dpnl, spécifique de sites présents sur l'ADN méthylé, mais pas sur l'ADN non méthylé (Lacks et al., 1980, Methods in Enzymology, 65:138). Les enzymes NanII, NmuDI et NmuEI peuvent également être utilisées pour remplir le même objectif. Dans une réaction de mutagenèse par extension circulaire d'un oligonucléotide hybridé sur un plasmide, ces enzymes digèrent les brins parentaux (produits *in vivo* par les bactéries, méthylés), mais pas les brins synthétisés lors de la réaction de mutagenèse, non méthylés. La digestion en utilisant ces enzymes a donc pour effet d'augmenter la fréquence de molécules mutantes en éliminant les brins parentaux non mutants.

L'effet de ces enzymes sur les espèces moléculaires dont les deux brins sont identiques est claire ; mais leur action sur les molécules d'ADN dont un seul des deux brins est méthylé, - l'autre ayant été synthétisé *de novo* -, n'a pas été élucidé de façon aussi définitive. Il est cependant probable que ces molécules, parfois appelés « hétéroduplex », ne puissent pas être digérées efficacement par les enzymes Dpnl, NanII, NmuDI ou NmuEI. Or, lorsque l'on utilise un seul oligonucléotide mutant, les hétéroduplex sont sensés constituer le groupe majoritaire (la mutation recherchée n'est présente que sur un seul des deux brins). Après digestion par une des enzymes précédemment citées, on s'attendrait à avoir une importante fréquence de mutagenèse (50%). Cependant, cette efficacité reste faible et le plus souvent comprise entre 1 et 10% de molécules mutantes, selon les cas. Ce faible taux de mutants est notamment dû au fait qu'à l'issue de la réaction de mutagenèse, les hétéroduplex sont introduits dans des bactéries contenant un système de réparation de l'ADN, qui utilise le brin méthylé (et donc non mutant) comme matrice à copier. Ce système de réparation aboutit donc à la correction de la mutation introduite, et à une importante perte de rendement de mutagenèse.

Pour améliorer l'activité de mutagenèse, l'utilisation d'un second oligonucléotide, destiné à synthétiser le deuxième brin, est préconisée.

Ce second oligonucléotide peut être non mutant, et localisé au niveau d'une région différente de la région à muter (EP 96942905; WO 9935281).

Alternativement, le second oligonuclétide peut être réverse complémentaire du premier, et contenir alors, également, la mutation. C'est cette dernière approche qui donne les meilleurs rendements de mutagenèse et qui est à la base du procédé Quickchange (Stratagene 2003 référence #200518). Ce procédé est devenu ces dernières années la référence en terme de mutagenèse, en raison de son rendement inégalé.

La mutagenèse dirigée est une approche technologique très puissante. Sa limite principale réside dans son débit : en effet, il n'est guère possible de générer plus de un ou quelques mutants dirigés par jour et par personne.

La quatrième approche pour générer de la diversité est la « mutagenèse à saturation ». Cette approche consiste à utiliser des oligonucléotides pour générer au niveau d'un codon ciblé non pas une unique substitution, mais un ensemble de mutants contenant les 64 codons possibles, ou un sous-ensemble de ces 64 codons.

La mutagenèse à saturation est basée sur l'utilisation - d'oligonucléotides dégénérés : Lors de la synthèse des oligonucléotides, il est facile de créer une dégénérescence à n'importe quel endroit de la séquence de l'oligonucléotide : au niveau de la position souhaitée, on utilise alors non pas une base unique, mais un mélange équimolaire de plusieurs bases. Dans une séquence, on note conventionnellement N le mélange équimolaire des quatre bases. Par exemple, ATN correspond à 25% de ATA, 25% de ATT, 25% de ATG et 25% de ATC. Un oligonucléotide contenant deux positions dégénérées est en fait constitué d'un mélange équimolaire de 16 oligonucléotides. Un oligonucléotide avec trois positions dégénérées N est constitué d'un mélange équimolaire de 64 oligonucléotides. Ces oligonucléotides incluant des bases dégénérées sont généralement vendus par les sociétés spécialisées dans la synthèse d'oligonucléotides sans surcoût.

Les oligonucléotides contenant un codon totalement dégénéré (NNN) permettent d'introduire en un point une diversité maxima le, c'est-à-dire les 64 codons possible. A partir de ces 64 codons possibles seront traduits les 19 acides aminés substituants possibles.

L'introduction des oligonucléotides dégénérés peut être réalisée en utilisant à peu près n'importe quelle technique de mutagenèse dirigée ; il a cependant été noté que la technique Quickchange ne s'adaptait pas bien à cette approche.

Par rapport à la mutagenèse dirigée, la mutagenèse à saturation permet de réduire sensiblement le travail à mener pour obtenir une molécule mutante : plusieurs mutants à saturation peuvent être générés en un jour par une seule personne, ce qui correspond à chaque fois à 19 mutants différents. Toutefois, ce gain en efficacité n'est obtenu qu'au prix d'un certain nombre de concessions techniques :

Chacun des 19 acides aminés est intégré à des fréquences diverses en raison de la dégénérescence du code génétique : Ainsi la Sérine est-elle intégrée 6 fois plus souvent que le Tryptophane, et 3 fois plus souvent que l'Acide Aspartique. Il sera nécessaire de fournir des efforts importants avant d'isoler les mutants correspondant à des acides aminés représentés seulement une ou deux fois.

Dans 3 cas sur 64, un codon STOP est intégré : environ 5% (3/64) des clones produits sont simplement sans intérêt.

Une partie importante des codons introduits sont des codons minoritairement représentés dans l'organisme choisi pour l'expression des molécules mutantes. Ces codons minoritairement représentés sont généralement défavorables à l'expression, et peuvent compliquer l'analyse en masquant une mutation positive.

Une solution pour minimiser ces imperfections consiste à utiliser des oligonucléotides partiellement dégénérés, c'est-à-dire constitués de codons de type NNG/T (encore notés NNK) au niveau du codon à modifier. Plusieurs solutions ont été décrites pour la mutagenèse à saturation à grande échelle : Savino et al, P.N.A.S 1993 90 :4067-71 ; Olins et al., Journal of Biological Chemistry 1995 270 :23754-60 ; brevets US6,562,594 et US6,171,820 (Diversa) ; US6,180,341 (université du Texas) ; Maynard JA et al. Methods Mol Biol 2002 182:149-63. Ce type de dégénérescence constitue le codon minimal permettant d'introduire les 19 acides-aminés. Les différences de représentation d'un acide-aminé à l'autre sont atténuées (rapport 3 au maximum au lieu d'un rapport de 6 dans le cas des codons NNN). Par contre, la fréquence des codons STOP est légèrement augmentée (2 codons sur 32 au lieu de 3 sur 64). L'effet sur la qualité des codons en terme d'expression n'est pas général, et dépend de l'organisme utilisé pour l'expression.

L'utilisation des oligonucléotides de type NNN ou NNK reste donc très imparfaite : l'idéal serait de disposer des 19 oligonucléotides correspondant aux 19 substitutions possibles au niveau d'une position. Cette approche permettrait d'introduire les 19 substitutions possibles à la même fréquence, sans introduire de codons STOP, et en respectant parfaitement les contraintes de représentation de codons de l'organisme choisi pour l'expression.

Si l'on synthétise les 19 oligonucléotides indépendamment, le coût associé augmente proportionnellement : l'achat des 19 oligonucléotides simples coûte 19 fois plus cher que l'achat de l'oligonucléotide dégénéré permettant d'introduire en une seule fois toutes les mutations. Le bénéfice apporté par la résolution des trois imperfections précédemment citées ne vaut pas, dans la plupart des cas, cet important surcoût.

Une solution alternative permet d'introduire, au niveau du codon mutant de chaque oligonucléotide, uniquement les 20 codons souhaités, c'est-à-dire chacun des 20 codons préférentiellement utilisé par l'organisme dans lequel seront exprimés les mutants. Deux techniques peuvent être utilisées pour synthétiser ces oligonucléotides : la première est basée sur le fractionnement des colonnes de résine pendant le déroulement de la synthèse d'oligonucléotides (US 20030175887). Elle est fastidieuse, et n'est pas adaptée à la synthèse d'un grand nombre d'oligonucléotides. Dans une seconde approche, les 20 triplets de nucléotides sont individuellement synthétisés par procédé chimique sous forme de phosphoramidites. Les vingt trinucléotides sont ensuite réunis dans un mélange utilisables sur un synthétiseur d'oligonucléotides (au même titre que n'importe quel nucléotide-phosphoramidite). Le brevet US 5,869,644 décrit la synthèse de tels oligonucléotides pour la biologie moléculaire. Le brevet US 6,436,675 décrit l'utilisation de tels oligonucléotides dans un contexte de recombinaison par synthèse de gène. Les phosphoramidites-trinucléotides restent cependant très complexes à synthétiser, et coûtent excessivement cher. Le surcoût varie ainsi d'un facteur 3 à 10 par rapport au coût d'un oligonucléotide simple, ou dégénéré de type NNN. Ce surcoût est inférieur à celui associé à la synthèse indépendante des 19 oligonucléotides, mais reste critiquable au regard du bénéfice résultant.

Il peut aussi être souhaitable d'introduire au niveau d'un résidu non pas toutes les substitutions possibles, mais seulement une partie de celles-ci. Par exemple, on peut vouloir conserver la classe chimique de l'acide aminé et ne le substituer que par un acide aminé de même classe ; on peut aussi, par exemple, vouloir éviter de remplacer des résidus hydrophobes par des résidus hydrophiles. On peut alors avoir recours à l'utilisation d'oligonucléotides semi-dégénérés, c'est-à-dire composés en réalité de mélanges de 2 à 63 oligonucléotides ne différant qu'au niveau du codon mutant, et permettant d'intégrer une diversité composée de 2 à 18 acides aminés différents. Le codon mutant est alors composé de la combinaison de bases totalement dégénérées, simples, et/ou semi-dégénérées, c'est-à-dire composées d'un mélange de deux ou trois bases. Les sociétés fournissant des oligonucléotides offrent toutes la possibilité d'incorporer des bases semi-dégénérées. Cette diversité « sur mesure » peut se montrer moins simple à introduire que la diversité totale : dans certains cas, il n'est pas possible de concevoir un seul oligonucléotide partiellement dégénérées pour introduire la diversité souhaitée, et deux ou trois oligonucléotides doivent être synthétisés et utilisés de façon complémentaire. Bien entendu, on peut concevoir que ces mutations semi-dégénérées puissent également être introduites en utilisant l'approche des triplets de nucléotides décrite précédemment. Il est cependant nécessaire, dans ce cas, de prévoir autant de mélanges de trinucléotides que de diversités différentes à introduire, et un volume minimal de chaque mélange doit être réalisé afin de pouvoir disposer d'un flacon suffisamment plein pour être utilisable sur un synthétiseur d'oligonucléotides. Si l'on souhaite introduire la même diversité en tous les points ciblés du gène, cette approche peut être utilisée. Mais si l'on souhaite générer des diversités sur mesure, différentes d'un résidu à l'autre, le coût élevé des trinucléotides et la nécessité d'un volume minimal de mélange constituent une barrière majeure.

La cinquième approche pour générer de la diversité est la Mutagenèse Massive (Delcourt et Blésa, WO/0216606) : cette technique permet d'introduire des mutations dirigées non plus de façon unique, mais de façon multiple et combinatoire. Ces mutations multiples présentent des caractéristiques propres par rapport aux mutations simples : des synergies entre les mutations peuvent faire qu'un double mutant correspond à une activité améliorée par rapport à la molécule sauvage, alors que chacun des deux simples mutants ne présente aucune amélioration.

La Mutagenèse Massive est une technique basée sur l'utilisation simultanée d'un nombre important d'oligonucléotides (supérieur à 5 et de préférence compris entre 50 et 5000), tous orientés dans le même sens, pour amorcer une réaction de réplication d'un plasmide circulaire en utilisant une polymérase thermostable. Eventuellement, une ligase thermostable peut également être ajoutée dans la réaction pour augmenter le taux de mutation en effectuant activement la ligation des brins néosynthétisés sur l'extrémité 5' des oligonucléotides hybridés. Cette technique permet d'obtenir un rendement supérieur à 50% de molécules ayant incorporé au moins une mutation. Le nombre moyen de mutations par molécule mutante peut être modulé à loisir, soit en modifiant la concentration des divers réactifs soit en effectuant la procédure plusieurs fois successivement.

A l'issue de la réaction de Mutagenèse Massive, on dispose d'une banque de molécules mutantes dont la diversité peut dans certains cas être supérieure à 10⁸ molécules différentes.

Une approche alternative à la mutagenèse massive a été décrite pour générer de la diversité combinatoire. Elle est basée sur la synthèse complète de gènes en utilisant des oligonucléotides contenant des bases dégénérées (Brevet Maxygen US 6,579,678 ; Crea US 5,798,208). Cette approche par synthèse de gène se heurte cependant au problème de la fidélité de synthèse des oligonucléotide (environ 0,5 % d'erreur à chaque position), bien inférieure à la fidélité de réplication de l'ADN par une polymérase (moins de 0,01 % d'erreur par position lors d'une amplification par PCR); la plupart des gènes synthétisés contiennent ainsi, en plus des mutations ciblées, une ou plusieurs mutations additionnelles, le plus souvent des délétions d'une ou de quelques bases. Dans la majorité des cas, ces délétions décalent la phase de lecture et rendent impossible la traduction de la protéine. Ce procédé de génération de la diversité combinatoire par synthèse complète aboutit ainsi à une très importante proportion de molécules mutantes sans intérêt, ce qui augmente les efforts de criblage qui seront nécessaires avant d'identifier un mutant positif. Dans les cas où le système de criblage est extrêmement efficace, comme dans les approches par sélection de masse, la qualité de la diversité importe peu. Mais lorsque le criblage nécessite des efforts importants, il est préférable d'utiliser une technologie aboutissant au plus fort taux de mutants utiles. C'est le cas de la Mutagenèse Massive, qui n'incorpore de mutations indésirables qu'extrêmement rarement.

Dans une de ses applications, la Mutagenèse Massive permet de réaliser l'ensemble des mutants Alanine (ou un autre acide aminé quelconque donné d'un gène), utilisables dans le but d'identifier les positions essentielles à l'activité d'une protéine. Dans cette application, on obtient une banque contenant des mutants ayant intégré soit aucune mutation, soit une ou quelques substitutions d'un codon en codon Alanine. L'activité de ces mutants est mesurée individuellement, et une cartographie fonctionnelle de la protéine peut être établie.

Dans une seconde application, la Mutagenèse Massive permet de générer un très grand nombre de mutants simples ou multiples en introduisant au niveau de certains sites d'un gène une diversité variable. Le nombre de sites ciblés, la nature de la diversité, et le nombre moyen de mutations par molécule sont modulables à loisir. Si l'on cherche à introduire une large diversité, des oligonucléotides contenant des bases dégénérées, tels que décrits précédemment, peuvent être utilisés. Egalement, on peut n'introduire que des substitutions ayant été pré-sélectionnées par bioinformatique (par modélisation ou par analyse des séquences naturelles homologues) et étant associées à une probabilité accrue de véhiculer une amélioration.

De toutes les technologies de mutagenèse, la Mutagenèse Massive est la seule qui permette de réaliser une diversité sur mesure, c'est-à-dire un grand nombre de molécules contenant des combinaisons de mutations définies obtenues en une seule réaction et en un temps court, sans nécessité de connaître des séquences autres que celle du gène à muter. Appliquée à l'évolution moléculaire des protéines, cette technologie permet d'intégrer dans la diversité introduite des éléments rationnels, et permet ainsi d'augmenter la fréquence des mutants positifs et donc d'élargir l'espace des séquences effectivement exploré tout en diminuant les coûts de criblage.

Toutefois, la Mutagenèse Massive connaît deux limitations :
d'une part, la technologie est basée sur l'utilisation d'un grand nombre d'oligonucléotides. Le coût associé à ces oligonucléotides peut constituer une limite à l'utilisation de la technologie, dès lors que leur nombre est important ;
d'autre part, lorsque l'on souhaite introduire en plusieurs points une large diversité, en utilisant des oligonucléotides contenant des bases dégénérés (de type NNN ou NNK par exemple), les biais de représentation des différents acides-aminés, décrits plus haut dans le cadre de la mutagenèse dirigée, se retrouvent ici d'une façon exacerbée : par exemple, le biais introduit en un point par un oligonucléotide dégénéré de type NNN est d'un facteur 6 entre le tryptophane (Trp) et la sérine (Ser). Lorsque l'on considère des mutants doubles, le biais est d'un facteur 36 entre la combinaison Trp-Trp et la combinaison Ser-Ser. Les biais d'adaptation de certains codons pour l'expression dans l'organisme choisi, également décrits plus haut dans le cadre de la mutagenèse dirigée, sont également retrouvés dans le cadre de la Mutagenèse Massive avec une intensité amplifiée.

Ces limitations de coût et de qualité nuisent à l'efficacité de la technologie. La limitation de qualité peut être en partie résolue en utilisant une approche basée sur l'utilisation de cassettes de trinucléotides, mais il a été décrit plus haut, dans la partie dédiée à la description de la mutagenèse dirigée, que la solution apportée par cette approche n'est que partielle ; le coût très important de la synthèse chimique des trinucléotides, et la lourdeur de l'approche (interdisant de moduler à loisir la diversité introduite en chacune des positions), s'appliquent également dans le contexte de la Mutagenèse Massive.

Une technologie permettant de s'affranchir de ces deux limitations, de coût et de qualité, faciliterait l'obtention de mutants améliorés et aurait donc une valeur économique, principalement dans le domaine des enzymes industrielles et des protéines thérapeutiques ; elle rendrait également plus simples certains projets de recherche fondamentale, notamment dans le domaine de la cartographie fonctionnelle des protéines.

L'objet de l'invention est un procédé permettant de produire, directement sous forme de banques, des polynucléotides mutants dirigés simples ou multiples d'une meilleure qualité et/ou à un coût réduit par rapport aux techniques selon l'art antérieur.

Dans le procédé de Mutagenèse Massive, les oligonucléotides utilisés pour introduire les mutations sont employés sous forme de banque, en très faible quantité chacun. Ces oligonucléotides sont synthétisés et remis en solution individuellement puis il sont mélangés avant d'être utilisés pour la réaction de mutagenèse qui consomme typiquement 0.1 à 10 picomoles de chaque oligonucléotide. Or l'échelle de synthèse de ces oligonucléotides, même si l'on choisit la plus petite possible sur les synthétiseurs commerciaux, représente plusieurs dizaines de nanomoles. Seule une faible proportion de chacun des oligonucléotides est donc utilisée. Ce gaspillage est à mettre en regard du coût important que représente la synthèse individuelle des oligonucléotides dans la mise en oeuvre de la technologie de Mutagenèse Massive.

La présente invention concerne un procédé de mutagenèse notamment caractérisé par l'utilisation d'un grand nombre d'oligonucléotides synthétisés sur une puce à ADN En effet, les mélanges d'oligonucléotides générés en utilisant des puces d'ADN correspondraient à un coût pouvant être quarante fois inférieur au même mélange synthétisé en utilisant l'approche classique de synthèse individuelle d'oligonucléotides.

L'invention est encore caractérisée par le recours à un procédé physique et/ou chimique permettant, à l'issue de l'étape de synthèse des oligonucléotides sur ladite puce à ADN, d'isoler ces oligonucléotides de leur support pour les mettre en solution. Plus précisément, ces oligonucléotides sont obtenus directement de la puce sous forme de mélange. Dans un mode de réalisation, un composé chimique labile sous certaines conditions physico-chimiques, est déposé sur la puce à ADN préalablement à la synthèse des oligonucléotides. En fin de réaction de synthèse, les oligonucléotides sont mis en solution (sous forme d'un mélange) en soumettant la puce aux conditions associées à cette labilité.

La présente invention concerne un procédé de production d'une banque de gènes mutés comprenant les étapes suivantes :
a. Synthèse sur une puce à ADN d'une banque d'oligonucléotides comprenant des oligonucléotides complémentaires d'une ou plusieurs régions d'un ou de plusieurs gènes cibles et comprenant chacun, de préférence en leur milieu, une ou plusieurs mutations par rapport à la séquence du ou des gènes cibles ;
b. Mise en solution de la banque d'oligonucléotides synthétisée en a) ; et,
c. Génération d'une banque de gènes mutés en utilisant la banque d'oligonucléotides en solution obtenue en b) et une ou plusieurs matrices contenant ledit ou lesdits gènes cibles.

De préférence, dans l'étape c), la banque de gènes mutés est générée par le procédé de Mutagenèse Massive (décrite notamment dans WO/0216606). Plus particulièrement, l'invention concerne le procédé ci-dessus décrit, dans lequel l'étape c) comprend les étapes suivantes :
i. Fournir une ou plusieurs matrices contenant ledit ou lesdits gènes cibles ;
ii. Mettre en contact ladite ou lesdites matrices avec la banque d'oligonucléotides synthétisée en a) dans des conditions permettant l'hybridation des oligonucléotides de la banque sur ladite ou lesdites matrices pour produire un mélange réactionnel;
iii. Effectuer une réplication de ladite ou desdites matrices à partir du mélange réactionnel en utilisant une ADN polymérase;
iv. Eliminer la ou les matrices initiales du produit de l'étape iii) et ainsi sélectionner les brins d'ADN néosynthétisés ; et, éventuellement,
v. Transformer un organisme avec le mélange d'ADN obtenu à l'étape iv).

De préférence, la matrice est un acide nucléique circulaire, plus particulièrement un plasmide. De façon alternative, la matrice peut être un acide nucléique linéaire. Dans un mode de réalisation préféré, la matrice contient les éléments permettant l'expression dudit ou desdits gènes cibles.

De préférence, les oligonucléotides de ladite banque synthétisés sur la puce à ADN sont liés à ladite puce à ADN par l'intermédiaire d'une molécule espaceur clivable et lesdits oligonucléotides sont mis en solution en soumettant les oligonucléotides liés ladite puce à ADN aux conditions associées au clivage de la molécule espaceur. La molécule espaceur peut être clivable en milieu basique, par l'action de la lumière ou par action enzymatique. Cependant, la présente invention ne se limite pas à ce mode de réalisation et considère tout moyen de synthèse d'une banque d'oligonucléotides sur une puce à ADN permettant de mettre ultérieurement cette banque d'oligonucléotides en solution. Dans un mode de réalisation particulier, ladite molécule espaceur est clivable en milieu basique. Par exemple, le milieu basique est une solution d'ammoniaque. Dans un mode de réalisation préféré, ladite molécule espaceur est le composé de formule (composé A) :

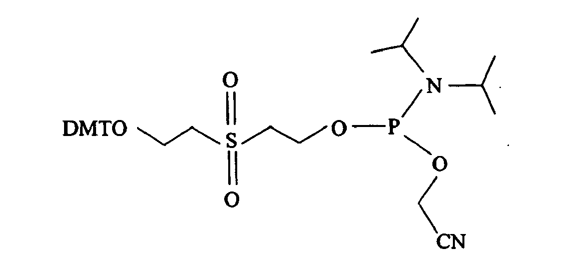

Dans un mode de réalisation préféré, ladite molécule espaceur est le composé de formule (composé B):

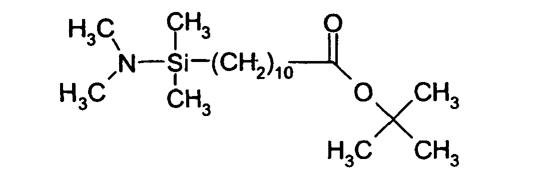

De préférence, chacun des oligonucléotides de la banque obtenue à l'étape b) est présent en une quantité comprise entre 1 femtomole et 1 picomole.

Dans un mode de réalisation particulier, l'étape iv est réalisée au moyen d'une enzyme de restriction spécifique des brins d'ADN méthylés, de préférence appartenant au groupe des enzymes : Dpnl, NanII, NmuDI ou NmuEI.

Dans un mode de réalisation préféré, les oligonucléotides synthétisés à l'étape a) sont tous complémentaires d'un même gène cible.

De préférence, tous les oligonucléotides complémentaires d'un même gène cible sont complémentaires du même brin dudit gène cible.

Dans un premier mode de réalisation préféré, la banque d'oligonucléotides synthétisés à l'étape a) contient les oligonucléotides portant les mutations permettant d'introduire l'ensemble des substitutions possibles sur chaque codon dudit ou desdits gènes cibles. Dans un second mode de réalisation préféré, la banque d'oligonucléotides synthétisés à l'étape a) contient les oligonucléotides portant les mutations permettant d'introduire un même acide aminé, de préférence une alanine, sur chaque codon dudit ou desdits gènes cibles.

De préférence, la synthèse de la banque d'oligonucléotides sur la puce à ADN est réalisé par toutes méthodes adéquates de synthèse d'oligonucléotides sur puces bien connues de l'homme du métier, parmi lesquelles on trouve les méthodes décrites précédemment.

De préférence, ledit organisme de l'étape v) est une bactérie ou une levure.

Dans un premier mode de réalisation, l'ADN polymérase est une polymérase thermosensible. Par exemple, elle peut être sélectionnée parmi l'ADN polymérase de T4, de E. coli, ou encore le seul fragment de Klenow de la polymérase d'E. coli. Dans un second mode de réalisation, l'ADN polymérase est une polymérase thermostable. Par exemple, elle peut être sélectionnée parmi les polymérases Taq, Pfu, Vent, Pfx ou KOD.

L'invention concerne en outre un procédé de mutagenèse dirigée comprenant les étapes du procédé de production d'une banque de gènes mutés selon la présente invention.

La présente invention concerne également un procédé de mutagenèse d'une protéine cible ou de plusieurs protéines cibles, caractérisé en ce qu'il comprend la préparation d'une banque d'expression de gènes mutés à partir d'un gène cible codant pour ladite protéine, ou de plusieurs gènes cible codant pour lesdites protéines, par le procédé de production d'une banque de gènes mutés selon la présente invention, puis l'expression desdits gènes mutés pour produire une banque de protéines mutées.

La présente divulgation concerne un procédé d'évolution d'un gène ou d'une protéine comprenant la préparation d'une banque de gènes mutés ou de protéines mutées selon la présente invention puis la sélection des gènes mutés ou des protéines mutées présentant la propriété recherchée.

La présente divulgation concerne un support solide portant une banque d'oligonucléotides comprenant des oligonucléotides complémentaires d'une ou plusieurs régions d'un ou de plusieurs gènes cibles et comprenant chacun, de préférence en leur milieu, une ou plusieurs mutations par rapport à la séquence du ou des gènes cibles. Dans un premier mode de réalisation préféré, la banque d'oligonucléotides contient les oligonucléotides portant les mutations permettant d'introduire l'ensemble des substitutions possibles sur chaque codon dudit ou desdits gènes cibles. Dans un second mode de réalisation préféré, la banque d'oligonucléotides contient les oligonucléotides portant les mutations permettant d'introduire un même acide aminé, de préférence une alanine, sur chaque codon dudit ou desdits gènes cibles. De préférence, les oligonucléotides de ladite banque sont liés au dit support solide par l'intermédiaire d'une molécule espaceur clivable. La molécule espaceur peut par exemple être clivable en milieur basique, par l'action de la lumière ou par action enzymatique. Dans un mode de réalisation particulier, ladite molécule espaceur est clivable en milieu basique. Par exemple, le milieu basique est une solution d'ammoniaque. Dans un mode de réalisation préféré, ladite molécule espaceur est le composé A. Dans ce mode de réalisation, ladite molécule espaceur est de préférence le composé B.

### DESCRIPTION DETAILLEE

Les puces à ADN sont constituées d'un support solide de quelques millimètres ou centimètres carrés sur lequel sont disposés, d'une façon ordonnée, un grand nombre d'ADN différents (Heller MJ et al. Annu Rev Biomed Eng. 2002;4:129-53). Les premières puces à ADN fonctionnelles ont été fabriquées de manière artisanale dans des laboratoires de biologie moléculaire. Dans ces premières expériences, les ADN fixés sur les puces étaient issus d'une synthèse biochimique, par exemple des fragments de PCR des ORFs du génome de levure (Schena M et al. Science. 1995. 270(5235): 467-70 ; Spellman PT et al. Mol Biol Cell 1998. 9(12):3273-97).

Aujourd'hui, dans le cas le plus courant, ces ADN sont des oligonucléotides, synthétisés chimiquement, d'une longueur de 5 à 200 bases, typiquement de 15 à 100 bases. Sur ces puces à oligonucléotides (appelées indifféremment « puces à ADN » ou simplement « puces » dans la suite du brevet), l'hybridation d'acides nucléiques de diverses origines (ADNc de différents tissus, ADN génomique, etc...) permet d'obtenir des informations, notamment dans le domaine de l'analyse du transcriptome et de la détection de polymorphismes (voir un ensemble de revues complètes dans Nature Genetics volume 32 supplement pp 461 - 552). Ces techniques sont maintenant utilisées en routine par un grand nombre de laboratoires de recherche ou de diagnostic médical dans le monde pour évaluer, de manière massivement parallèle et semi-quantitative, des concentrations d'acides nucléiques dans des mélanges d'acides nucléiques.

Deux grandes familles de technologies permettent de produire ces puces. Dans une première approche, les différents oligonucléotides sont synthétisés par voie chimique en utilisant des phosphoramidites et un synthétiseur d'oligonucléotides classique. Ces oligonucléotides sont ensuite déposés sur une lame au moyen, par exemple, de spotter ou de technologies de microfluidique assez comparables à celles utilisées dans les imprimantes à jet d'encre. Une seconde approche consiste à fabriquer les puces en synthétisant les oligonucléotides directement sur la lame. La synthèse parallèle et *in situ* d'un grand nombre d'oligonucléotides est rendue possible soit par une chimie particulière de couplage des nucléotides dépendant de la présence de lumière, soit par une chimie classique en adressant les nucléotides de manière bien localisée (par piezo, microvannes ou tout système de projection) sur des zones définies (WO 95/35505, WO 02/26373). Dans le premier mode de réalisation, une étape d'éclairage sélectif de certains des « pixels » composant la puce, en présence de l'une des quatre bases, permet d'accrocher, par une réaction photoactivée, ladite base sur certains seulement des oligonucléotides en cours de synthèse. A l'étape suivante, l'éclairage sélectif d'autres pixels, en présence d'une autre base, permettra d'allonger un autre sous-ensemble de ces oligonucléotides. Par exemple, voir EP 619 321.

Lors de la fabrication des puces à oligonucléotides, 10² à 10⁶ (typiquement : 10³ à 10⁵) oligonucléotides de séquence différente sont donc synthétisés en parallèle, à une très petite échelle de synthèse (moins d'une picomole dans la plupart des cas). Plusieurs techniques permettent de créer précisément un éclairage sélectif . Une première méthode utilise des masques fabriqués par photolithographie (Pease, AC et al. Proc. Natl Acad. Sci. USA, 91, 5022-5026 et brevets Affymetrix Inc.) qui coutent chers mais sont utiles lorsqu'on veut produire une grande série de puces identiques et offrent un excellent rapport de contraste. Une deuxième méthode utilise des micromiroirs digitaux (DMD ; Sangeet Singh-Gasson et al. Nat Biotech 1999 17 (10) : 974 - 978 ; LeProust E. et al. J. Comb. Chem., 2, 349-354 et WO9942813 WO0047548 ; US 6,271,957). Bien que le rapport de contraste soit plus faible, ce type de technique présente l'avantage d'une très grande souplesse, qui la destine particulièrement à la fabrication en petite série de puces à façons pour un prix raisonnable. D'autres techniques s'affranchissant de l'usage de masques permanents, et utilisant par exemple des écrans à cristaux liquides, ont été décrites (US 5,424,186). D'autres méthodes sont également décrites dans WO 02 26373 et WO 95/35505.

Cette approche miniaturisée et parallèle permet d'abaisser radicalement le coût de synthèse des oligonucléotides, sous réserve que ceux-ci puissent être utilisés sous forme d'un mélange où chacun n'est représenté qu'en petite quantité. Par synthèse chimique classique, le coût de la synthèse d'oligonucléotides est, en première approximation, proportionnel au nombre d'oligonucléotides et augmente avec leur longueur. Avec l'approche utilisant des puces, et sous les réserves précédemment exprimées, le coût de la synthèse d'un mélange d'oligonucléotides dépend uniquement de leur longueur et devient forfaitaire (à la puce). Aujourd'hui, le coût est de l'ordre de 2000 euros pour la synthèse d'une puce contenant 8000 oligonucléotides différents, d'une trentaine de bases chacun. A titre de comparaison, si l'on souhaite synthétiser individuellement ces 8000 oligonucléotides par l'approche classique, c'est-à-dire en utilisant un synthétiseur, le coût est d'environ 80 000 euros.

Ainsi, les mélanges d'oligonucléotides qui seraient générés en utilisant des puces d'ADN correspondraient à un coût pouvant être quarante fois inférieur au même mélange synthétisé en utilisant l'approche classique de synthèse individuelle d'oligonucléotides..

Plus précisément, le procédé est caractérisé par la succession des étapes suivantes :
a) On détermine une stratégie de mutagenèse pour un ou plusieurs gènes cible(s). Cette stratégie peut avoir pour objectif final soit d'améliorer certaines des propriétés dudit gène cible, soit d'obtenir des informations scientifiques .sur ce gène, notamment dans le but de caractériser les acides-aminés directement reliés à sa fonction. Pour un codon cible, une ou plusieurs mutations peuvent être déterminées.
b) A partir de cette stratégie, on conçoit une série d'oligonucléotides mutants. Chaque oligonucléotide contient une ou quelques mutations, de préférence localisées au niveau de son centre. Le nombre d'oligonucléotides mutants est généralement égal à la somme des mutations différentes à introduire au niveau de chacun des codons. Avantageusement, les oligonucléotides sont tous homologues au même brin de la matrice.
c) On synthétise les oligonucléotides mutants tels que conçus en b) en utilisant une approche de synthèse d'oligonucléotides sur puces. Préférentiellement, on aura, préalablement à l'étape de synthèse des oligonucléotides, déposé un composé chimique sur la puce, labile dans certaines conditions physico-chimiques, ayant fonction d'espaceur.
d) On libère les oligonucléotides de leur support, de façon à les mettre en solution. Préférentiellement, la libération des oligonucléotides est effectuée en appliquant les conditions physico-chimiques associées à la labilité de l'espaceur chimique. Chacun des oligonucléotides doit être présent en une quantité comprise entre 1 femtomole et 1 picomole.
e) Séparément, on prépare une quantité suffisante d'une ou plusieurs matrices (plasmides ou matrices linéaires, de préférence plasmides) contenant le ou les gènes cibles et éventuellement un, deux ou plusieurs marqueurs de sélection (par exemple, des gènes de résistance à des antibiotiques). De préférence, la matrice, de préférence le plasmide, contient également un gène de résistance à un antibiotique et le promoteur permettant l'expression de ce gène de résistance, une origine de réplication, et éventuellement un promoteur permettant l'expression du gène cible ainsi que toutes les séquences de maturation (Poly-A, signaux d'épissage, etc...) permettant d'optimiser l'expression d'une protéine mature, à partir du gène cible, dans l'organisme choisi.
f) On prépare un mélange réactionnel contenant la matrice préparée en e) et le mélange d'oligonucléotides obtenu en d).
g) On soumet le mélange réactionnel à une température élevée (supérieure à 80°C et de préférence voisine de 94°C), de façon à disposer temporairement d'ADN simple brin.
h) On abaisse la température à une valeur comprise entre 0 et 60°C, et de préférence entre 20 et 50°C, de façon à ce que chaque oligonucléotide présent dans le mélange se fixe au niveau de son site d'homologie sur le (ou l'un des) gène(s) cible(s).
i) On soumet le mélange réactionnel à une température compatible avec l'activité d'une ADN polymérase, ajoutée au mélange réactionnel avec une quantité suffisante de chacun des nucléotides triphosphates, les tampons, et les co-facteurs nécessaires. La réaction est menée pendant un temps suffisant à ce que la réplication complète de la matrice soit effectuée.
j) On utilise tout moyen approprié pour éliminer les matrices initiales et ainsi sélectionner les brins d'ADN néosynthétisés générés à l'issue de l'étape i). Avantageusement, cette étape de sélection se fait au moyen d'une enzyme de restriction spécifique des brins d'ADN méthylés, et de préférence appartenant au groupe des enzymes : DpnI, NanII, NmuDI ou NmuEI. Facultativement, on utilise le fragment d'ADN synthétisé en j) comme insert à insérer dans un plasmide préalablement linéarisé, par exemple en utilisant l'approche dite de « TA-cloning ».
k) On transforme, par exemple par électroporation ou par choc thermique, le mélange réactionnel obtenu en j) dans un organisme adapté, tel que des levures ou des bactéries rendues compétentes à la transformation.

Avantageusement, les oligonucléotides sont conçus, à l'étape b), de telle façon que tous les oligonucléotides homologues d'un même gène cible soient homologues du même brin dudit gène cible.

De préférence, la banque d'oligonucléotides est synthétisée sur un même support solide. Dans une autre alternative, les oligonucléotides de la banque présentant un A en 3' sont synthétisés sur un même support solide, ceux présentant un C en 3' sont synthétisés sur un autre support solide, ceux présentant un G en 3' sont synthétisés sur un encore autre support solide, et finalement ceux présentant un T en 3' sont synthétisés sur un autre support solide. Par banque d'oligonucléotides est entendu une composition comprenant au moins 2, 10, 20 ou 50 oligonucléotides différents. Cette banque d'oligonucléotides comprend de préférence plus de 50, 100, 200, 500, 1000, ou 5000 oligonucléotides différents. De préférence, le support solide est une puce. Dans un mode de réalisation, le support solide est du verre.

Dans un mode de réalisation préféré, un composé chimique jouant le rôle d'espaceur entre le support solide ou la lame et les oligonucléotides (un « espaceur ») est déposé sur le support solide ou la lame préalablement à la synthèse des oligonucléotides telle que décrite à l'étape c). Cet espaceur a par ailleurs la caractéristique d'être labile sous certaines conditions physico-chimiques. Par exemple, cet espaceur peut être clivable en milieu basique, par l'action de la lumière ou par action enzymatique. Par exemple, ce composé chimique peut être le composé A de formule :

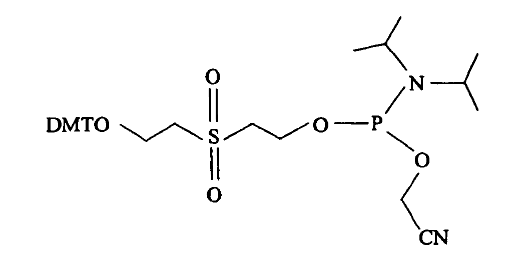

Ce composé est sensible à des conditions basiques.

Dans un autre exemple, ce composé chimique peut être le composé B de formule :

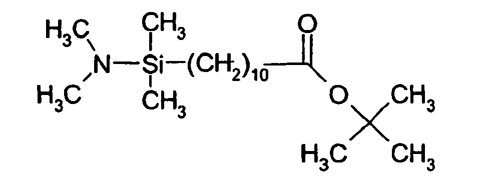

La liaison entre le composé et l'oligonucléotide est clivable à l'ammoniaque.

Dans un mode de réalisation préféré, la mise en solution des oligonucléotides à l'étape d) est réalisée en appliquant les conditions de labilité de espaceur chimique, par exemple des conditions basiques pour le composé A ou le composé B. Lorsque le composé A est utilisé, les oligonucléotides obtenus sont phosphorylés en 3'. Le procédé comprend facultativement une étape de « déprotection », c'est-à-dire d'élimination de ce groupement phosphate présent à l'extrémité 3'.

La quantité de matrice (de préférence plasmidique) de l'étape e) est préférentiellement comprise entre 10 ng et 100 µg, plus préférentiellement comprise entre 100 ng et 10 µg et de façon encore plus préférée comprise entre 100 ng et 1 µg.

De préférence, la matrice est un plasmide.

Dans un premier mode de réalisation, le mélange réactionnel de l'étape i) contient une polymérase thermosensible. Par exemple, et sans que cette liste puisse être considérée comme limitative, on utilise l'ADN polymérase de T4, de E. coli, ou encore le seul fragment de Klenow de la polymérase d'E. coli.

Dans un second mode de réalisation, le mélange réactionnel de l'étape i) contient une polymérase thermostable ayant ou non une fidélité de lecture particulière. Par exemple, et sans que cette liste puisse être considérée comme limitative, on utilise les polymérases Taq, Pfu, Vent, Pfx ou KOD. On peut également utiliser un mélange de deux ou plus de ces enzymes (par exemple 1 unité de Pfu polymérase et 5 unités de Taq polymérase).

Dans un mode de réalisation particulier, les étapes g), h), et i) sont réalisées plusieurs fois de façon à constituer plusieurs cycles de température. Dans ce cas, la polymérase utilisée est de préférence thermostable, afin de ne pas rendre nécessaire l'ajout de polymérase à chaque cycle.

Dans un mode de réalisation particulier, on ajoute dans le mélange réactionnel de l'étape i) une ligase, ainsi que les tampons et cofacteurs nécessaires. Dans ce cas, les oligonucléotides du mélange tel que décrit en d) intègrent en 5' un groupement acide phosphorique. Ce groupement acide phosphorique peut avoir été intégré directement pendant la synthèse des oligonucléotides. Préférentiellement, les oligonucléotides sont synthétisés normalement puis phosphorylés en 5' grâce à une kinase (par exemple, la T4 polynucleotide kinase), après leur synthèse.

Dans le cas où plusieurs cycles de température g), h), i) sont réalisés, et où la polymérase utilisée est thermostable, il est préférable d'utiliser une ligase également thermostable, de façon à ne pas rendre nécessaire l'ajout de cette enzyme à chaque cycle. Par exemple, et sans que cette liste puisse être considérée comme limitative, on utilise : la Taq Ligase, la Tth ligase ou la Amp ligase.

Dans le cas où un seul cycle de température est mené et où la polymérase utilisée est thermosensible, il est préférable d'utiliser une ligase également thermosensible, ou au moins partiellement active à la même température que la polymérase utilisée.

Le procédé peut en outre contenir l'étape suivante :
1) On étale les bactéries sur un milieu contenant un agent sélectif de façon à sélectionner les bactéries ayant intégré une matrice, de préférence un plasmide, contenant un gène cible potentiellement mutant.

Le procédé peut en outre contenir l'étape suivante :
m) On prélève individuellement les colonies bactériennes obtenues en 1) et on les utilise pour ensemencer un milieu nutritif sélectif.

Le procédé peut en outre contenir l'étape suivante :
n) A partir des différentes cultures réalisées en m), on réalise autant de préparations d'ADN, de préférence plasmidique, correspondant chacune à un clone isolé contenant un gène cible potentiellement muté en un ou plusieurs endroits.

Le procédé peut en outre contenir l'étape suivante :
o) On utilise la préparation d'ADN, de préférence plasmidique, obtenue en n) pour exprimer la protéine correspondante. Pour ce faire, on introduit l'ADN plasmidique dans un organisme adapté à l'expression, procaryote ou eucaryote. Par exemple, et sans que cette liste puisse être considérée comme limitative, on utilise une bactérie, une levure, un champignon, une cellule d'insecte, des cellules de plantes, des cellules de mammifères. L'expression peut être constitutive ou inductible (par exemple : par la température, un inducteur biochimique). Dans le cas d'une expression inductible, on se place dans des conditions qui permettent l'induction et l'expression. Alternativement, pour produire la protéine correspondante, on peut utiliser l'un des systèmes existants de transcription-traduction *in vitro* (Betton JM. Curr Protein Pept Sci. 2003. 4(1):73-80). Dans le cas où la traduction a lieu *in vitro,* une étape de maturation ou de repliement des protéines *in vitro* peut être ajoutée après leur synthèse (GAO YG et al. Biotechnol Prog. 2003. 19(3):915-20 ; Kosinski-Collins MS et al. Protein Sci. 2003. 12(3):480-90). Dans le cas où la traduction a lieu dans une cellule, comme dans le cas où la traduction a lieu *in vitro,* on peut, si l'on fait auparavant appel à un code génétique non standard lors de la conception des oligonucléotides servant à introduire les mutations, intégrer des acides aminés non naturels (Chin JW et al. Science. 2003. 301(5635):964-7; Hohsaka T et al. Nucleic Acids Res Suppl. 2003(3):271-2 ; Taki M et al. Nucleic Acids Res Suppl. 2001;(1):197-8; I Hirao et al. Nat Biotech. 20, 177 - 182).

Le procédé peut en outre contenir l'étape suivante :
p) On mesure directement ou indirectement l'activité (ou d'autres paramètres tels que la stabilité, la thermostabilité, la spécificité de substrat, l'activité en présence d'un inhibiteur, etc...) de la protéine obtenue par lyse ou sans lyse des cultures obtenues en m) ou en o), et on compare cette activité à celle de la protéine produite dans les mêmes conditions à partir de l'ADN, de préférence plasmidique, contenant le gène cible non mutant. Lorsque l'observation de ces mesures fait apparaître une différence jugée significative, les molécules mutantes sont éventuellement séquencées afin de mettre en évidence la position de la mutation à l'origine de cette modification d'activité.

Dans un mode de réalisation particulier, la banque générée par le procédé est soumise à une technique dite de sélection, où les produits génétiques (phénotypes), préalablement liés aux acides nucléiques qui les codent (génotypes) sont triés tous en même temps (en masse). Dans ce cas les premières étapes a) à k) du procédé restent inchangées mais les étapes 1), m), n), o) et p) décrites plus haut sont supprimées et remplacées par les étapes suivantes :
l') les cellules issues de l'étape k) sont mises en culture liquide dans un milieu de sélection adapté.
m') une méthode de sélection existante est utilisée. Par exemple, et sans que cette liste puisse être considérée comme exhaustive, la survie des cellules transformées sur certains milieux minimum peut être utilisées, on peut également utiliser le « phage display », le « cell-surface display », le « ribosome display », le fusion mRNA-peptide, la sélection en émulsion ou le test de complémentation de fragment de protéine.
n') les acides nucléiques sélectionnés sont, si besoin est, reclonés dans le plasmide initial puis les plasmides sont réutilisés à l'étape f) d'une nouvelle réalisation du procédé. Alternativement, ces acides nucléiques sont soumis à un criblage secondaire et/ou séquencés.

Dans un mode de réalisation particulier, on utilise à l'étape f) non pas la matrice préparée en e) mais de l'ADN, de préférence plasmidique, préparé à partir des cellules transformées à l'étape k) lors d'une précédente réalisation du procédé. On peut ainsi réaliser plusieurs (typiquement : 2 à 20) tours successifs de mutagenèse ; à chaque tour, le pourcentage de gènes mutants dans la banque ainsi que le nombre moyen de mutations par molécule augmente.

Dans un mode de réalisation particulier, on utilise à l'étape f) non pas la matrice préparée en e) mais de l'ADN, de préférence plasmidique, préparé à partir de cellules qui ont exprimé une activité protéique jugée améliorée à l'étape p) lors d'une précédente réalisation du procédé. Les mutations à introduire sur ces molécule déjà mutées et déjà améliorées, peuvent être identiques ou non aux mutations introduite lors de la première réalisation du procédé. On peut ainsi réaliser plusieurs tours d'évolution moléculaire par mutation - sélection (ou criblage).

Dans un mode de réalisation particulier, le procédé est caractérisé par l'évolution non pas de protéines mais d'un ou plusieurs acides nucléiques (ADN ou ARN).

Dans un mode de réalisation particulier, les oligonucléotides sont conçus de manière à introduire non pas des substitutions ponctuelles mais des délétions de quelques bases (1 à 20, typiquement 1 à 9) ou des insertions de quelques bases (1 à 20, typiquement 1 à 9).

### ** MODE DE RÉALISATION #1 : HAUTE TEMPERATURE **

Dans un premier mode de réalisation, une banque de mutants combinatoire est créée à partir d'un gène et le procédé de l'invention est caractérisé par la succession des étapes suivantes :
a) On détermine une stratégie de mutagenèse pour un gène cible constitué de n codons, avec n de préférence compris entre 50 et 5000. Cette stratégie peut concerner soit l'ensemble des n codons du gène cible, soit seulement une partie de ces n codons.
b) A partir de cette stratégie, on conçoit une série d'oligonucléotides mutants, d'une taille de préférence comprise entre 15 et 45 nucléotides et chacun homologues à une région du gène cible.
c) On synthétise les oligonucléotides mutants correspondants tels que conçus en b) en utilisant une approche de synthèse d'oligonucléotides sur puces.
d) On libère les oligonucléotides de leur support, de façon à obtenir un mélange d'oligonucléotides en solution.
e) Indépendamment, on prépare une matrice, de préférence un plasmide, contenant le gène cible, en utilisant un système de préparation adapté (systèmes de mini-midi-, ou maxi-prep commercialisés par des sociétés spécialisées (Qiagen, Macherey-Nagel, etc...).
f) On prépare un mélange réactionnel contenant la matrice préparée en e) et le mélange d'oligonucléotides obtenu en d), à une concentration telle que le rapport entre le nombre de molécules de matrice et le nombre de molécules de chacun des oligonucléotides mutants soit compris entre 0,01 et 100, et de préférence entre 0,1 et 10. On ajoute une polymérase thermostable, et tous les éléments nécessaires pour réaliser une réaction de réplication de la matrice à partir des oligonucléotides mutants : le tampon permettant son activité, des nucléotides triphosphates en quantité suffisante, les éventuels cofacteurs nécessaires.
g) On soumet le mélange à une température élevée (supérieure à 80°C et de préférence voisine de 94°C), pendant au moins une seconde et de préférence de l'ordre de une minute, de façon à disposer temporairement d'ADN simple brin.
h) On abaisse la température à une valeur comprise entre 0 et 60 °C et de préférence comprise entre 20 et 50 °C, pendant au moins une seconde et de préférence de l'ordre de une minute, de façon à ce que les oligonucléotides présents dans le mélange se fixent sur leur site d'homologie au niveau de leur gène cible.
i) On soumet le mélange réactionnel à une température d'environ 68 à 72°C, ce qui permet l'activité optimale de la polymérase, pendant un temps suffisant pour que la réplication complète de la matrice, de préférence du plasmide, puisse être effectuée, et calculé en fonction de la vitesse de synthèse de la polymérase (en bases par minute) et de la taille de la matrice, de préférence du plasmide, contenant le gène cible.
   On répète les étapes g), h), et i), de préférence en utilisant un thermocycleur, de façon à ce que les cycles de températures puissent être effectués automatiquement.
j) On utilise tout moyen approprié pour sélectionner les brins d'ADN néosynthétisés générés lors de la l'étape g), des matrices initiales.
k) On transforme le mélange réactionnel obtenu en j) dans un organisme adapté, tel que levures ou bactéries, rendues compétentes à la transformation.

A l'étape f), on ajoute éventuellement une ligase thermostable, comme par exemple la Taq Ligase ou la Tth ligase. Avantageusement dans ce cas, les oligonucléotides auront été phosphorylés en 5' au moyen d'une kinase préalablement à

Le procédé selon ce mode de réalisation peut en outre contenir une ou plusieurs des étapes l), m), n), o), ou p) décrites précédemment.

### ** MODE DE RÉALISATION #2 : BASSE TEMPERATURE **

Dans un second mode de réalisation, le procédé de l'invention est caractérisé par la succession des étapes suivantes :
On détermine une stratégie, on conçoit des oligonucléotides, on les synthétise sur support solide, on les libère et, indépendamment, on prépare une quantité suffisante de matrice contenant le gène cible, tel que décrit en a), b), c), d), et e) de l'exemple précédent. Les étapes suivantes sont :
f) On prépare un mélange réactionnel contenant la matrice préparée en e) et le mélange d'oligonucléotides obtenu en d), à une concentration telle que le rapport entre le nombre de molécules de matrice et le nombre de molécules de chacun des oligonucléotides mutants soit compris entre 0,01 et 100, et de préférence entre 0,1 et 10.
g) On soumet le mélange à une température élevée (supérieure à 80°C et de préférence voisine de 94°C), pendant au moins une seconde et de préférence de l'ordre de une minute, de façon à disposer temporairement d'ADN simple brin.
h) On abaisse la température à une valeur comprise entre 0 et 60 °C et de préférence comprise entre 20 et 50 °C, pendant au moins une seconde et de préférence de l'ordre de une minute, de façon à ce que les oligonucléotides présents dans le mélange se fixent sur leur site d'homologie au niveau de leur gène cible.
i) On ajoute une polymérase thermosensible, par exemple la T4 polymérase, et tous les éléments nécessaires pour réaliser une réaction de réplication de la matrice à partir des oligonucléotides mutants : le tampon permettant son activité, des nucléotides triphosphates en quantité suffisante, les éventuels cofacteurs nécessaires. On soumet le mélange réactionnel à une température d'environ 37°C, ce qui permet l'activité optimale de la T4 polymérase, pendant un temps suffisant pour que la réplication complète du plasmide puisse être effectuée, et calculé en fonction de la vitesse de synthèse de la polymérase (en bases par minute) et de la taille du plasmide contenant le gène cible.

Eventuellement, on répète les étapes g), h) et i) une ou plusieurs fois.
j) On utilise tout moyen approprié pour sélectionner les brins d'ADN néosynthétisés générés lors de la l'étape g), des matrices initiales.
k) On transforme le mélange réactionnel obtenu en j) dans un organisme adapté, tel que levures ou bactéries, rendues compétentes à la transformation.

A l'étape f), on ajoute éventuellement une ligase de préférence thermosensible et en tous cas active à la température d'activité de la polymérase utilisée, comme par exemple la T4 ligase. Avantageusement dans ce cas, les oligonucléotides auront été phosphorylés en 5' au moyen d'une kinase préalablement à leur utilisation.

Dans un mode de réalisation particulier, on utilise à l'étape f) non pas la matrice préparée en e) mais de l'ADN, de préférence plasmidique, préparé à partir des cellules transformées à l'étape k). On peut ainsi réaliser deux ou plusieurs tours successifs de mutagenèse ; à chaque tour, le pourcentage de gènes mutants dans la banque ainsi que le nombre moyen de mutations par molécule augmente.

Le procédé selon ce mode de réalisation peut en outre contenir une ou plusieurs des étapes l, m, n, o, ou p décrites précédemment.

### ** MODE DE REALISATION #3 : FAUX MULTI-GENE **

Dans un mode de réalisation particulier, des oligonucléotides correspondant à plusieurs gènes sont simultanément synthétisés, puis ces oligonucléotides dont séparés (par exemple, par chromatographie ou par electrophorèse capillaire, sur la base de leur masse, si on choisit des oligonucléotides de longueur différente pour chaque gène, par exemple des oligonucléotides de longueur 18 pour le gène 1, 20 pour le gène 2, 22 pour le gène 3, ..., 36 pour le gène 10). Ces différents mélanges d'oligonucléotides peuvent ensuite être utilisés normalement dans l'un des modes de réalisation décrit plus haut.

### ** MODE DE RÉALISATION #4 : MULTI-GÈNES EN POOL **

Dans un quatrième mode de réalisation, plusieurs gènes sont mutés simultanément au sein d'un unique mélange réactionnel contenant tous les oligonucléotides permettant d'introduire les mutations souhaitées dans l'ensemble des gènes. Le procédé est caractérisé par la succession des étapes suivantes :
a) On s'intéresse à un ensemble de gènes cibles Gᵢ, avec *i* variant de 1 à *g,* et g de préference compris entre 2 et 1000. Chacun de ces gènes cibles *Gᵢ* est constitué de *nᵢ* codons, avec nᵢ de préférence compris entre 50 et 5000. Pour chaque gène *Gᵢ,* on élabore une stratégie de mutagenèse. Pour chaque gène Gᵢ, la stratégie correspondante peut concerner soit l'ensemble des *nᵢ* codons, soit seulement une partie de ces codons.
b) A partir de chaque stratégie, on conçoit pour chaque gène *Gᵢ* correspondant une série d'oligonucléotides mutants, d'une taille de préférence comprise entre 15 et 45 nucléotides et chacun homologues à une région du gène Gᵢ. Il est possible que les séquences de deux gènes ou plus aient un degré de similitude élevé en certaines régions et donc qu'un certain nombre d'oligonucléotides conçus pour introduire des mutations sur l'un de ces gènes s'hybride non pas seulement sur le gène souhaité mais également sur un ou plusieurs autres gènes, créant par là même des mutations non désirées. Ce mode de réalisation suppose donc d'éviter de mélanger des gènes présentant des similitudes de séquence très élevées et, dans tous les cas, de tenir compte de possibles phénomènes d'hybridations croisées dans la conception des oligonucléotides. Pour aider à la conception des oligonucléotides dans ce mode de réalisation du procédé, il est possible d'utiliser les algorithmes et les logiciels existants pour optimiser la séquence des oligonucléotides et éviter ces phénomènes d'hybridation croisée. Ces logiciels, actuellement dédiés à la conception de puces à oligonucléotides pour l'analyse du transcriptome ou à de PCR en multiplex sont utilisables en l'état, avec des adaptations mineures (voir par exemple Emrich SJ Nucleic Acids Res. 2003. 31(13):3746-50 ; Xu D Bioinformatics. 2002. 18(11):1432-7).
c) On synthétise sur une puce les oligonucléotides mutants correspondants tels que conçus en b).
d) On libère les oligonucléotides de leur support, de façon à obtenir un mélange d'oligonucléotides en solution.
e) Indépendamment, on prépare séparement chacunes des *g* matrices, de préférence des plasmides, contenant chacune un des gènes cibles *Gᵢ.* La quantité de chaque matrice, de préférence de chaque plasmide, préparée est de préférence comprise entre 10 ng et 10 µg. Dans un mode de réalisation particulier, chaque matrice contient plusieurs agents de sélection, de manière à pouvoir, avec un milieu de sélection adapté, faire pousser les clones contenant cette matrice, à l'exclusion de tous les autres clones. (Par exemple, on peut récupérer sélectivement n'importe quelle matrice parmi quatre si on introduit dans leur séquence les marqueurs suivants : chloramphénicol et ampiciline pour le premier, chloramphénicol, ampiciline et tétracycline pour le second, chloramphénicol et tétracycline pour le troisième, chloramphénicol seul pour le quatrième).
f) On prépare un mélange réactionnel contenant l'ensemble des matrices préparées en e) et le mélange d'oligonucléotides obtenu en d), à des concentrations telles que le rapport entre le nombre de molécules de matrice et le nombre de molécules de chacun des oligonucléotides mutants correspondants soit compris, pour chaque matrice entre 0,01 et 100, et de préférence entre 0,1 et 10. On ajoute une polymérase thermostable et tous les éléments nécessaires pour réaliser une réaction de réplication de la matrice à partir des oligonucléotides mutants : le tampon permettant son activité, des nucléotides triphosphates en quantité suffisante, les éventuels cofacteurs nécessaires.
g) On soumet le mélange à une température élevée (supérieure à 80°C et de préférence voisine de 94°C), pendant au moins une seconde et de préférence de l'ordre de une minute, de façon à disposer temporairement d'ADN simple brin.
h) On abaisse la température à une valeur comprise entre 0 et 60 °C et de préférence comprise entre 20 et 50 °C, pendant au moins une seconde et de préférence de l'ordre de une minute, de façon à ce que les oligonucléotides présents dans le mélange se fixent sur leur site d'homologie au niveau de leur gène cible.
i) On soumet le mélange réactionnel à une température d'environ 68 à 72°C, ce qui permet l'activité optimale de la polymérase, pendant un temps suffisant pour que la réplication complète de la matrice, de préférence du plasmide, puisse être effectuée, et calculé en fonction de la vitesse de synthèse de la polymérase (en bases par minute) et de la taille du plasmide contenant le gène cible.
   On répète les étapes g), h), et i), de préférence en utilisant un thermocycleur, de façon à ce que les cycles de températures puissent être effectués automatiquement.
j) On utilise tout moyen approprié pour sélectionner les brins d'ADN néosynthétisés générés lors de la l'étape g), des matrices initiales.
k) On transforme le mélange réactionnel obtenu en j) dans un organisme adapté, tel que levures ou bactéries, rendues compétentes à la transformation.

A l'étape f), on ajoute éventuellement une ligase thermostable, comme par exemple la Taq Ligase ou la Tth ligase. Avantageusement dans ce cas, les oligonucléotides auront été phosphorylés en 5' au moyen d'une kinase préalablement à leur utilisation.

Le procédé selon ce mode de réalisation contient en outre une ou plusieurs étapes d'individualisation des différents gènes *Gᵢ.*

Une possibilité est l'étalement sur une boite de culture contenant un milieu sélectif des cellules de l'étape k), suivi par le repiquage en milieu liquide de ces clones ou d'une partie d'entre eux suivi par une réaction de PCR sur chacune de ces cultures en utilisant un ensemble d'oligonucléotides conçus de façon à ce que la taille du produit obtenu indique le gène porté par le plasmide du clone correspondant.

Alternativement, les cellules de l'étape k) sont étalées sur une boite de culture contenant un milieu sélectif, repiquées en milieu liquide et chacune des cultures est soumise à un ensemble de *g* PCR utilisant chacune deux oligonucléotides conçus de façon à ce que, pour chaque clone, l'existence d'un produit dans une des *g* réactions de PCR, et d'aucun produit dans les (*g*-1) autres réactions indique le gène porté par le plasmide du clone correspondant.

Alternativement, les cellules de l'étape k) sont mises en culture toutes ensembles dans un milieu liquide sélectif, *g* réactions de PCR de type PCR préparative sont ensuite réalisées à partir de ces cultures de façon à amplifier à chaque fois une portion de la séquence des plasmides correspondant à un seul des *g* gènes. Les g produits de PCR sont ensuite indépendamment purifiés (par exemple avec un kit utilisant une colonne ou après dépôt sur gel avec un kit adapté) et on obtient sous forme linéaire les g banques correspondant chacune à l'un des *g* gènes qui ont été mutés. Ces banques linéaires sont indépendamment clonées par les méthodes classiques dans les plasmides initiaux ou dans d'autres plasmides adaptés puis transformées, étalées sur milieu solide de façon à obtenir des clones indépendants sont ensuite soumis à un criblage. Ces banques linéaires peuvent de manière alternative être indépendamment clonées puis transformées, exprimées et soumises à une sélection.

Alternativement, dans le cas où les plasmides utilisés à l'étape e) contiennent chacun un ensemble de marqueurs de sélection combinés de façon unique, on étale les cellules de l'étape k) sur *g* boites de cultures différentes contenant chacune une combinaison d'agents de sélection permettant de ne laisser pousser uniquement les cellules contenant une combinaison particulière de marqueurs de sélection et donc conduisant à l'obtention dans chaque boite de clones contenant un seul des gènes *Gᵢ.*

Alternativement, les cellules de l'étape k) sont étalées sur une boite de culture contenant un milieu sélectif, chacun des clones indépendants obtenus est repiqué en milieu liquide, on prépare l'ADN plasmidique de chacune de ces cultures et on le séquence. Grâce au résultat du séquencage, on sait pour chaque clone quel gène parmi les g est présent et on a des informations sur tout ou partie des mutations introduites dans la séquence de ce gène. Les cultures réalisées avant le séquencage de chaque clone sont ensuite utilisées pour un test de criblage et on a donc accès à un ensemble de données du type (gène muté, séquence, résultat au test de criblage). Les cultures réalisées avant séquençage peuvent également être mélangées en fonction du gène qu'elles contiennent tel qu'indiqué par le séquencage afin de récupérer des banques correspondant à chacun des gènes, qui peuvent ensuite être soumises à un criblage ou à une sélection.

Alternativement, l'ADN plasmidique de l'ensemble des cellules de l'étape k) est isolé puis soumis en parallèle à *g* digestions enzymatiques multiples *Rᵢ* (*i*=1,2...*g*) par des enzymes de restriction. Chaque réaction *Rᵢ* (*i*=1,2...*g*) est conçue afin de linéariser à chaque fois tous les plasmides sauf les plasmides contenant le gène *Gᵢ.* Après chaque réaction *Rᵢ* (*i*=1,2...*g*), les plasmides sont utilisés pour transformer des cellules de bactéries ou de levure et seuls les plasmides circulaires, donc seuls les plasmides contenant des versions du gène *Gₗ* sont transformés efficacement. Cette approche peut être possible ou non suivant le type de plasmide et de gène utilisé. Cette approche s'inspire directement de la digestion multiple différentielle (WO9928451).

Dans un mode de réalisation particulier, on utilise à l'étape f) non pas les matrices préparées en e) mais un mélange d'ADN, de préférence plasmidique, préparé à partir des cellules transformées à l'étape k). On peut ainsi réaliser successivement deux tours ou plus de mutagenèse ; à chaque tour, on augmente, pour chaque gène le pourcentage de gènes mutants dans la banque correspondante ainsi que le nombre moyen de mutations par molécule.

### ** MODE DE RÉALISATION #5 : MULTI-GÈNES EN PARALLELE **

Dans un cinquième mode de réalisation, plusieurs gènes sont mutés de manière indépendantes en parallèle, mais les oligonucléotides permettant d'introduire des mutations dans un ensemble de *g* gènes (*g* étant typiquement compris entre 2 et 1000, de préférence entre 2 et 50) sont synthétisés simultanément sur la même puce.

Dans ce mode de réalisation, le procédé est caractérisé par la succession des étapes suivantes :
a) On s'intéresse à un ensemble de gènes cibles Gᵢ (*i*=1,2,...*g*) avec g compris entre 2 et 1000. Chacun de ces gènes cibles *Gᵢ* est constitué de *nᵢ* codons, avec *nᵢ* de préférence compris entre 50 et 5000. Pour chaque gène *Gᵢ,* on élabore une stratégie de mutagenèse. Pour chaque gène *Gᵢ,* la stratégie correspondante peut concerner soit l'ensemble des *nᵢ* codons, soit seulement une partie de ces codons.
b) A partir de chaque stratégie, on conçoit, pour chaque gène *Gᵢ* correspondant une série d'oligonucléotides mutants, d'une taille de préférence comprise entre 15 et 45 nucléotides et chacun homologue à une région du gène *Gᵢ.* Il est possible que les séquences de deux ou plusieurs gènes aient un degré de similitude élévé en certaines régions et donc qu'un certain nombre d'oligonucléotides conçus pour introduire des mutations sur l'un de ces gènes s'hybride non pas seulement sur le gène souhaité mais également sur un ou plusieurs autres gènes, créant par la même des mutations non désirées. Ce mode de réalisation suppose donc d'éviter de chercher à muter des gènes présentant des similitudes de séquence très élevées et dans tous les cas de tenir compte de possibles phénomènes d'hybridation croisées dans la conception des oligonucléotides. Les algorithmes et les logiciels existants pour optimiser la séquence des oligonucléotides et éviter ces phénomènes d'hybridation croisée lors de la conception de puces à oligonucléotides pour l'analyse du transcriptome ou lors de PCR en multiplex sont utilisables, avec des adaptations mineures pour aider à la conception des oligonucléotides dans ce mode de réalisation du procédé (par exemple : Emrich SJ Nucleic Acids Res. 2003 Jul 1;31(13):3746-50 ; Xu D Bioinformatics. 2002 Nov;18(11):1432-7). Les oligonucléotides correspondant à chaque gène peuvent avoir des longueurs différentes ou non entre eux et différentes ou non des longueurs des oligonucléotides correspondant aux autres gènes.
c) On synthétise sur une puce à ADN les oligonucléotides mutants correspondants tels que conçus en b).
d) On libère les oligonucléotides de leur support, de façon à obtenir un mélange d'oligonucléotides en solution.
e) Indépendamment, on prépare séparément chacunes des g matrices, de préférence des plasmides, contenant chacun l'un des gènes cibles *Gᵢ.* De préférence, matrice contient également un gène de résistance à un antibiotique et le promoteur permettant l'expression de ce gène de résistance, une origine de réplication, et éventuellement un promoteur permettant l'expression du gène cible ainsi que toutes les séquences de maturation (Poly-A, signaux d'épissage, etc...) permettant d'optimiser l'expression d'une protéine mature, à partir du gène cible, dans l'organisme choisi.
f) On prépare g mélanges réactionnels. Le mélange réactionnel *Mᵢ* contient la matrice, de préférence le plasmide, porteur du gène *Gᵢ* et le mélange d'oligonucléotides obtenu en d), à une concentration telle que le rapport entre le nombre de molécules de matrice et le nombre de molécules de chacun des oligonucléotides mutants soit compris entre 0,01 et 100, et de préférence entre 0,1 et 10. On ajoute une polymérase thermostable, et tous les éléments nécessaires pour réaliser une réaction de réplication de la matrice à partir des oligonucléotides mutants : le tampon permettant son activité, des nucléotides triphosphates en quantité suffisante, les éventuels cofacteurs nécessaires.
g) On soumet indépendamment chacun des mélanges *Mᵢ* à une température élevée (supérieure à 80°C et de préférence voisine de 94°C), pendant au moins une seconde et de préférence de l'ordre de une minute, de façon à disposer temporairement d'ADN simple brin.
h) On abaisse la température à une valeur comprise entre 0 et 60 °C et de préférence comprise entre 20 et 50 °C, pendant au moins une seconde et de préférence de l'ordre de une minute, de façon à ce que les oligonucléotides présents dans les mélanges se fixent sur leur site d'homologie au niveau de leur gène cible.
i) On soumet chacun des mélanges réactionnels *Mᵢ* à une température d'environ 68 à 72°C, ce qui permet l'activité optimale de la polymérase, pendant un temps suffisant pour que la réplication complète de la matrice, de préférence du plasmide, puisse être effectuée, et calculé en fonction de la vitesse de synthèse de la polymérase (en bases par minute) et de la taille du plasmide contenant le gène cible.
   On répète les étapes g), h), et i), de préférence en utilisant un thermocycleur, de façon à ce que les cycles de températures puissent être effectués automatiquement.
j) On utilise tout moyen approprié pour sélectionner les brins d'ADN néosynthétisés générés lors de la l'étape i), des matrices initiales. Avantageusement, cette étape de sélection se fait au moyen d'une enzyme de restriction spécifique des brins d'ADN méthylés, et de préférence appartenant au groupe des enzymes Dpnl, NanII, NmuDI ou NmuEI.
k) On transforme les mélanges réactionnels obtenus en j) dans un organisme adapté, tel que levures ou bactéries, rendues compétentes à la transformation.

A l'étape f), on ajoute éventuellement une ligase thermostable, comme par exemple la Taq Ligase ou la Tth ligase. Avantageusement dans ce cas, les oligonucléotides auront été phosphorylés en 5' au moyen d'une kinase préalablement à leur utilisation.

Dans un mode de réalisation particulier, on utilise à l'étape f) non pas les matrices préparées en e) mais des ADN, de préférence plasmidiques, préparés à partir des cellules transformées à l'étape k). On peut ainsi réaliser deux ou plusieurs tours successifs de mutagenèse ; à chaque tour, on augmente pour chaque gène le pourcentage de gènes mutants dans la banque correspondante ainsi que le nombre moyen de mutations par molécule.

### ** MODE DE RÉALISATION #6 : MUTAGENÈSE ET SÉLECTION PAR PLASMID DISPLAY **

Dans un sixième mode de réalisation, une banque de mutants combinatoire est créée à partir d'un gène et cette banque est sélectionnée par « plasmid display » (Speight RE et al. Chem Biol. 2001 8(10):951-65 ; Zhang Y et al J Biochem (Tokyo). 2000 Jun;127(6):1057-63; Cull MG et al. Proc Natl Acad Sci U S A. 1992 Mar 1;89(5):1865-9).

Dans ce mode de réalisation, le procédé est caractérisé par la succession des étapes suivantes :
a) On détermine une stratégie de mutagenèse pour un gène cible constitué de n codons, avec *n* de préférence compris entre 50 et 5000. Cette stratégie peut concerner soit l'ensemble des *n* codons du gène cible, soit seulement une partie de ces *n* codons.
b) A partir de cette stratégie, on conçoit une série d'oligonucléotides mutants, d'une taille de préférence comprise entre 15 et 45 nucléotides et chacun homologue à une région du gène cible.
c) On synthétise les oligonucléotides mutants correspondants tels que conçus en b) en utilisant n'importe laquelle des techniques de synthèse sur puce.
d) On libère les oligonucléotides de leur support, de façon à obtenir un mélange d'oligonucléotides en solution.
e) Indépendamment, on prépare une matrice, de préférence un plasmide, contenant le gène cible. La matrice contient également, accolé au gène cible (en amont ou en aval) et sous le contrôle du même promoteur (de manière à produire une protéine de fusion), le gène codant une protéine P¹ qui a la propriété de reconnaître certaines séquences courtes d'ADN et de s'y fixer avec une forte affinité. Le plasmide contient également une séquence d'ADN qui fait partie des séquences reconnues par P¹.
f) On prépare un mélange réactionnel contenant la matrice préparée en e) et le mélange d'oligonucléotides obtenu en d), à une concentration telle que le rapport entre le nombre de molécules de matrice et le nombre de molécules de chacun des oligonucléotides mutants soit compris entre 0,01 et 100, et de préférence entre 0,1 et 10. On ajoute une polymérase thermostable, et de tous les éléments nécessaires pour réaliser une réaction de réplication de la matrice à partir des oligonucléotides mutants : le tampon permettant son activité, des nucléotides triphosphates en quantité suffisante, les éventuels cofacteurs nécessaires.
g) On soumet le mélange à une température élevée (supérieure à 80°C et de préférence voisine de 94°C), pendant au moins une seconde et de préférence de l'ordre de une minute, de façon à disposer temporairement d'ADN simple brin.
h) On abaisse la température à une valeur comprise entre 0 et 60°C et de préférence comprise entre 20 et 50 °C, pendant au moins une seconde et de préférence de l'ordre de une minute, de façon à ce que les oligonucléotides présents dans le mélange se fixent sur leur site d'homologie au niveau de leur gène cible.
i) On soumet le mélange réactionnel à une température d'environ 68 à 72°C, ce qui permet l'activité optimale de la polymérase, pendant un temps suffisant pour que la réplication complète de la matrice, de préférence du plasmide, puisse être effectuée, et calculé en fonction de la vitesse de synthèse de la polymérase (en bases par minute) et de la taille du plasmide contenant le gène cible.
   A l'étape f), on ajoute éventuellement une ligase thermostable, comme par exemple la Taq Ligase ou la Tth ligase. Avantageusement dans ce cas, les oligonucléotides auront été phosphorylés en 5' au moyen d'une kinase préalablement à leur utilisation.
   On répète les étapes g), h), et i), de préférence en utilisant un thermocycleur, de façon à ce que les cycles de températures puissent être effectués automatiquement.
j) On utilise tout moyen approprié pour sélectionner les brins d'ADN néosynthétisés générés lors de la l'étape g), des matrices initiales.
k) On transforme le mélange réactionnel obtenu en j) dans un organisme adapté, tel que levures ou bactéries, rendues compétentes à la transformation.
l) On met en culture liquide, éventuellement avec un agent de sélection adapté, les cellules transformées à l'étape k). On se place dans des conditions (notamment : de température) permettant l'expression de la protéine de fusion protéine d'interet-P¹.
m) On extrait les matrices, de préférence les plasmides (génotype), auxquels sont fixés la protéine P¹ et donc indirectement la protéine cible (phénotype). On se place dans des conditions (notamment : de salinité) où le lien entre P¹ et le plasmide est conservé.
n) On réalise la sélection proprement dite : les complexes matrice- P¹-protéine d'intérêt sont mis en présence de billes à la surface desquelles est fixé le ligand L¹ (alternativement, des plaques sur lesquelles ce ligand a été adsorbé sont utilisées). Les plasmides codant une protéine ayant une forte affinité pour L¹ sont accrochés aux billes ; les autres plasmides restent libres en solution. Les billes sont isolées par centrifugation (alternativement, des billes magnétiques sont utilisées) et rincées plusieurs fois avec un milieu adéquat.
o) On récupère les billes rincées et on les place dans des conditions (notamment : de salinite) dans lesquelles le lien entre P¹ et le plasmide n'est plus assuré. On centrifuge et on récupère le surnageant. On extrait l'ADN présent dans le surnageant (avec un kit adapté ou une méthode connue, par exemple : extraction au phenolchloroforme).
p) On recommence autant de fois que nécessaire (entre 0 et 100 fois ; généralement 2 à 20 fois) l'ensemble des étapes g) à o) du procédé suivant ce mode de réalisation. Les matrices récupérés à l'étape o) d'une itération du protocole sont utilisés à l'étape g) de l'itération suivante.

Dans un mode de réalisation particulier, la méthode de sélection utilisée n'est pas le « plasmid display » mais le « cell-surface display » (Lee Sy et al. Trends Biotechnol. 2003 Jan;21(1):45-52). Dans ce cas, une matrice adaptée, de préférence un plasmide, est utilisée à l'étape e), la protéine d'intérêt est exprimée fusionnée avec une protéine transporteuse qui va s'ancrer dans la membrane cytoplasmique ou la membrane externe de bactéries gram négatives ou dans la paroi de bactéries gram positives (US5874267, US6274345, US535697), WO9324636, WO950479, WO9735022, WO9410330, WO9310214, WO9737025, WO9967366, WO0246388, WO006010, WO9709437, US5616686, WO9318163, US5958736, WO9640943 et US5821088). Alternativement, la protéine d'intérêt est exprimée fusionnée avec une protéine transporteuse qui va s'ancrer dans la paroi d'une cellule de levure. Dans ces cas, les étapes a) à d) sont identiques, l'étape e) devient :
'e') Indépendamment, on prépare une matrice contenant le gène cible. La matrice, de préférence un plasmide, contient également, accolé au gène cible (en amont
ou en aval) et sous le contrôle du même promoteur (de manière à produire une protéine de fusion), le gène codant une protéine P² qui a la propriété d'être adressée, *in vivo,* vers la surface cellulaire puis fixée à cette surface, exposant la protéine d'intérêt vers l'exterieur de la cellule.

Puis les étapes f) à k) sont identiques et les étapes suivantes sont supprimées et remplacées par :
'l') On met en culture liquide, éventuellement avec un agent de sélection adapté, les cellules transformées à l'étape k). On se place dans des conditions (notamment : de température) permettant l'expression de la protéine de fusion protéine d'intérêt-P² à la surface cellulaire.
'm') Par un système de sélection adapté (par exemple : microbilles greffées, microbilles magnétiques greffées, FACS, microFACS), on isole la sous population des cellules qui exposent à leur surface une protéine d'intérêt possédant une affinité voulue pour un ligand adapté à la propriété que l'on souhaite améliorer. Par exemple, et sans que cette liste puisse être considérée comme exhaustive, le ligand est un antigène, un substrat ou un complexe de transition.
'n') On prépare l'ADN plasmidique des cellules sélectionnées. Cette préparation d'ADN est enrichie en plasmides contenant un gène codant une protéine améliorée. Ces plasmides sont transformés dans des bactéries, les bactéries transformées sont mises en culture sur un milieu solide contenant un agent de sélection adapté, une fraction ou la totalité des clones individuels obtenus est mise en culture en milieu liquide et chaque clone subit un test de criblage. L'ADN plasmidique des clones jugés améliorés à l'issue du test de criblage est séquence. Alternativement, on prépare l'ADN plasmidique des cellules sélectionnées et on réalise de nouveau les étapes f), g),h), i), j, k), l'), m') et n') du procédé en utilisant cet ADN à la place des plasmides préparés en e). On réalise ainsi plusieurs tours successifs d'évolution moléculaire par mutation-sélection.

Dans un mode de réalisation particulier, le procédé est adapté de manière évidente pour les gens de l'art afin que la méthode de sélection utilisée soit l'une des méthodes suivantes : le « phage display » (Smith GP Science 1985 228 : 1315-1317; Gupta A et al., J Mol Biol. 2003 Nov 21;334(2):241-54 et US6593081 ; US2003148372 ), le « cell-surface display » (Kretzzschmar T et al. Curr Opin Biotechnol. 2002 Dec;13(6):598-602), l'auto-réplication compartimentée (CSR ; Ghadessy FH et al. Proc Natl Acad Sci U S A. 2001 Apr 10;98(8):4552-7 et WO0222869), la compartimentation *in vitro* (Sepp A et al. FEBS Lett. 2002 Dec 18;532(3):455-8 et WO9902671 ), le « ribosome display » (Cesaro-Tadic S et al. Nat Biotechnol. 2003 Jun;21(6):679-85 ; Matsuura T et al. FEBS Lett. 2003 Mar 27;539(1-3):24-8 ; Amstutz P et al. J Am Chem Soc. 2002 Aug 14;124(32):9396-403 et US6620587 ; US2002076692), la fusion ARNm-peptide ou le « mRNA display » (Nemoto N et al. FEBS Lett. 1997 Sep 8;41 4(2):405.8 ; Takahashi, T.T et al. TIBS 28(3): 159-165).

### ** MODE DE RÉALISATION #7 : MUTAGENÈSE ET SÉLECTION D'ACIDES NUCLEIQUES **

Dans un septième mode de réalisation, le procédé est caractérisé par l'évolution moléculaire d'un ou plusieurs acides nucléiques différents possédant des propriétés nouvelles ou améliorées. L'étape de traduction est supprimée et des adaptations évidentes pour les gens de l'art sont effectuées. A titre d'exemple, si on veut faire évoluer un ARN catalytique (un ribozyme), on peut réaliser les étapes a) à j) sans modifications (le terme gène d'intérêt renvoit alors à un ADN complémentaire de l'ARN d'intérêt) puis remplacer les étapes suivantes par : transcription *in vitro,* mise en présence du substrat et criblage des ARN ayant une activité catalytique nouvelle ou améliorée.

### ** MODE DE REALISATION #8 CAS INSERTIONS / DELETIONS **

Dans un mode de réalisation particulier, une partie ou l'ensemble des oligonucléotides conçus à l'étape a), synthétisés à l'étape b) et mis en solution sous forme de mélange à l'étape c) d'un quelconque des modes de réalisation décrits plus haut n'introduit pas de substitution mais une insertion ou une délétion. Dans le cas d'une délétion, les oligonucléotides peuvent, par exemple, être conçus sur le modèle suivant :
5'-TTCATAGCTAGGCGGTGCATCG3' portion du gène cible
3'-AAGTATCG---CGCCACGTAGG-5' oligonucléotide introduisant une délétion

L'oligonucléotide a donc la séquence suivante :
3'-AAGTATCGCGCCACGTAGG-5'
et, à l'issue de la réaction de mutagenèse, les trois bases TAG sont supprimées (délétées) et le gène a donc la séquence suivante :
5'-TTCATAGCGCGGTGCATCC-3'.

Dans le cas d'une insertion, les oligonucléotides peuvent, par exemple, être conçus sur le modèle suivant :
5'-TTCATAGCTAG-GCGGTGCATCC-3' portion du gène cible
3'-AAGTATCGTAGCTTCGCCACGTAGG-5' oligonucléotide introduisant une insertion

Le gène a donc initialement la séquence suivante :
5'-TTCATAGCTAGGCGGTGCATCC-3'
et, à l'issue de la réaction de mutagenèse les trois bases GAA sont ajoutées (insérées) et le gène a la séquence suivante :
5'-TTCATAGCTAGGAAGCGGTGCATCC-3'.

### ** MODE DE REALISATION #9 **

Dans un neuvième mode de réalisation; le procédé est caractérisé par la succession des étapes suivantes :
a) On détermine une stratégie de mutagenèse d'une façon identique à celle décrite dans le premier mode de réalisation.
b) A partir de cette stratégie, on conçoit une série d'oligonucléotides mutants, d'une taille de préférence comprise entre 15 et 45 nucléotides et chacun homologue à une région du gène cible. Dans ce mode de réalisation particulier, les oligonucléotides les plus externes sont d'orientation inverse, c'est-à-dire chacun homologue d'un brin différent du gène cible, de façon à ce qu'une amplification du fragment d'ADN localisé entre ces deux oligonucléotides puisse être réalisée. Les autres oligonucléotides peuvent être homologues à l'un ou à l'autre des deux brins indifféremment.
c) On synthétise les oligonucléotides mutants correspondants tels que conçus en b) en utilisant n'importe laquelle des techniques de synthèse sur puce
   Alternativement, une partie des oligonucléotides, par exemple les deux oligonucléotides externes, peuvent être synthétisés par la voie chimique classique, tandis que les autres oligonucléotides sont synthétisés en utilisant l'approche par puce à ADN.
d) On libère les oligonucléotides synthétisés sur la puce de leur support, de façon à obtenir un mélange d'oligonucléotides en solution
e) Indépendamment, on prépare une matrice contenant le gène cible.
f) On prépare un mélange réactionnel contenant la matrice préparée en e) et le mélange d'oligonucléotides obtenu en d), à une concentration telle que le rapport entre le nombre de molécules de matrice et le nombre de molécules de chacun des oligonucléotides mutants soit compris entre 0,01 et 100, et de préférence entre 0,1 et 10. On ajoute une polymérase thermostable, et tous les éléments nécessaires pour réaliser une réaction de réplication de la matrice à partir des oligonucléotides mutants : le tampon permettant son activité, des nucléotides triphosphates en quantité suffisante, les éventuels cofacteurs nécessaires.
g) On soumet le mélange à une température élevée (supérieure à 80°C et de préférence voisine de 94°C), pendant au moins une seconde et de préférence de l'ordre de une minute, de façon à disposer temporairement d'ADN simple brin.
h) On abaisse la température à une température comprise entre 0 et 60°C et de préférence comprise entre 20 et 50 °C, pendant au moins une seconde et de préférence de l'ordre de une minute, de façon à ce que les oligonucléotides présents dans le mélange se fixent sur leur site d'homologie au niveau de leur gène cible.
i) On soumet le mélange réactionnel à une température d'environ 68 à 72°C, ce qui permet l'activité optimale de la polymérase, pendant un temps suffisant pour que la réplication complète du plasmide puisse être effectuée, et calculé en fonction de la vitesse de synthèse de la polymérase (en bases par minute) et de la taille du gène cible.
   On répète les étapes g), h), et i), de préférence en utilisant un thermocycleur, de façon à ce que les cycles de températures puissent être effectués automatiquement.
j) On utilise tout moyen approprié pour sélectionner les brins d'ADN néosynthétisés générés lors de la l'étape i), des matrices initiales.
j') On utilise le fragment d'ADN synthétisé en j) comme insert à insérer dans un plasmide préalablement linéarisé, par exemple en utilisant l'approche dite de « TA-cloning », permettant le clonage rapide et efficace de fragments d'ADN obtenus par amplification.
k) On transforme le mélange réactionnel obtenu en j') dans un organisme adapté, tel que levures ou bactéries, rendues compétentes à la transformation.

A l'étape f), on ajoute éventuellement une ligase thermostable, comme par exemple la Taq Ligase ou la Tth ligase. Avantageusement dans ce cas, les oligonucléotides auront été phosphorylés en 5' au moyen d'une kinase préalablement à leur utilisation.

L'invention selon ce mode de réalisation peut en outre contenir une ou plusieurs des étapes l, m, n, o, ou p décrites précédemment.

### ** MODE DE REALISATION #10 **

Dans un dixième mode de réalisation, le procédé est caractérisé par la succession des étapes suivantes :
on réalise les étapes a), b), c), et d) de la même façon que dans le premier mode de réalisation.
e) Indépendamment, à partir d'une souche de bactérie ung- transformée par un plasmide contenant le gène cible, on prépare l'ADN plasmidique. Cet ADN plasmidique, du fait qu'il a été produit par une souche ung-, contient des Uraciles au lieu des Thymidines.

On mène les étapes f), g), h) et i) de la même façon que ce qui a été décrit précédemment. L'étape f) est effectuée en présence ou en absence de ligase, thermostable ou thermosensible.
j) et k) Pour sélectionner les brins d'ADN néosynthétisés générés lors de la l'étape g) des matrices initiales, on utilise le système de sélection déjà décrit par Kunkel et al (Kunkel TA, Bebenek K, McClary J. Methods Enzymol. 1991;204:125-39) : la simple introduction du mélange réactionnel dans des bactéries ung+ (représentées par la majorité des souches de laboratoires, telles que DH5a, DH10B, JM109, etc...) permet la sélection des plasmides ayant été synthétisés pendant les étapes f) à h), à l'exclusion des matrices initiales.

Le procédé selon ce mode de réalisation peut en outre contenir une ou plusieurs des étapes l, m, n, o, ou p décrites précédemment.

L'invention concerne aussi un procédé de mutagenèse d'une protéine cible ou de plusieurs protéines cibles, caractérisé en ce qu'il comprend la préparation d'une banque d'expression de gènes mutés à partir d'un gène cible codant pour ladite protéine, ou de plusieurs gènes cible codant pour lesdites protéines, selon le procédé de mutagenèse décrit précédemment, puis l'expression desdits gènes mutés pour produire une banque de protéines mutées, et éventuellement le criblage desdites protéines mutées pour une fonction désirée, avantageusement par rapport à la protéine cible.

Cette divulgation a également pour objet un mélange contenant des oligonucléotides mutés par rapport à un ou plusieurs gène(s) cible, ayant été produits tel que décrit dans les étapes a), b), c), et d) précédemment détaillées. Dans un mode de réalisation particulier, le mélange contient tous les oligonucléotides suffisants à générer l'intégralité des substitutions possibles au niveau d'un ou de plusieurs gènes cible, c'est-à-dire un nombre d'oligonucléotides égal à dix-neuf fois le nombre de codons encodés par ledit ou lesdits gènes cibles. Dans un second mode de réalisation particulier, le mélange contient tous les oligonucléotides suffisants pour générer les mutations de chaque codon vers une Alanine au niveau d'un ou de plusieurs gènes cible, c'est-à-dire autant d'oligonucléotides qu'il y a de codons sur ce ou ces gènes cible, une fois déduits les codons encodant déjà une Alanine.

Une banque de gènes mutés susceptible d'être obtenue par un des procédés décrits précédemment est décrite également.

D'autres avantages et caractéristiques de l'invention apparaîtront dans les exemples qui suivent, donnés à titre non limitatif et dans les dessins en annexe.

### Légende des figures

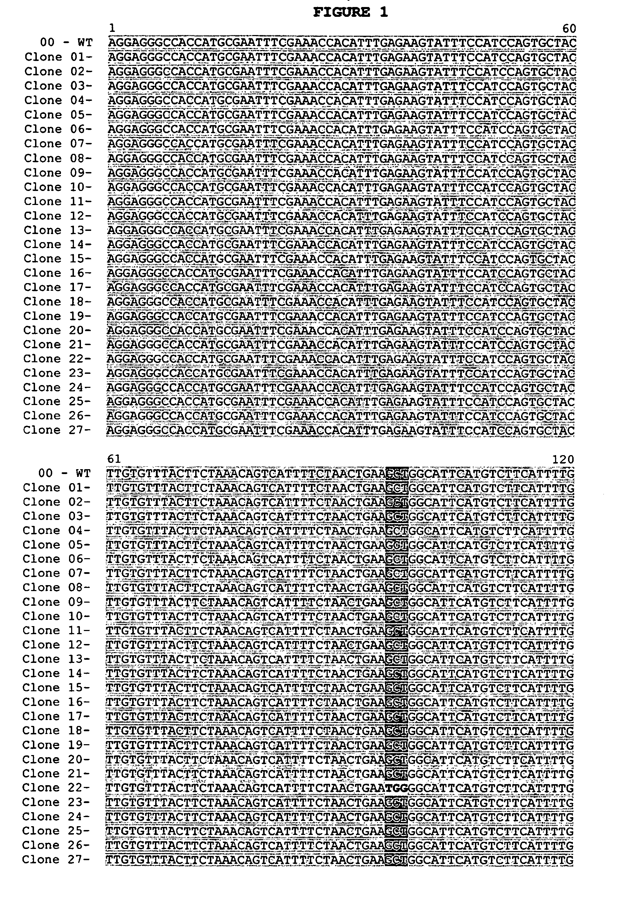

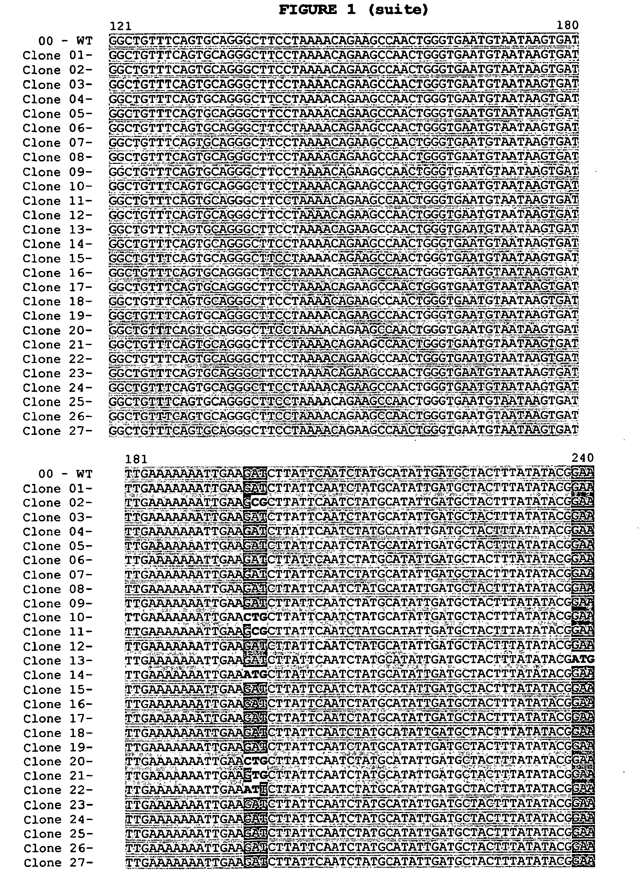

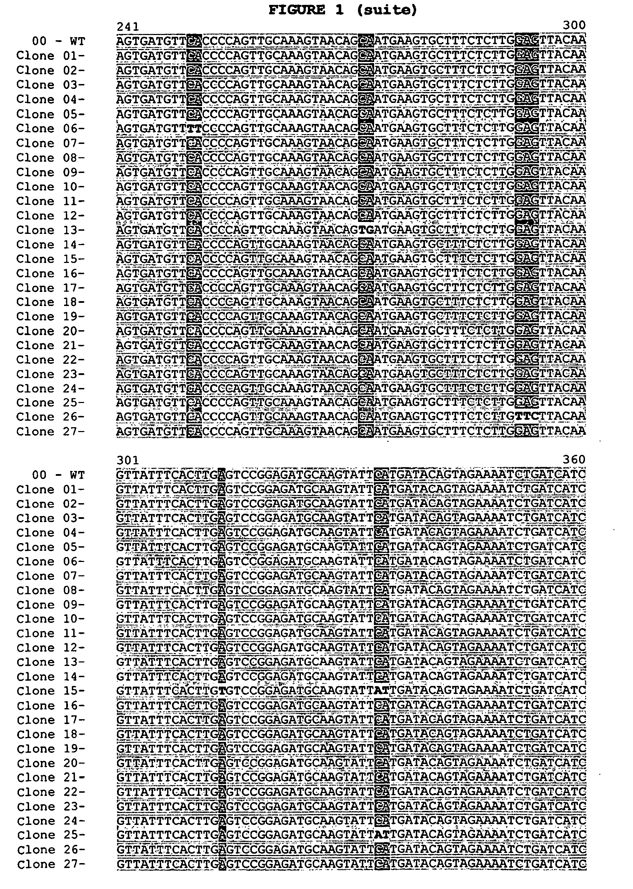

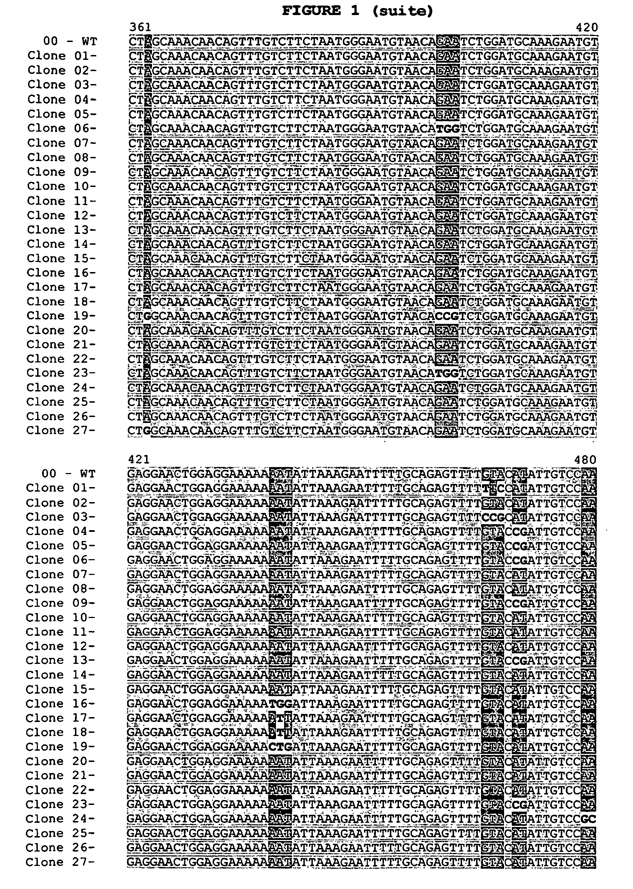

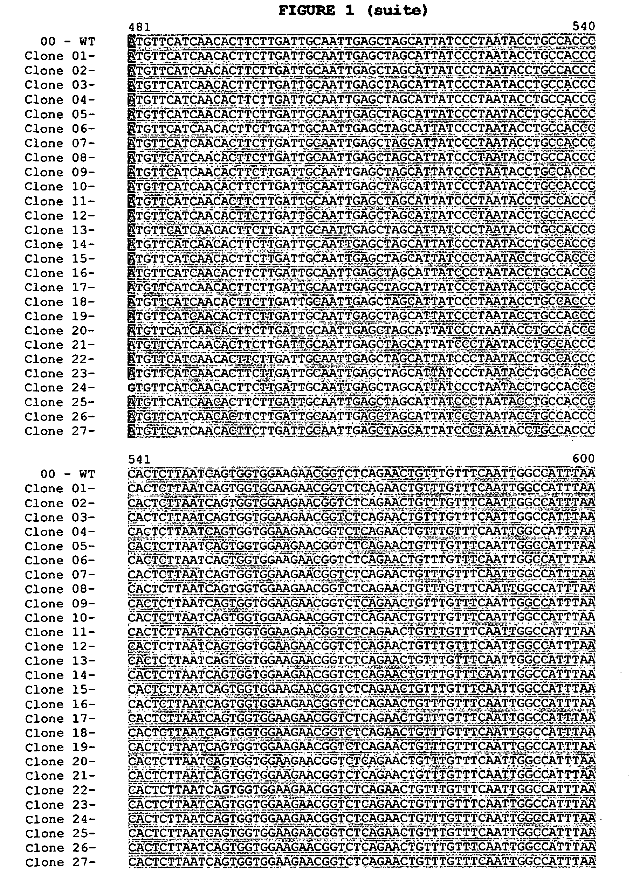

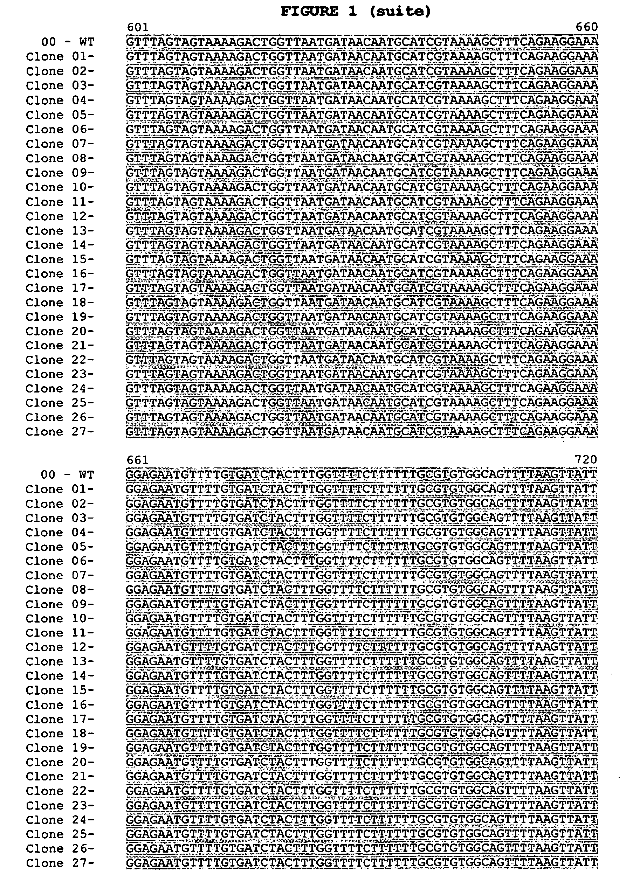

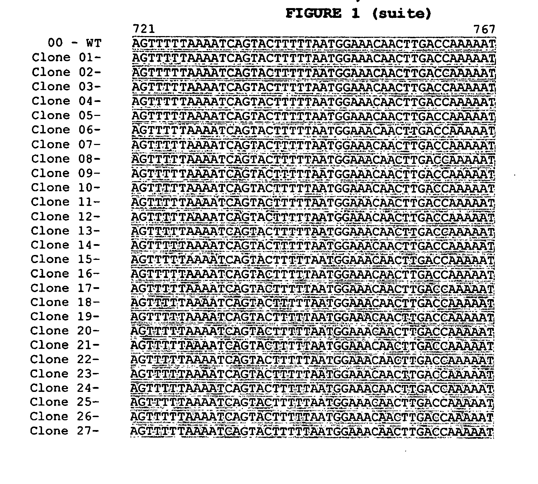

Figure 1. Alignements des séquences des clones obtenus dans l'exemple 8.

### EXEMPLES

### Exemple 1 : Evolution moléculaire d'une amylase

Les amylases constituent une famille d'enzymes ayant une activité sur l'amidon, qu'elles clivent de façon à obtenir des chaînes glucidiques de plus petite taille, voire des monomères. Les amylases sont utilisées dans de nombreux domaines industriels, et notamment dans l'agro-alimentaire et dans le domaine des lessives.

Ici, l'objectif est d'améliorer l'activité d'une amylase dans des conditions de faible concentration d'amidon, en abaissant son Km.
a) On détermine une stratégie de mutagenèse pour faire diminuer le Km de cette amylase. Comme les amylases sont extrêmement bien caractérisées et que plusieurs de ces enzymes ont été cristallisées (de façon à résoudre leur structure aux rayons X), on conçoit une stratégie de mutagenèse basée sur ces structures. Plus précisément, les résidus impliqués dans la constitution du site actif, ainsi que les résidus leur étant directement voisins sont ciblés. Au total, une trentaine de résidus sont ainsi ciblés. On souhaite les voir substitués, d'une façon combinatoire, avec une moyenne de deux mutations par molécule, non pas par la totalité de la diversité possible, mais seulement par les résidus ayant une proximité structurale, c'est-à-dire appartenant à la même sous-classe d'acides aminés (les hydrophobes, les aromatiques, etc...), ce qui représente une moyenne de 5 substitutions par résidu ciblé.
b) A partir de cette stratégie, on conçoit une série d'oligonucléotides mutants, contenant de chaque côté du codon mutant 15 bases totalement homologues à la séquence cible. Environ 150 oligonucléotides 33mers sont ainsi conçus (30 résidus ciblés multiplié par cinq substitutions en moyenne).
c) On synthétise les oligonucléotides mutants tels que conçus en b) en utilisant une approche de synthèse d'oligonucléotides sur puces. Au préalable, on aura pris soin d'adsorber, sur la puce, une fine couche de composé A, un composé basolabile. Le nombre maximal d'oligonucléotides à synthétiser sur une telle puce est d'environ 8000 : on synthétise alors chacun des 150 oligonucléotides en plusieurs exemplaires (environ 50) de façon à pouvoir disposer d'une large quantité du mélange d'oligonucléotides.
d) On libère les oligonucléotides de leur support, de façon à les mettre en solution. Pour ce faire, on place la puce dans des conditions basiques, ce qui a pour effet de cliver l'espaceur basolabile et de remettre automatiquement les oligonucléotides en suspension. Avant d'être utilisés, les oligonucléotides sont séchés par évaporation de l'amoniaque sous pression réduite, puis repris soit dans l'eau soit dans un tampon approprié. On dose la concentration de ces oligonucléotides en utilisant l'approche classique de spectrophotométrie.
e) Séparément, on effectue une minipréparation d'ADN plasmidique à partir de bactéries transformées par le plasmide contenant le gène de l'amylase ciblé, un promoteur bactérien pour exprimer ce gène, ainsi que le gène de résistance à l'Ampicilline.
f) On prépare un mélange réactionnel d'un volume de 7,5 microlitres et contenant 100 nanogrammes de matrice préparée en e) et 5 picomoles du mélange d'oligonucléotides obtenu en d).
g) On soumet le mélange réactionnel à une température de 95°C. de façon à disposer temporairement d'ADN simple brin.
h) On laisse le tube contenant le mélange revenir à température ambiante, de façon à ce que les oligonucléotides présents dans le mélange se fixent au niveau de leur site d'homologie sur le gène cible.
i) On ajoute 0,5 µl de T4 Polymérase (New England Biolabs), ainsi qu'un microlitre de son tampon 10X et un microlitre d'une solution contenant les quatre desoxyribonucléotides triphosphate à une concentration totale de 1 mM. On soumet le mélange réactionnel à une température de 37°C pendant vingt minutes.
j) On ajoute 0,5 µl d'enzyme Dpn I (New England Biolabs), deux microlitres de tampon NEB 4, et 7,5 microlitres d'eau distillée, et on incube ce mélange réactionnel pendant trente minutes à 37°C, de façon à ce que les matrices initiales soient clivées:
k) On transforme le mélange réactionnel obtenu en j) dans des bactéries compétentes, en utilisant la technique de transformation par choc thermique.
l) On étale les bactéries transformées sur une boite de Pétri grand format contenant, outre les milieux nutritifs nécessaires (LB), une quantité suffisante d'Ampicilline. Le lendemain, on obtient environ 10 000 colonies bactériennes, chacune contenant un plasmide différent, éventuellement mutant.
   On étudie le contenu en mutations de ces colonies par séquençage d'un échantillon statistique. Tant que le taux de mutation observé n'est pas suffisant, c'est-à-dire tant qu'il est inférieur à 2 mutations par molécule en moyenne, on réalise une préparation d'ADN à partir des colonies bactériennes obtenues en l), et on reproduit les étapes f) - l) le nombre de fois suffisantes à ce que l'objectif de taux de mutation soit atteint. Pour augmenter l'efficacité de la réaction, et donc limiter le nombre de répétitions des étapes f) à l), on peut ajouter, à l'étape i), 0,5 µl de T4 ligase et 1 µl de son tampon 10X. Dans ce cas, les oligonucléotides obtenus en d) devront avoir été phosphorylés en utilisant une Kinase (PNK , New England Biolabs par exemple) préalablement à leur utilisation à l'étape f). Après 2 à 4 cycles, le taux de mutagenèse est supérieur à 2.
m) On prélève individuellement les colonies bactériennes obtenues et on les utilise pour ensemencer un milieu nutritif contenant de l'Ampicilline, soit par repiquage manuel, soit en utilisant un équipement robotisé spécialisé dans le repiquage de colonies.
n) On mesure l'activité de la protéine obtenue après lyse des cultures obtenues en m), et on compare cette activité à celle de la protéine produite dans les mêmes conditions à partir de l'ADN plasmidique contenant le gène cible non mutant. Ces mesures d'activité peuvent être réalisées en utilisant l'un des tests classiquement utilisés pour mesurer l'activité des amylases, tels que le test à l'iode (Guan, H.P., and Preiss, J. (1993) Plant Physiol. 102,1269-1273), ou le test dit « des sucres réducteurs » (M Lever (1973) Biochemical Medicine 7:274-281). Lorsque l'activité associée à une colonie bactérienne est, de façon reproductible, significativement supérieure à l'activité observée en utilisant le gène cible, la molécule mutante est étudiée plus complètement, d'une part au moyen de tests enzymatiques complets de façon à déterminer son Km, et d'autre part par séquençage du gène mutant, afin de mettre en évidence la nature de la mutation à l'origine de cette activité améliorée.

### Exemple 2 : Alanine Scanning d'une Acylase

Les Acylases sont des enzymes utilisées dans de nombreux domaines industriels, et notamment dans le domaine de la synthèse d'antibiotiques de la famille des β-lactames. De nombreux travaux de caractérisation de l'activité de ces enzymes ont déjà été menés, mais le mécanisme précis de leur fonctionnement reste imparfaitement connu. Il est donc utile d'effectuer un Alanine Scanning complet de l'une de ces enzymes, afin d'effectuer sa cartographie fonctionnelle complète. L'objectif est d'obtenir tous les mutants Alanine de cette enzyme, et de tester tous ces mutants au moyen d'un test fonctionnel simple. Les mutants ayant perdu leur activité sont alors séquences afin d'identifier la ou les positions à l'origine de ladite perte d'activité. On utilise ici un approche parallèle, dans laquelle tous les mutants sont créés dans la même réaction ; le taux de mutations moyen recherché est inférieur à une mutation par molécule, de façon à pouvoir disposer principalement de mutants simples.
a) La stratégie de mutagenèse consiste à cibler l'ensemble des résidus d'une acylase utile pour la synthèse d'antibiotiques, à l'exception du premier (codon d'initiation de la traduction), du dernier (codon de terminaison de la traduction), et de tous les codons naturellement associés à une Alanine. Au total, environ 700 positions sont ainsi ciblées.
b) On conçoit les 700 oligonucléotides mutants. Chacun de ces oligonucléotides 33mers est constitué, de chaque côté du codon mutant, de 15 bases parfaitement homologues à la région correspondante au niveau du gène cible. Le codon mutant est invariablement de type GCG, car ce codon est le plus favorable pour l'expression dans E. Coli.
c) On synthétise les oligonucléotides mutants tels que conçus en b) en utilisant une approche de synthèse d'oligonucléotides sur puces. Au préalable, on aura pris soin d'adsorber, sur la puce, une fine couche de composé A, un composé basolabile. Le nombre maximal d'oligonucléotides à synthétiser sur une telle puce est d'environ 8000 : on synthétise alors chacun des 700 oligonucléotides en plusieurs exemplaires (environ 10) de façon à pouvoir disposer d'une large quantité du mélange d'oligonucléotides.
d) On libère les oligonucléotides de leur support, de façon à les mettre en solution. Pour ce faire, on place la puce dans des conditions basiques, ce qui a pour effet de cliver l'espaceur basolabile et de remettre automatiquement les oligonucléotides en suspension. On dose la concentration de ces oligonucléotides en utilisant l'approche classique de spectrophotométrie.
e) Séparément, on effectue une minipréparation d'ADN plasmidique à partir de bactéries transformées par le plasmide contenant le gène de l'acylase, un promoteur bactérien pour exprimer ce gène, ainsi que le gène de résistance à la Tetracycline.
f) On prépare le mélange réactionnel suivant :
   200 nanogrammes de matrice préparée en e)
   10 picomoles du mélange d'oligonucléotides obtenu en d)
   1 microlitre d'un mélange des quatre désoxyribonucléotides tri-phosphates à une concentration totale de 100 mM
   2,5 µl de tampon 10X de Pfu polymerase
   0,5 µl de Pfu Polymerase
   1 µl de MgSO4 à la concentration de 100 mM
   la quantité d'eau distillée suffisante pour atteindre 25µl
g) On soumet le mélange réactionnel à une température de 94°C, de façon à disposer temporairement d'ADN simple brin.
h) On soumet le mélange réactionnel à une température de 45°C, de façon à ce que chaque oligonucléotide présent dans le mélange se fixe au niveau de son site d'homologie sur le gène cible.
i) On soumet le mélange réactionnel à une température de 68°C pendant 20 minutes, soit le temps suffisant à ce que la réplication complète du plasmide soit effectuée.
   On répète les étapes g), h), et i) onze fois, en utilisant un thermocycleur pour effectuer automatiquement les cycles de température.
j) On prélève 10 µl du mélange réactionnel précédent, auquel on ajoute 0,5 µl d'enzyme Dpn I (New England Biolabs), deux microlitres de tampon NEB 4, et 7,5 microlitres d'eau distillée. On incube ce mélange réactionnel pendant trente minutes à 37°C, de façon à ce que les matrices initiales soient clivées.
k) On transforme le mélange réactionnel obtenu en j) dans des bactéries compétentes, en utilisant la technique de transformation par choc thermique.
l) On étale les bactéries transformées sur une boite de Petri grand format contenant, outre les milieux nutritifs nécessaires (LB), une quantité suffisante de Tetracycline. Le lendemain, on obtient environ 10 000 colonies bactériennes, chacune contenant un plasmide différent, éventuellement mutant.
   On étudie le contenu en mutations de ces colonies par séquençage d'un échantillon statistique. Tant que le taux de mutation observé n'est pas suffisant, c'est-à-dire tant qu'il est inférieur à 0,8 mutation par molécule en moyenne, on réalise une préparation d'ADN à partir des colonies bactériennes obtenues en l), et on reproduit les étapes f) - l) le nombre de fois suffisantes à ce que l'objectif de taux de mutation soit atteint. Pour augmenter l'efficacité de la réaction, et donc limiter le nombre de répétitions des étapes f) à l), on peut ajouter, à l'étape i), 0,5 µl de Pfu ligase et 1,25 µl de son tampon 10X en remplacement de la moitié du.tampon 10X de la Pfu Polymérase. Dans ce cas, les oligonucléotides obtenus en d) devront avoir été phosphorylés en utilisant une Kinase (PNK, New England Biolabs par exemple) et de l'ATP, préalablement à leur utilisation à l'étape f). Après 1 à 3 cycles, le taux de mutagenèse est supérieur à 0,8.
m) On prélève individuellement les colonies bactériennes obtenues et on les utilise pour ensemencer un milieu nutritif contenant de la Tétracycline, soit par repiquage manuel, soit en utilisant un équipement robotisé spécialisé dans le repiquage de colonies.
n) On mesure l'activité de la protéine obtenue après lyse des cultures obtenues en m), et on compare cette activité à celle de la protéine produite dans les mêmes conditions à partir de l'ADN plasmidique contenant le gène cible non mutant. Ces mesures d'activité peuvent être réalisées en utilisant l'un des tests classiquement utilisés pour mesurer l'activité des Acylases, tel que le test basé sur la dérivation au Fluoram du produit de la réaction. Lorsque l'activité associée à une colonie bactérienne est, de façon reproductible, significativement inférieure à l'activité observée en utilisant le gène cible, la molécule mutante est étudiée plus complètement, d'une part au moyen de tests enzymatiques complets de façon à déterminer certains de ses paramètres enzymatiques, et d'autre part par séquençage du gène mutant, afin d'identifier la mutation à l'origine de cette activité améliorée et de compléter ainsi la cartographie fonctionnelle en cours d'établissement pour cette enzyme.

### Exemple 3 : Stabilisation de l'inteféron gamma

Dans cet exemple, on souhaite obtenir un mutant amélioré de l'interféron gamma, utilisé dans le traitement des hépatites.

L'objectif est d'obtenir une molécule qui soit plus stable, de façon à .limiter le nombre d'injections à réaliser, à une par semaine au lieu de trois par semaine en utilisant l'interféron gamma de séquence naturelle a) On détermine une stratégie de mutagenèse : Même si la structure de l'interferon gamma, et ses interactions avec d'autres molécules, ont été caractérisés dans le détail, il n'est pas simple d'établir une stratégie destinée à améliorer sa stabilité. Aussi, dans le but de maximiser les chances d'obtenir un mutant positif, une bonne stratégie à poursuivre consiste à cibler l'ensemble des résidus (165) et à introduire au niveau de chacun d'eux la diversité maximale (les 19 résidus possibles), le tout dans une approche combinatoire, avec un taux moyen de mutations par molécule de 2.
b) On conçoit les 165x19 soit 3135 oligonucléotides mutants. Chacun de ces oligonucléotides est constitué, de chaque côté du codon mutant, de 15 bases parfaitement homologues à la région correspondante au niveau du gène cible.
c) On synthétise les oligonucléotides mutants tels que conçus en b) en utilisant une approche de synthèse d'oligonucléotides sur puces. Au préalable, on aura pris soin d'adsorber, sur la puce, une fine couche de composé A, un composé basolabile. Le nombre maximal d'oligonucléotides à synthétiser sur une telle puce est d'environ 8000 : on synthétise alors chacun des 3135 oligonucléotides en deux exemplaires de façon à pouvoir disposer d'une quantité maximale du mélange d'oligonucléotides.
d) On libère les oligonucléotides de leur support, de façon à les mettre en solution. Pour ce faire, on place la puce dans des conditions basiques, ce qui a pour effet de cliver l'espaceur basolabile et de remettre automatiquement les oligonucléotides en suspension. On dose la concentration de ces oligonucléotides en utilisant l'approche classique de spectrophotométrie.
e) Séparément, on effectue une minipréparation d'ADN plasmidique à partir de bactéries transformées par le plasmide contenant le gène de l'interféron gamma, un promoteur eucaryote pour exprimer ce gène, ainsi que le gène de résistance à l'Ampicilline sous le contrôle d'un promoteur bactérien.
f) On prépare le mélange réactionnel suivant :
   200 nanogrammes de matrice préparée en e)
   10 picomoles du mélange d'oligonucléotides obtenu en d)
   1 microlitre d'un mélange des quatre désoxyribonucléotides tri-phosphates à une concentration totale de 100 mM
   2,5 µl de tampon 10X de Pfu polymerase
   0,5 µl de Pfu Polymerase
   1 µl de MgSO4 à a concentration de 100 mM
   la quantité d'eau distillée suffisante pour atteindre 25µl
g) On soumet le mélange réactionnel à une température de 94°C, de façon à disposer temporairement d'ADN simple brin.
h) On soumet le mélange réactionnel à une température de 45°C, de façon à ce que chaque oligonucléotide présent dans le mélange se fixe au niveau de son site d'homologie sur le gène cible.
i) On soumet le mélange réactionnel à une température de 68°C pendant 20 minutes, soit le temps suffisant à ce que la réplication complète du plasmide soit effectuée.

On répète les étapes g), h), et i) onze fois, en utilisant un thermocycleur pour effectuer automatiquement les cycles de température.
j) On prélève 10 µl du mélange réactionnel précédent, auquel on ajoute 0,5 µl d'enzyme Dpn I (New England Biolabs), deux microlitres de tampon NEB 4, et 7,5 microlitres d'eau distillée. On incube ce mélange réactionnel pendant trente minutes à 37°C, de façon à ce que les matrices initiales soient clivées.
k) On transforme le mélange réactionnel obtenu en j) dans des bactéries compétentes, en utilisant la technique de transformation par choc thermique.
l) On étale les bactéries transformées sur une boite de Petri grand format contenant, outre les milieux nutritifs nécessaires (LB), une quantité suffisante d'Ampicilline. Le lendemain, on obtient environ 10 000 colonies bactériennes, chacune contenant un plasmide différent, éventuellement mutant.

On étudie le contenu en mutations de ces colonies par séquençage d'un échantillon statistique. Tant que le taux de mutation observé n'est pas suffisant, c'est-à-dire tant qu'il est inférieur à 2 mutations par molécule en moyenne, on réalise une préparation d'ADN à partir des colonies bactériennes obtenues en l), et on reproduit les étapes f) - l) le nombre de fois suffisantes à ce que l'objectif de taux de mutation soit atteint. Pour augmenter l'efficacité de la réaction, et donc limiter le nombre de répétitions des étapes f) à l), on peut ajouter, à l'étape i). 0,5 µl de Pfu ligase et 1,25 µl de son tampon 10X en remplacement de la moitié du tampon 10X de la Pfu Polymérase. Dans ce cas, les oligonucléotides obtenus en d) devront avoir été phosphorylés en utilisant une Kinase (PNK, New England Biolabs par exemple) et de l'ATP, préalablement à leur utilisation à l'étape f). Après 2 à 4 cycles, le taux de mutagenèse est supérieur à 2.
m) On prélève individuellement les colonies bactériennes obtenues et on les utilise pour ensemencer un milieu nutritif contenant de l'Ampicilline, soit par repiquage manuel, soit en utilisant un équipement robotisé spécialisé dans le repiquage de colonies. Chacune des colonies contient un gène cible potentiellement muté en un ou plusieurs endroits, intégré dans le plasmide.
m) A partir de chacune des cultures réalisées en m), on réalise une préparation d'ADN plasmidique.
o) On utilise la préparation d'ADN plasmidique obtenue en n) pour exprimer la protéine correspondante. Pour ce faire, on introduit individuellement chaque lot d'ADN plasmidique dans des cellules mammifères, par transfection.
p) On mesure l'activité de chaque interféron gamma mutant obtenu dans le surnageant des cellules transfectées en o), et on compare cette activité à celle de la protéine produite dans les mêmes conditions à partir de l'ADN plasmidique contenant le gène cible non mutant. Ces mesures d'activité peuvent être réalisées en utilisant l'un des tests classiquement utilisés pour mesurer l'activité de l'interféron gamma. Pour mesurer la stabilité des mutants, il est nécessaire de pré-incuber les molécules mutantes, à une température donnée, afin de mesurer dans ces conditions la décroissance de l'activité, et de comparer cette décroissance à celle observée dans le cas du gène non mutant. Lorsque la décroissance de l'activité observée pour un clone particulier est plus faible que celle observée dans le cas du gène non mutant, on effectue toutes les mesures nécessaires à caractériser ce gain de stabilité, et on séquence le gène mutant afin de mettre en évidence la nature de la mutation à l'origine de cette activité améliorée.

### Exemple 4: Mutagenèse Massive Multiplex: Utilisation d'un unique mélange d'oligonucléotides synthétisés sur une puce pour réaliser les Alanine Scanning de plusieurs gènes simultanément

De façon à réaliser des économies supplémentaires, il est possible de réaliser des stratégies de mutagenèse massive de plusieurs gènes simultanément, en utilisant un seul mélange d'oligonucléotides. Cet exemple détaille l'Alanine-Scanning complet de quatre gènes simultanément ; cette approche peut néanmoins être adaptée pour n'importe quelle stratégie de mutagenèse.
a) On détermine une stratégie de mutagenèse pour plusieurs gènes cible. Au niveau de chaque gène cible, tous les codons sont ciblés, sauf les premier, dernier, et les codons naturellement associés à une Alanine. On prend soin que les gènes cible ne présentent pas entre eux un trop fort taux d'homologie, de façon à ce que les oligonucléotides destinés à muter un des gènes ne puisse pas s'hybrider sur un autre, ce qui pourrait être à l'origine de l'introduction de mutations supplémentaires indésirables.
b) A partir de cette stratégie, on conçoit une série d'oligonucléotides mutants. Les oligonucléotides sont ici analogues à ceux décrits dans l'exemple 2.
c) On synthétise les oligonucléotides mutants tels que conçus en b) en utilisant une approche de synthèse d'oligonucléotides sur puces, de la même façon que ce qui a été décrit dans les exemples précédents. Tous les oligonucléotides, destinés à chacun des quatre gènes, sont synthétisés sur une seule puce.
d) On libère les oligonucléotides de leur support, de façon à les mettre en solution.
e) Séparément, on prépare les quatre plasmides contenant chacun un gène cible, de façon à pouvoir disposer d'une quantité d'ADN plasmidique purifié suffisante. Chaque plasmide contient, outre le gène cible et la séquence promotrice associée, un gène de résistance à un antibiotique différent (par exemple, le premier plasmide contient le gène de résistance à l'Ampicilline, le second le gène de résistance à la Kanamycine, le troisième le gène de résistance à la Tétracycline, et le quatrième le gène de résistance au Chloramphénicol).
f) On prépare un mélange réactionnel contenant 100 ng de chaque matrice préparée en e) et le mélange d'oligonucléotides obtenu en d). On complète le mélange réactionnel avec les éléments décrits dans l'exemple 2.

On effectue les étapes g) à k) telles qu'elles ont été décrites dans l'exemple 2.
l) On partage les bactéries transformées par le mélange réactionnel en quatre parties, et on étale chacune de ces parties sur un milieu contenant un agent sélectif différent. Ainsi, seules les bactéries contenant le premier plasmide pousseront sur les boites de Petri contenant de l'Ampicilline, tandis que les bactéries contenant des mutants du second plasmide pousseront sur une boite de petri contenant de la Kanamycine, etc... Ainsi, les banques de mutants correspondant à chacun des plasmides seront séparées en quatre sous-banques contenant chacune les mutants correspondant à un seul gène cible.

La suite de cet exemple est identique à ce qui a déjà été décrit dans l'exemple 2, mis à part de fait que la réalisation de plusieurs cycles contenant les étapes f) à l) inclut une étape supplémentaire de mélange de l'ADN obtenu à partir de chacune des quatre sous-banques.

### Exemple 5 : Second exemple de Mutagenèse Massive Multiplex

Cet exemple est voisin du précédent mais permet l'utilisation simultanée de 6 plasmides différents, et l'individualisation des banques de mutants leur correspondant, en fin d'expérimentation, au moyen d'une simple sélection sur milieux sélectifs.

Comme le nombre d'antibiotique couramment utilisés en recherche est principalement limité à quatre, seule l'utilisation de combinaisons d'antibiotiques et de combinaisons de gènes de résistance permet d'utiliser simultanément autant de plasmides, et de les ré-individualiser facilement.

Ainsi, chacune des 6 matrices contient, en plus du gène cible, deux gènes de résistance (Amp - Kan ; Amp - Tet ; Amp - Cam ; Tet - Kan ; Tet - Cam ; Kan - Cam).

Le protocole est mené comme dans l'exemple 4. A l'issue du protocole, les bactéries transformées sont étalées sur des milieux sélectifs contenant deux antibiotiques, de façon à individualiser chacune des 6 sous-banques de mutants obtenues.

### Exemple 6 : Utilisation d'un unique mélange d'oligonucléotides synthétisés sur une puce pour réaliser des stratégies de mutagenèse sur plusieurs gènes séquentiellement.

Cet exemple est voisin de l'exemple 4 mais permet l'utilisation d'un nombre en théorie infini de plasmides différents, chacun contenant un gène cible différent. Dans cet exemple, les plasmides ne sont pas utilisés simultanément, mais séquentiellement, ce qui permet de lever les difficultés précédemment décrites d'individualisation des sous-banques de mutants : un seul mélange d'oligonucléotides est synthétisé comme dans les exemples précédents, et ce mélange est ensuite utilisé dans plusieurs réactions indépendantes, contenant chacune un gène cible différent. L'approche est ensuite menée d'une façon analogue à ce qui a été décrit dans l'exemple 2.

### Exemple 7 : Création simultanée de mutants sur deux gènes cibles.

Dans certains cas, il peut être nécessaire de créer des banques de mutants sur deux gènes simultanément, et d'effectuer le criblage simultané des deux banques de gènes mutants.

Cette nécessité s'applique notamment lorsque les gènes ont un effet synergique. Un exemple particulier est celui de deux sous-unités d'une même protéine. D'autres cas, comme notamment le cas de vaccins, peuvent également révéler des synergies fortes entre deux gènes. Dans tous ces cas, la création simultanée de banques de mutants de deux gènes, et la co-expression de ces deux types de molécules mutantes, peut être un élément d'une stratégie d'évolution moléculaire de l'ensemble.

Le point de départ est ici un plasmide contenant non pas un seul mais deux gènes cible, chacun étant gouverné par un promoteur d'expression eucaryote ou procaryote, selon le modèle d'étude. (La disposition des deux gènes cible sur le même plasmide permet de simplifier les étapes ultérieures de transformation ou de transfection. Toutefois, l'utilisation alternative de deux plasmides contenant chacun un gène cible est également possible).

Un mélange d'oligonucléotides est synthétisé de la même façon que ce qui a été décrit dans les exemples précédents : certains des oligonucléotides de ce mélange sont destinés à introduire des mutations sur le premier gène, les autres sur le second.

Ce mélange d'oligonucléotides est ensuite utilisé pour générer une banque de plasmides mutants, qui pourra par exemple être synthétisée et utilisée comme dans l'exemple 4.

### Exemple 8 : Utilisation d'un mélange d'oligonucléotides synthétisés sur une puce pour réaliser la mutagenèse d'IL15.

Le modèle choisi dans cet exemple est le gène IL15 (Interleukine 15) cloné dans le vecteur pORF (sequence IL15 SEQ ID No 1). Les 296 oligonucléotides choisis modifient 37 sites sur le gène IL 15, dont 18 correspondent à des sites de restriction détruits (séquences des oligonucléotides et sites modifiés : SEQ ID Nos 3-298). Les autres portent sur les positions 157, 490, 205, 238, 265, 292, 175, 226, 250, 280, 301, 325, 346, 370, 391, 415, 433, 457, et 478 du gène IL15. Chaque site est muté par les codons suivants : GCG ; TTC ; ATT ; CTG ; CCG ; GTG ; TGG ; ATG.

Les 18 sites de restrictions sont les suivants :

| Site de restriction modifiémélange de 296 oligonucléotides | Position de la mutations dans Seq ID No 1 | SEQ ID Nos |
|---|---|---|
| MsII | 220-222 | 3-10 |
| XmnI | 37-39 | 11-18 |
| AluI | 97-99 | 19-26 |
| BsmI | 100-102 | 27-34 |
| BgIII | 196-198 | 35-42 |
| SmII | 310-312 | 43-50 |
| NsiI | 214-216 | 51-58 |
| BsrDI | 271-273 | 59-66 |
| BspHI | 334-336 | 67-74 |
| BfaI | 361-363 | 75-82 |
| SspI | 439-441 | 83-90 |
| RsaI | 466-468 | 91-98 |
| BsrGI | 469-471 | 99-106 |
| BsaWI | 316-318 | 107-114 |
| TfiI | 400-402 | 115-122 |
| MseI | 445-447 | 123-130 |
| MlyI | 313-315 | 131-138 |
| TaqI | 22-24 | 139-146 |

| Site de restriction modifé | Position de la mutation Dans SEQ ID No 1 | SEQ ID Nos |
|---|---|---|
| - | 157-159 | 147-154 |
| - | 490-492 | 155-162 |
| - | 205-207 | 156-170 |
| - | 238-240 | 171-178 |
| - | 265-267 | 179-186 |
| - | 292-294 | 187-194 |
| - | 175-177 | 195-202 |
| - | 226-228 | 203-210 |
| - | 250-252 | 211-218 |
| - | 280-282 | 219-226 |
| - | 301-303 | 227-234 |
| - | 325-327 | 235-242 |
| - | 346-348 | 243-250 |
| - | 370-372 | 251-258 |
| - | 391-393 | 259-266 |
| - | 415-417 | 267-274 |
| - | 433-435 | 275-282 |
| - | 457-459 | 283-290 |
| - | 478-480 | 291-298 |

Les oligonucléotides ont été synthétisés sur un support de silice poreuse auquel ils sont rattachés par un espaceur clivable. La méthode de fonctionnalisation d'un support par un tel espaceur est par exemple décrite dans WO03008360. La méthode de synthèse est décrite dans WO0226373. Plus particulièrement, l'espaceur clivable est un t-butyl11-(diméthylaminodiméthylsilyl) undécanoate qui est greffé sur le support de silice et dont l'ester est déprotégé.

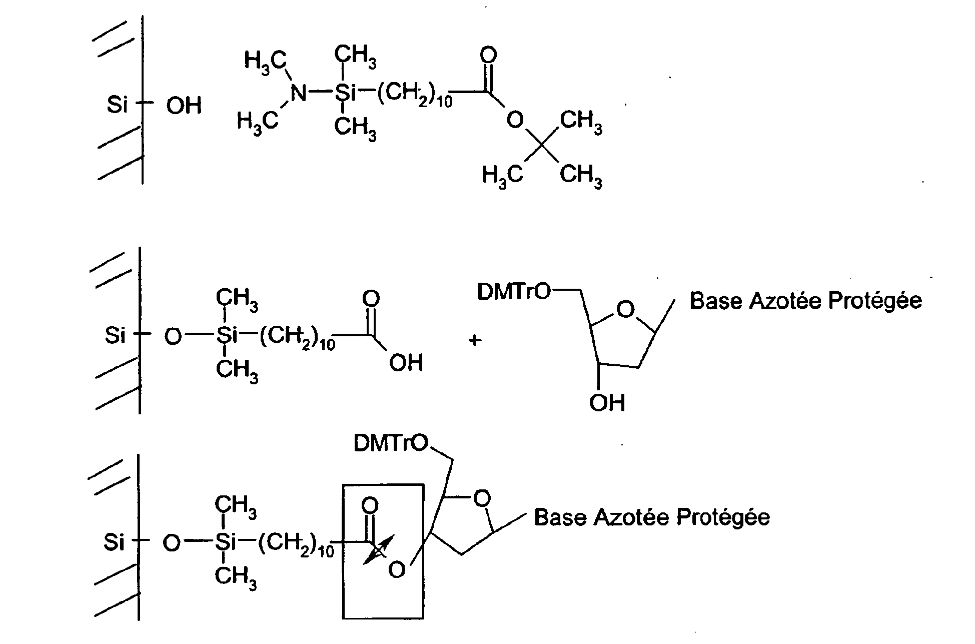

Les oligonucléotides ont été synthétisés sur un support solide selon le procédé décrit dans WO0226373. Ils ont été synthétisés sur 4 puces : 1 puce pour les oligonucléotides ayant un A en 3', 1 pour un C en 3', 1 pour un G en 3', et 1 pour un T en 3'. Les oligonucléotides ont ensuite été libérés par un traitement à pH basique en solution d'ammoniaque.

Cette synthèse a abouti à un dans un volume de 10 µl, à la concentration totale de 30 pmol pour l'ensemble des 296 oligonucléotides. Le mélange d'oligonucléotides a été utilisé pour générer une banque de mutants en utilisant le protocole suivant :

### 1-Purification des oligonucléotides

Diluer 3 µl ou 2 µl du pool d'oligonucléotides dans 100 µl d'H₂O ;

Placer sur une colonne Centricon YM3 (Millipore); centrifuger 40 min. à 9000 rpm ; et

Retourner la colonne et récupérer 15 µl après centrifugation 1 min. à 9000 rpm.

### 2-Phosphorylation des oligonucléotides

15 µl d'oligonucléotides purifiés
2 µl de tampon PNK ; 2 µl d'ATP 10 mM V_{f} = 20 µl
1 µl de PNK 1 h à 37°C; pas d'inactivation à 65°C

### 3-PLCR (Polymerase Ligase Chain Reaction)

| | |
|---|---|
| 1 µl (200 ng de matrice pORF IL15) | 1 µl d'ATP 10Mm |
| 1 µl de dNTP (25mM) | 0.2 µl de NAD(100 mM) |
| 1 µl de MgSO₄ (100 mM) | 0.2 µl de dTT (1 M) |
| 3.5 µl de tampon 10X pfu pol | 0.8 µl de pfu pol |
| 0.8 µl de Tth ligase | 0.5 µl de Taq |

V_{f}=10µl

Réaction : 10 µl de mix + 20 µl d'oligonucléotides phosphorylés + 5 µl d'H₂O
Témoin négatif : 10 µl de mix + 25 µl d'H₂O
Programme thermocycleur : 1' à 94°C; 2' à 40°C ; 20' à 68°C; 12 cycles

### 4-Digestion Dpn I

35 µl de PLCR
4 µl de tampon 4 (NEB) ; 0,5 µl de Dpn I (20 000u/mL)
0,5 µl d'H₂O 30' à 37°C

### 5-Dialyse + Transformation

8 µl dialysés sur membrane contre H₂O 30' ; électroporation avec 40 µl de bactéries DH10B électrocompétentes ; reprendre dans1 mL de SOC, puis 45' à 37°C
Centrifugation 4' à 6000 rpm, puis reprendre dans 200 µl de LB
Etalement sur milieu LB+Amp. : 5000 colonies (boite n°1 : 9/10)
Repiquer 96 colonies sur plaque dans milieu LB+Amp. et mettre à pousser en agitation à 37°C pendant 3 heures : 3 plaques

### 6-PCR sur cultures

Mix pour 96 réactions :
4,5 mL d'H₂O ; 500 µl de thermopol buffer ; 100 µl de dNTP (2,5 mM)
20 µl d'oligo IL15 (421) (100 µM) ; 20 µl d'oligo IL15 (1500) (100 µM)
250 µl de Taq ; 50 µl de mix + 5 µl de culture

Programme thermocycleur : 10' à 96°C ; [1' à 94°C ; 1' à 50°C; 1'30 à 72°C] 35 cycles

### 7-Digestion des produits de PCR

La digestion a été effectuée en plaque de 96 puits (1 unité par puit)
10 µl de PCR + 10 µl de MIX ; enzymes de restriction : BsrG I ; Bgl II ; Ssp I ; Mly

### 8-Séquençage des clones

### 9-Raclage des banques + Nouveaux Tours de PLCR

La boite de colonies n°1 (cf étape 5) a été raclée, une préparation d'ADN a ensuite été réalisée (Kit E.Z.N.A^{™} Plasmid Miniprep). 200 ng de cette banque (n°1) ont servi de matrice pour une deuxième PLCR avec 2 µl du pool d'oligonucléotides purifiés (cf étapes 1 et 2). Une recherche de mutants a été réalisée sur cette banque (n°2), selon le protocole décrit précedemment.

Après raclage de la banque (n°2), 200 ng de la préparation d'ADN ont servi à réaliser une troisième PLCR, avec à nouveau 2 µl du pool d'oligonucléotides purifiés. Les mutants ont été recherchés sur la banque n°3 issue de cette troisième PLCR.

### RESULTATS

La sélection des clones a été réalisée sur la disparition d'un site de restriction enzymatique sur le gène IL15. Les enzymes de restriction utilisées pour ce criblage étaient BsRG I, BgI II, Ssp I, et Mly I. Sept clones additionels ont été sélectionnés au hasard (notés *), sans analyse préalable de leur profil de restriction.

| CLONE | Tour de PLCR | MUTATION | SEQ ID No |
|---|---|---|---|
| 1 | 1^{er} | BSRG I | 299 |
| 2 | 1^{er} | Bgl II | 300 |
| 3 | 1^{er} | BsrG I | 301 |
| 4 | 2^{ème} | BsrG I | 302 |
| 5 | 2^{ème} | BsrG I | 303 |
| 6 | 2^{ème} | position 251/Tfi I /BsrG I | 304 |
| 7 | 2^{ème} | WT IL15* | 305 |
| 8 | 2^{ème} | WT IL15* | 306 |
| 9 | 2^{ème} | BsrG I | 307 |
| 10 | 2^{ème} | Bgl II | 308 |
| 11 | 2^{ème} | Bgl II | 309 |
| 12 | 3^{ème} | WT IL15* | 310 |
| 13 | 3^{ème} | position 237/ BsrG I /BsrD I | 311 |
| 14 | 3^{éme} | Bgl II | 312 |
| 15 | 3^{éme} | BspH I / Mly I | 313 |
| 16 | 3^{ème} | Ssp I | 314 |
| 17 | 3^{ème} | Ssp I | 315 |
| 18 | 3^{éme} | Ssp I | 316 |
| 19 | 3^{éme} | Bfa I / Tfi I / Ssp I | 317 |
| 20 | 3^{ème} | Bgl II | 318 |
| 21 | 3^{ème} | Bgl II | 319 |
| 22 | 3^{ème} | Alu I / Bgl II | 320 |
| 23 | 3^{éme} | Tfi I /BsrG I | 321 |
| 24 | 3^{ème} | position 478* | 322 |
| 25 | 3^{ème} | BspH I * | 323 |
| 26 | 3^{éme} | position 292* | 324 |
| 27 | 3^{ème} | Bfa I * | 325 |

| | | | |
|---|---|---|---|
| * : clones sélectionnés au hasard | | | |

Cet exemple a permis de démontrer pour la première fois que des mutants peuvent être préparés à partir d'une banque d'oligonucléotides synthétisés sur une puce à ADN : La qualité du mélange d'oligonucléotides synthétisés sur puce est en effet suffisante, et la qualité est comparable à celle obtenue par synthèse classique.

## Revendications

1. Procédé de production d'une banque de gènes mutés comprenant les étapes suivantes :
a. Synthèse sur une puce à ADN d'une banque d'oligonucléotides comprenant des oligonucléotides complémentaires d'une ou plusieurs régions d'un ou de plusieurs gènes cibles et comprenant chacun, de préférence en leur milieu, une ou plusieurs mutations par rapport à la séquence du ou des gènes cibles ;
b. Mise en solution sous forme de mélange de la banque d'oligonucléotides synthétisée sur une puce à ADN en a) ; et,
c. Génération d'une banque de gènes mutés en utilisant la banque d'oligonucléotides en solution obtenue en b) et une ou plusieurs matrices contenant ledit ou lesdits gènes cibles.

2. Procédé selon la revendication 1, dans lequel la banque de gènes mutés est générée dans l'étape c) par le procédé de Mutagenèse Massive.

3. Procédé selon la revendication 1, dans lequel l'étape c) comprend les étapes suivantes :
i. Fournir une ou plusieurs matrices contenant ledit ou lesdits gènes cibles
ii. Mettre en contact ladite ou lesdites matrices avec la banque d'oligonucléotides synthétisée en a) dans des conditions permettant l'hybridation des oligonucléotides de la banque sur ladite ou lesdites matrices pour produire un mélange réactionnel ;
iii. Effectuer une réplication de ladite ou desdites matrices à partir du mélange réactionnel en utilisant une ADN polymérase ;
iv. Eliminer la ou les matrices initiales du produit de l'étape iii) et ainsi sélectionner les brins d'ADN néosynthétisés ; et, éventuellement,
v. Transformer un organisme avec le mélange d'ADN obtenu à l'étape iv).

4. Procédé selon l'une des revendications 1-3, dans lequel la matrice est un acide nucléique circulaire, de préférence un plasmide.

5. Procédé selon l'une des revendications 1-4, dans lequel la matrice contient les éléments permettant l'expression dudit ou desdits gènes cibles.

6. Procédé selon l'une des revendications précédentes, dans lequel les oligonucléotides de ladite banque synthétisés sur la puce à ADN sont liés à ladite puce à ADN par l'intermédiaire d'une molécule espaceur clivable et dans lequel lesdits oligonucléotides sont mis en solution sous forme de mélange en soumettant les oligonucléotides liés à ladite puce à ADN aux conditions associées au clivage de la molécule espaceur.

7. Procédé selon la revendication 6, dans lequel ladite molécule espaceur est clivable en milieu basique, par l'action de la lumière ou par action enzymatique.

8. Procédé selon la revendication 7, dans lequel ladite molécule espaceur est clivable en milieu basique.

9. Procédé selon la revendication 8, dans lequel ladite molécule espaceur est le composé de formule :

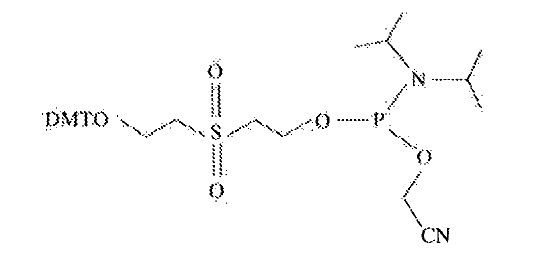

10. Procédé selon la revendication 8, dans lequel ladite molécule espaceur est le composé de formule :

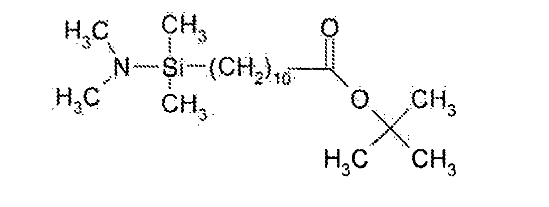

11. Procédé selon l'une des revendications 3 à 10, dans lequel l'étape iv est réalisée au moyen d'une enzyme de restriction spécifique des brins d'ADN méthylés, de préférence appartenant au groupe des enzymes : DpnI, NanII, NmuDI ou NmuEI.

12. Procédé selon l'une quelconque des revendications précédentes, dans lequel les oligonucléotides synthétisés à l'étape a) sont tous complémentaires d'un même gène cible.

13. Procédé selon l'une quelconque des revendications précédentes, dans lequel tous les oligonucléotides complémentaires d'un même gène cible sont complémentaires du même brin dudit gène cible.

14. Procédé selon l'une quelconque des revendications précédentes, dans lequel la banque d'oligonucléotides synthétisés à l'étape a) contient les oligonucléotides portant les mutations permettant d'introduire l'ensemble des substitutions possibles sur chaque codon dudit ou desdits gènes cibles.

15. Procédé selon l'une des revendications 1 à 13, dans lequel la banque d'oligonucléotides synthétisés à l'étape a) contient les oligonucléotides portant les mutations permettant d'introduire un même acide aminé, de préférence une alanine, sur chaque codon dudit ou desdits gènes cibles.

16. Procédé de mutagenèse d'une protéine cible ou de plusieurs protéines cibles, **caractérisé en ce qu'**il comprend la préparation d'une banque d'expression de génes mutés à partir d'un gène cible codant pour ladite protéine, ou de plusieurs gènes cible codant pour lesdites protéines, par le procédé de production d'une banque de gènes mutés selon les revendications 1-14, puis l'expression desdits gènes mutés pour produire une banque de protéines mutées.

## Claims

1. A method for producing a library of mutant genes comprising the following steps:
a. Synthesizing on a DNA chip an oligonucleotide library comprising oligonucleotides complementary to one or more regions of one or more target genes and each comprising, preferably in their center, one or more mutations of the sequence of the target gene(s);
b. Placing in solution as a mixture the oligonucleotide library synthesized on the DNA chip in a); and,
c. Generating a library of mutant genes by using the oligonucleotide library in solution obtained in b) and one or more templates containing said target gene(s).

2. The method according to claim 1, wherein the mutant gene library is generated in step c) by a Massive Mutagenesis method.

3. The method according to claim 1, wherein step c) comprises the following steps:
i. Providing one or more templates containing said target gene(s);
ii. Contacting said template(s) with the oligonucleotide library synthesized in a) in conditions that allow the oligonucleotides in the library to anneal to said template(s) so as to produce a reaction mixture;
iii. Carrying out a replication of said template(s) from the reaction mixture by using a DNA polymerase;
iv. Eliminating the starting template(s) from the product of step iii) and thereby selecting newly synthesized DNA strands; and, optionally,
v Transforming an organism with the DNA mixture obtained in step iv).

4. The method according to anyone of claims 1-3, wherein the template is a circular nucleic acid, preferably a plasmid.

5. The method according to anyone of claims 1-4, wherein the template contains elements enabling the expression of said target gene(s).

6. The method according to anyone of previous claims, wherein the oligonucleotides of said library synthesized on the DNA chip are coupled to said DNA chip via a cleavable spacer molecule and wherein said oligonucleotides are placed in solution as a mixture by subjecting the oligonucleotides coupled to said DNA chip to conditions associated with cleavage of the spacer molecule.

7. The method according to claim 6, wherein said spacer molecule can be cleaved in basic medium, by reaction to light or by enzymatic reaction.

8. The method according to claim 7, wherein said spacer molecule can be cleaved in basic medium.

9. The method according to claim 8, wherein said spacer molecule is the compound represented by the formula :

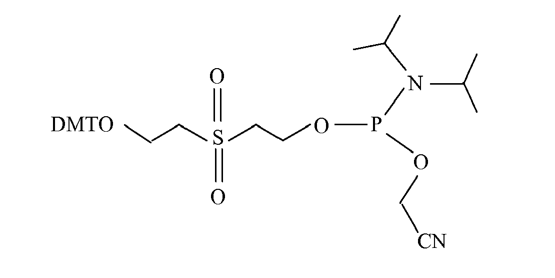

10. The method according to claim 8, wherein said spacer molecule is the compound represented by the formula :

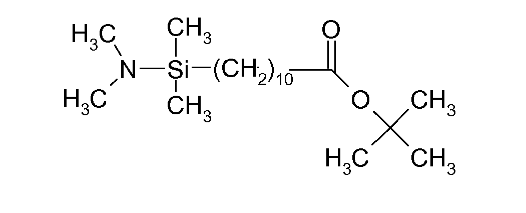

11. The method according to anyone of claims 3-10, in which step iv) is carried out by means of a restriction enzyme specific for methylated DNA strands, preferably belonging to the group of enzymes : Dpnl, Nanll, NmuDI and NmuEl.

12. The method according to claim 1, wherein the oligonucleotides synthesized in step a) are all complementary to a same target gene.

13. The method according to anyone of previous claims, wherein all the oligonucleotides complementary to a same target gene are complementary to the same strand of said target gene.

14. The method according to anyone of previous claims, wherein the oligonucleotide library synthesized in step a) contains oligonucleotides carring mutations allowing introduction of all possible substitutions at each codon of said target gene(s).

15. The method according to anyone of previous claims, wherein the oligonucleotide library synthesized in step a) contains oligonucleotides carrying mutations allowing introduction of a same amino acid, preferably an alanine, at each codon of said target gene(s).

16. The method of mutagenesis of a target protein or of several target proteins, **characterized in that** it comprises preparing a mutant gene expression library from a target gene coding for said protein, or from several target genes coding for said proteins, by the method for producing a mutant gene library according to anyone of claims 1-14, then expressing said mutant genes to produce a library of mutant proteins.

## Patentansprüche

1. Verfahren zur Herstellung einer Bank mutierter Gene, umfassend die folgenden Schritte:
a. Synthese einer Oligonukleotid-Bank auf einem DNA-Chip, umfassend Oligonukleotide, die zu einer oder mehreren Regionen eines oder mehrerer Zielgene komplementär sind und jeweils, vorzugsweise in ihrer Mitte, eine oder mehrere Mutationen verglichen mit der Sequenz des oder der Zielgene umfassen;
b. In Lösung bringen der auf einem DNA-Chip in a) synthetisierten Oligonukleotid-Bank in Form eines Gemisches; und
c. Erzeugung einer Bank mutierter Gene unter Verwendung der in b) in Lösung erhaltenen Oligonukleotid-Bank und einer oder mehrerer Matrizen, die besagtes oder besagte Zielgene enthalten.

2. Verfahren gemäß Anspruch 1, in dem die Bank mutierter Gene mithilfe des Verfahrens der Massenmutagenese in Schritt c) erzeugt wird.

3. Verfahren gemäß Anspruch 1, in dem Schritt c) die folgenden Schritte umfasst:
i. Bereitstellen einer oder mehrerer Matrizen, die besagtes oder besagte Zielgene enthalten;
ii. Inkontaktbringen besagter Matrize oder Matrizen mit der in a) synthetisierten Oligonukleotid-Bank unter Bedingungen, welche die Hybridisierung der Oligonukleotide der Bank an besagter oder besagten Matrizen erlauben, um ein Reaktionsgemisch herzustellen;
iii. Durchführen einer Replikation besagter Matrize oder Matrizen aus dem Reaktionsgemisch unter Verwendung einer DNA-Polymerase;
iv. Entfernen der ursprünglichen Matrize oder Matrizen des Produkts von Schritt iii) und daher Selektieren der neusynthetisierten DNA-Stränge; und gegebenenfalls
v. Transformieren eines Organismus mit dem in Schritt iv) erhaltenen DNA-Gemisch.

4. Verfahren gemäß einem der Ansprüche 1 bis 3, in dem die Matrize eine zirkuläre Nukleinsäure, vorzugsweise ein Plasmid, ist.

5. Verfahren gemäß einem der Ansprüche 1 bis 4, in dem die Matrize die Elemente enthält, welche die Expression besagten oder besagter Zielgene erlauben.

6. Verfahren gemäß einem der vorhergehenden Ansprüche, in dem die auf dem DNA-Chip synthetisierten Oligonukleotide besagter Bank über ein spaltbares intermediäres Spacer-Molekül an besagtem DNA-Chip gebunden sind und in dem besagte Oligonukleotide als Gemisch in Lösung gebracht werden, indem die an besagtem DNA-Chip gebundenen Oligonukleotide Bedingungen ausgesetzt werden, die mit einem Spalten des Spacer-Moleküls verbunden sind.

7. Verfahren gemäß Anspruch 6, in dem besagtes Spacer-Molekül in basischem Milieu durch Licht- oder Enzymwirkung gespalten werden kann.

8. Verfahren gemäß Anspruch 7, in dem besagtes Spacer-Molekül in basischem Milieu gespalten werden kann.

9. Verfahren gemäß Anspruch 8, in dem besagtes Spacer-Molekül die Verbindung mit folgender Formel ist:

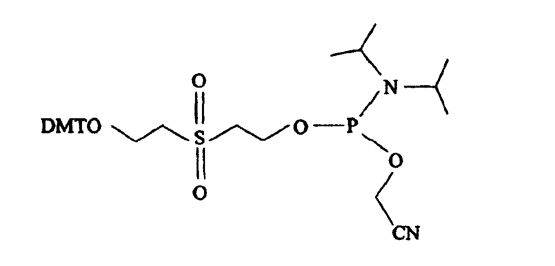

10. Verfahren gemäß Anspruch 8, in dem besagtes Spacer-Molekül die Verbindung mit folgender Formel ist:

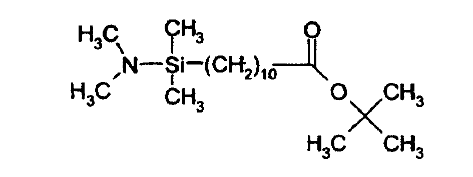

11. Verfahren gemäß einem der Ansprüche 3 bis 10, in dem Schritt iv) mithilfe eines für methylierte DNA-Stränge spezifischen Restriktionsenzyms durchgeführt wird, das vorzugsweise zu der Gruppe von Enzymen gehört: DpnI, NanII, NmuDI oder NmuEI.

12. Verfahren gemäß einem der vorhergehenden Ansprüche, in dem die in Schritt a) synthetisierten Oligonukleotide alle zum gleichen Zielgen komplementär sind.

13. Verfahren gemäß einem der vorhergehenden Ansprüche, in dem alle zum gleichen Zielgen komplementären Oligonukleotide zum gleichen Strang besagten Zielgens komplementär sind.

14. Verfahren gemäß einem der vorhergehenden Ansprüche, in dem die in Schritt a) synthetisierte Oligonukleotid-Bank die Oligonukleotide enthält, welche die Mutationen tragen, welche die Einführung aller möglichen Substitutionen in jedes Codon besagten oder besagter Zielgene erlauben.

15. Verfahren gemäß einem der Ansprüche 1 bis 13, in dem die in Schritt a) synthetisierte Oligonukleotid-Bank die Oligonukleotide enthält, welche die Mutationen tragen, welche die Einführung der gleichen Aminosäure, vorzugsweise Alanin, in jedes Codon besagten oder besagter Zielgene erlauben.

16. Verfahren zur Mutagenese eines Zielproteins oder mehrerer Zielproteine, **dadurch gekennzeichnet, dass** das Verfahren die Herstellung einer Expressionsbank mutierter Gene von einem Zielgen, das für besagtes Protein codiert, oder von mehreren Zielgenen, die für besagte Proteine codieren, mithilfe des Verfahrens zur Herstellung einer Bank mutierter Gene gemäß Ansprüchen 1 bis 14 und anschließende Expression besagter mutierter Gene zur Herstellung einer Bank mutierter Proteine umfasst.


### SEQUENCE LISTING

<110> BIOMETHODES
<120> Procédé de mutagenèse dirigée massive
<130> B0248EP
   <150> FR0314892
   <151> 2003-12-18
<160> 325
<170> PatentIn version 3.1
<210> 1
   <211> 509
   <212> DNA
   <213> Homo sapiens
<220>
   <221> CDS
   <222> (13)..(498)
   <223>
<400> 1
   aggagggcca cc atg cga att tcg aaa cca cat ttg aga agt att tcc atc      51
                 Met Arg Ile Ser Lys Pro His Leu Arg Ser Ile Ser Ile
                 1               5                   10
   cag tgc tac ttg tgt tta ctt cta aac agt cat ttt cta act gaa gct        99
   Gln Cys Tyr Leu Cys Leu Leu Leu Asn Ser His Phe Leu Thr Glu Ala
       15                  20                  25
   ggc att cat gtc ttc att ttg ggc tgt ttc agt gca ggg ctt cct aaa       147
   Gly Ile His Val Phe Ile Leu Gly Cys Phe Ser Ala Gly Leu Pro Lys
   30                  35                  40                  45
   aca gaa gcc aac tgg gtg aat gta ata agt gat ttg aaa aaa att gaa       195
   Thr Glu Ala Asn Trp Val Asn Val Ile Ser Asp Leu Lys Lys Ile Glu
                   50                  55                  60
   gat ctt att caa tct atg cat att gat gct act tta tat acg gaa agt       243
   Asp Leu Ile Gln Ser Met His Ile Asp Ala Thr Leu Tyr Thr Glu Ser
               65                  70                  75
   gat gtt cac ccc agt tgc aaa gta aca gca atg aag tgc ttt ctc ttg       291
   Asp Val His Pro Ser Cys Lys Val Thr Ala Met Lys Cys Phe Leu Leu
           80                  85                  90
   gag tta caa gtt att tca ctt gag tcc gga gat gca agt att cat gat      339
   Glu Leu Gln Val Ile Ser Leu Glu Ser Gly Asp Ala Ser Ile His Asp
       95                  100                 105
   aca gta gaa aat ctg atc atc cta gca aac aac agt ttg tct tct aat      387
   Thr Val Glu Asn Leu Ile Ile Leu Ala Asn Asn Ser Leu Ser Ser Asn
   110                 115                 120                 125
   ggg aat gta aca gaa tct gga tgc aaa gaa tgt gag gaa ctg gag gaa      435
   Gly Asn Val Thr Glu Ser Gly Cys Lys Glu Cys Glu Glu Leu Glu Glu
                   130                 135                 140
   aaa aat att aaa gaa ttt ttg cag agt ttt gta cat att gtc caa atg      483
   Lys Asn Ile Lys Glu Phe Leu Gln Ser Phe Val His Ile Val Gln Met
               145                 150                 155
   ttc atc aac act tct tgattgcaat t                                     509
   Phe Ile Asn Thr Ser
           160
<210> 2
   <211> 162
   <212> PRT
   <213> Homo sapiens
<400> 2
   Met Arg Ile Ser Lys Pro His Leu Arg Ser Ile Ser Ile Gln Cys Tyr
   1               5                   10                  15
   Leu Cys Leu Leu Leu Asn Ser His Phe Leu Thr Glu Ala Gly Ile His
               20                  25                  30
   Val Phe Ile Leu Gly Cys Phe Ser Ala Gly Leu Pro Lys Thr Glu Ala
           35                  40                  45
   Asn Trp Val Asn Val Ile Ser Asp Leu Lys Lys Ile Glu Asp Leu Ile
       50                  55                  60
   Gln Ser Met His Ile Asp Ala Thr Leu Tyr Thr Glu Ser Asp Val His
   65                  70                  75                  80
   Pro Ser Cys Lys Val Thr Ala Met Lys Cys Phe Leu Leu Glu Leu Gln
                   85                  90                  95
   Val Ile Ser Leu Glu Ser Gly Asp Ala Ser Ile His Asp Thr Val Glu
               100                 105                 110
   Asn Leu Ile Ile Leu Ala Asn Asn Ser Leu Ser Ser Asn Gly Asn Val
           115                 120                 125
   Thr Glu Ser Gly Cys Lys Glu Cys Glu Glu Leu Glu Glu Lys Asn Ile
   130 135 140
   Lys Glu Phe Leu Gln Ser Phe Val His Ile Val Gln Met Phe Ile Asn
   145 150 155 160
   Thr Ser
<210> 3
   <211> 35
   <212> DNA ....
   <213> artificial sequence
<220>
   <223> amorce
   <400> 3
   tcaatctatg catattgcgg ctactttata tacgg 35
<210> 4
   <211> 35
   <212> DNA
   <213> artificial sequence
<220>
   <223> amorce
   <400> 4
   tcaatctatg catattttcg ctactttata tacgg 35
<210> 5
   <211> 35
   <212> DNA
   <213> artificial sequence
<220>
   <223> amorce
   <400> 5
   tcaatctatg catattattg ctactttata tacgg 35
<210> 6
   <211> 35
   <212> DNA
   <213> artificial sequence
<220>
   <223> amorce
   <400> 6
   tcaatctatg catattctgg ctactttata tacgg 35
<210> 7
   <211> 35
   <212> DNA
   <213> artificial sequence
<220>
   <223> amorce
   <400> 7
   tcaatctatg catattccgg ctactttata tacgg 35
<210> 8
   <211> 35
   <212> DNA
   <213> artificial sequence
<220>
   <223> amorce
   <400> 8
   tcaatctatg catattgtgg ctactttata tacgg 35
<210> 9
   <211> 35
   <212> DNA
   <213> artificial sequence
<220>
   <223> amorce
   <400> 9
   tcaatctatg catatttggg ctactttata tacgg 35
<210> 10
   <211> 35
   <212> DNA
   <213> artificial sequence
<220>
   <223> amorce
   <400> 10
   tcaatctatg catattatgg ctactttata tacgg 35
<210> 11
   <211> 35
   <212> DNA
   <213> artificial sequence
<220>
   <223> amorce
   <400> 11
   ttcgaaacca catttggcga gtatttccat ccagt 35
<210> 12
   <211> 35
   <212> DNA
   <213> artificial sequence
<220>
   <223> amorce
   <400> 12
   ttcgaaacca catttgttca gtatttccat ccagt 35
<210> 13
   <211> 35
   <212> DNA
   <213> artificial sequence
<220>
   <223> amorce
   <400> 13
   ttcgaaacca catttgatta gtatttccat ccagt 35
<210> 14
   <211> 35
   <212> DNA
   <213> artificial sequence
<220>
   <223> amorce
   <400> 14
   ttcgaaacca catttgctga gtatttccat ccagt 35
<210> 15
   <211> 35
   <212> DNA
   <213> artificial sequence
<220>
   <223> amorce
   <400> 15
   ttcgaaacca catttgccga gtatttccat ccagt 35
<210> 16
   <211> 35
   <212> DNA
   <213> artificial sequence
<220>
   <223> amorce
   <400> 16
   ttcgaaacca catttggtga gtatttccat ccagt 35
<210> 17
   <211> 35
   <212> DNA
   <213> artificial sequence
<220>
   <223> amorce
   <400> 17
   ttcgaaacca catttgtgga gtatttccat ccagt 35
<210> 18
   <211> 35
   <212> DNA
   <213> artificial sequence
<220>
   <223> amorce
   <400> 18
   ttcgaaacca catttgatga gtatttccat ccagt 35
<210> 19
   <211> 35
   <212> DNA
   <213> artificial sequence
<220>
   <223> amorce
   <400> 19
   tcattttcta actgaagcgg gcattcatgt cttca 35
<210> 20
   <211> 35
   <212> DNA
   <213> artificial sequence
<220>
   <223> amorce
   <400> 20
   tcattttcta actgaattcg gcattcatgt cttca 35
<210> 21
   <211> 35
   <212> DNA
   <213> artificial sequence

<220>
   <223> amorce
   <400> 21
   tcattttcta actgaaattg gcattcatgt cttca 35
<210> 22
   <211> 35
   <212> DNA
   <213> artificial sequence
<220>
   <223> amorce
   <400> 22
   tcattttcta actgaactgg gcattcatgt cttca 35
<210> 23
   <211> 35
   <212> DNA
   <213> artificial sequence
<220>
   <223> amorce
   <400> 23
   tcattttcta actgaaccgg gcattcatgt cttca 35
<210> 24
   <211> 35
   <212> DNA
   <213> artificial sequence
<220>
   <223> amorce
   <400> 24
   tcattttcta actgaagtgg gcattcatgt cttca 35
<210> 25
   <211> 35
   <212> DNA
   <213> artificial sequence
<220>
   <223> amorce
   <400> 25
   tcattttcta actgaatggg gcattcatgt cttca 35
<210> 26
   <211> 35
   <212> DNA ..
   <213> artificial sequence
<220>
   <223> amorce
   <400> 26
   tcattttcta actgaaatgg gcattcatgt cttca 35
<210> 27
   <211> 35
   <212> DNA
   <213> artificial sequence
<220>
   <223> amorce
   <400> 27
   ttttctaact gaagctgcga ttcatgtctt cattt 35
<210> 28
   <211> 35
   <212> DNA
   <213> artificial sequence
<220>
   <223> amorce
   <400> 28
   ttttctaact gaagctttca ttcatgtctt cattt 35
<210> 29
   <211> 35
   <212> DNA
   <213> artificial sequence
<220>
   <223> amorce
   <400> 29
   ttttctaact gaagctatta ttcatgtctt cattt 35
<210> 30
   <211> 35
   <212> DNA
   <213> artificial sequence
<220>
   <223> amorce
   <400> 30
   ttttctaact gaagctctga ttcatgtctt cattt 35
<210> 31
   <211> 35
   <212> DNA
   <213> artificial sequence
<220>
   <223> amorce
   <400> 31
   ttttctaact gaagctccga ttcatgtctt cattt 35
<210> 32
   <211> 35
   <212> DNA
   <213> artificial sequence
<220>
   <223> amorce
   <400> 32
   ttttctaact gaagctgtga ttcatgtctt cattt 35
<210> 33
   <211> 35
   <212> DNA
   <213> artificial sequence
<220>
   <223> amorce
   <400> 33
   ttttctaact gaagcttgga ttcatgtctt cattt 35
<210> 34
   <211> 35
   <212> DNA
   <213> artificial sequence
<220>
   <223> amorce
   <400> 34
   ttttctaact gaagctatga ttcatgtctt cattt 35
<210> 35
   <211> 35
   <212> DNA
   <213> artificial sequence
<220>
   <223> amorce
   <400> 35
   tttgaaaaaa attgaagcgc ttattcaatc tatgc 35
<210> 36
   <211> 35
   <212> DNA
   <213> artificial sequence
<220>
   <223> amorce
   <400> 36
   tttgaaaaaa attgaattcc ttattcaatc tatgc 35
<210> 37
   <211> 35
   <212> DNA
   <213> artificial sequence
<220>
   <223> amorce
   <400> 37
   tttgaaaaaa attgaaattc ttattcaatc tatgc 35
<210> 38
   <211> 35
   <212> DNA
   <213> artificial sequence
<220>
   <223> amorce
   <400> 38
   tttgaaaaaa attgaactgc ttattcaatc tatgc 35
<210> 39
   <211> 35
   <212> DNA
   <213> artificial sequence
<220>
   <223> amorce
   <400> 39
   tttgaaaaaa attgaaccgc ttattcaatc tatgc 35
<210> 40
   <211> 35
   <212> DNA
   <213> artificial sequence
<220>
   <223> amorce
   <400> 40
   tttgaaaaaa attgaagtgc ttattcaatc tatgc 35
<210> 41
   <211> 35
   <212> DNA
   <213> artificial sequence
<220>
   <223> amorce
   <400> 41
   tttgaaaaaa attgaatggc ttattcaatc tatgc 35
<210> 42
   <211> 35
   <212> DNA
   <213> artificial sequence
<220>
   <223> amorce
   <400> 42
   tttgaaaaaa attgaaatgc ttattcaatc tatgc 35
<210> 43
   <211> 35
   <212> DNA
   <213> artificial sequence
<220>
   <223> amorce
   <400> 43
   gttacaagtt atttcagcgg agtccggaga tgcaa 35
<210> 44
   <211> 35
   <212> DNA
   <213> artificial sequence

<220>
   <223> amorce
   <400> 44
   gttacaagtt atttcattcg agtccggaga tgcaa 35
<210> 45
   <211> 35
   <212> DNA
   <213> artificial sequence
<220>
   <223> amorce
   <400> 45
   gttacaagtt atttcaattg agtccggaga tgcaa 35
<210> 46
   <211> 35
   <212> DNA
   <213> artificial sequence
<220>
   <223> amorce
   <400> 46
   gttacaagtt atttcactgg agtccggaga tgcaa 35
<210> 47
   <211> 35
   <212> DNA
   <213> artificial sequence
<220>
   <223> amorce
   <400> 47
   gttacaagtt atttcaccgg agtccggaga tgcaa 35
<210> 48
   <211> 35
   <212> DNA
   <213> artificial sequence
<220>
   <223> amorce
   <400> 48
   gttacaagtt atttcagtgg agtccggaga tgcaa 35
<210> 49
   <211> 35
   <212> DNA
   <213> artificial sequence
<220>
   <223> amorce
   <400> 49
   gttacaagtt atttcatggg agtccggaga tgcaa 35
<210> 50
   <211> 35
   <212> DNA
   <213> artificial sequence
<220>
   <223> amorce
   <400> 50
   gttacaagtt atttcaatgg agtccggaga tgcaa 35
<210> 51
   <211> 35
   <212> DNA
   <213> artificial sequence
<220>
   <223> amorce
   <400> 51
   tcttattcaa tctatggcga ttgatgctac tttat 35
<210> 52
   <211> 35
   <212> DNA
   <213> artificial sequence
<220>
   <223> amorce
   <400> 52
   tcttattcaa tctatgttca ttgatgctac tttat 35
<210> 53
   <211> 35
   <212> DNA
   <213> artificial sequence
<220>
   <223> amorce
   <400> 53
   tcttattcaa tctatgatta ttgatgctac tttat 35
<210> 54
   <211> 35
   <212> DNA
   <213> artificial sequence
<220>
   <223> amorce
   <400> 54
   tcttattcaa tctatgctga ttgatgctac tttat 35
<210> 55
   <211> 35
   <212> DNA
   <213> artificial sequence
<220>
   <223> amorce
   <400> 55
   tcttattcaa tctatgccga ttgatgctac tttat 35
<210> 56
   <211> 35
   <212> DNA
   <213> artificial sequence
<220>
   <223> amorce
   <400> 56
   tcttattcaa tctatggtga ttgatgctac tttat 35
<210> 57
   <211> 35
   <212> DNA
   <213> artificial sequence
<220>
   <223> amorce
   <400> 57
   tcttattcaa tctatgtgga ttgatgctac tttat 35
<210> 58
   <211> 35
   <212> DNA
   <213> artificial sequence
<220>
   <223> amorce
   <400> 58
   tcttattcaa tctatgatga ttgatgctac tttat 35
<210> 59
   <211> 35
   <212> DNA
   <213> artificial sequence
<220>
   <223> amorce
   <400> 59
   cagttgcaaa gtaacagcga tgaagtgctt tctct 35
<210> 60
   <211> 35
   <212> DNA .
   <213> artificial sequence
<220>
   <223> amorce
   <400> 60
   cagttgcaaa gtaacattca tgaagtgctt tctct 35
<210> 61
   <211> 35
   <212> DNA
   <213> artificial sequence
<220>
   <223> amorce
   <400> 61
   cagttgcaaa gtaacaatta tgaagtgctt tctct 35
<210> 62
   <211> 35
   <212> DNA
   <213> artificial sequence
<220>
   <223> amorce
   <400> 62
   cagttgcaaa gtaacactga tgaagtgctt tctct 35
<210> 63
   <211> 35
   <212> DNA
   <213> artificial sequence
<220>
   <223> amorce
   <400> 63
   cagttgcaaa gtaacaccga tgaagtgctt tctct 35
<210> 64
   <211> 35
   <212> DNA
   <213> artificial sequence
<220>
   <223> amorce
   <400> 64
   cagttgcaaa gtaacagtga tgaagtgctt tctct 35
<210> 65
   <211> 35
   <212> DNA
   <213> artificial sequence
<220>
   <223> amorce
   <400> 65
   cagttgcaaa gtaacatgga tgaagtgctt tctct 35
<210> 66
   <211> 35
   <212> DNA
   <213> artificial sequence
<220>
   <223> amorce
   <400> 66
   cagttgcaaa gtaacaatga tgaagtgctt tctct 35
<210> 67
   <211> 35
   <212> DNA
   <213> artificial sequence

<220>
   <223> amorce
   <400> 67
   cggagatgca agtattgcgg atacagtaga aaatc 35
<210> 68
   <211> 35
   <212> DNA
   <213> artificial sequence
<220>
   <223> amorce
   <400> 68
   cggagatgca agtattttcg atacagtaga aaatc 35
<210> 69
   <211> 35
   <212> DNA
   <213> artificial sequence
<220>
   <223> amorce
   <400> 69
   cggagatgca agtattattg atacagtaga aaatc 35
<210> 70
   <211> 35
   <212> DNA
   <213> artificial sequence
<220>
   <223> amorce
   <400> 70
   cggagatgca agtattctgg atacagtaga aaatc 35
<210> 71
   <211> 35
   <212> DNA
   <213> artificial sequence
<220>
   <223> amorce
   <400> 71
   cggagatgca agtattccgg atacagtaga aaatc 35
<210> 72
   <211> 35
   <212> DNA
   <213> artificial sequence
<220>
   <223> amorce
   <400> 72
   cggagatgca agtattgtgg atacagtaga aaatc 35
<210> 73
   <211> 35
   <212> DNA
   <213> artificial sequence
<220>
   <223> amorce
   <400> 73
   cggagatgca agtatttggg atacagtaga aaatc 35
<210> 74
   <211> 35
   <212> DNA
   <213> artificial sequence
<220>
   <223> amorce
   <400> 74
   cggagatgca agtattatgg atacagtaga aaatc 35
<210> 75
   <211> 35
   <212> DNA
   <213> artificial sequence
<220>
   <223> amorce
   <400> 75
   agaaaatctg atcatcgcgg caaacaacag tttgt 35
<210> 76
   <211> 35
   <212> DNA
   <213> artificial sequence
<220>
   <223> amorce
   <400> 76
   agaaaatctg atcatcttcg caaacaacag tttgt 35
<210> 77
   <211> 35
   <212> DNA
   <213> artificial sequence
<220>
   <223> amorce
   <400> 77
   agaaaatctg atcatcattg caaacaacag tttgt 35
<210> 78
   <211> 35
   <212> DNA
   <213> artificial sequence
<220>
   <223> amorce
   <400> 78
   agaaaatctg atcatcctgg caaacaacag tttgt 35
<210> 79
   <211> 35
   <212> DNA
   <213> artificial sequence
<220>
   <223> amorce
   <400> 79
   agaaaatctg atcatcccgg caaacaacag tttgt 35
<210> 80
   <211> 35
   <212> DNA
   <213> artificial sequence
<220>
   <223> amorce
   <400> 80
   agaaaatctg atcatcgtgg caaacaacag tttgt 35
<210> 81
   <211> 35
   <212> DNA
   <213> artificial sequence
<220>
   <223> amorce
   <400> 81
   agaaaatctg atcatctggg caaacaacag tttgt 35
<210> 82
   <211> 35
   <212> DNA
   <213> artificial sequence
<220>
   <223> amorce
   <400> 82
   agaaaatctg atcatcatgg caaacaacag tttgt 35

<210> 83
   <211> 35
   <212> DNA
   <213> artificial sequence
<220>
   <223> amorce
   <400> 83
   ggaactggag gaaaaagcga ttaaagaatt tttgc 35
<210> 84
   <211> 35
   <212> DNA
   <213> artificial sequence
<220>
   <223> amorce
   <400> 84
   ggaactggag gaaaaattca ttaaagaatt tttgc 35
<210> 85
   <211> 35
   <212> DNA
   <213> artificial sequence
<220>
   <223> amorce
   <400> 85
   ggaactggag gaaaaaatta ttaaagaatt tttgc 35
<210> 86
   <211> 35
   <212> DNA
   <213> artificial sequence
<220>
   <223> amorce
   <400> 86
   ggaactggag gaaaaactga ttaaagaatt tttgc 35
<210> 87
   <211> 35
   <212> DNA
   <213> artificial sequence
<220>
   <223> amorce
   <400> 87
   ggaactggag gaaaaaccga ttaaagaatt tttgc 35
<210> 88
   <212> 35
   <212> DNA
   <213> artificial sequence
<220>
   <223> amorce
   <400> 88
   ggaactggag gaaaaagtga ttaaagaatt tttgc 35
<210> 89
   <211> 35
   <212> DNA
   <213> artificial sequence
<220>
   <223> amorce
   <400> 89
   ggaactggag gaaaaatgga ttaaagaatt tttgc 35
<210> 90
   <211> 35
   <212> DNA
   <213> artificial sequence
<220>
   <223> amorce
   <400> 90
   ggaactggag gaaaaaatga ttaaagaatt tttgc 35
<210> 91
   <211> 35
   <212> DNA
   <213> artificial sequence
<220>
   <223> amorce
   <400> 91
   atttttgcag agttttgcgc atattgtcca aatgt 35
<210> 92
   <211> 35
   <212> DNA
   <213> artificial sequence
<220>
   <223> amorce
   <400> 92
   atttttgcag agttttttcc atattgtcca aatgt 35
<210> 93
   <211> 35
   <212> DNA
   <213> artificial sequence
<220>
   <223> amorce
   <400> 93
   atttttgcag agttttattc atattgtcca aatgt 35
<210> 94
   <211> 35
   <212> DNA
   <213> artificial sequence
<220>
   <223> amorce
   <400> 94
   atttttgcag agttttctgc atattgtcca aatgt 35
<210> 95
   <211> 35
   <212> DNA
   <213> artificial sequence
<220>
   <223> amorce
   <400> 95
   atttttgcag agttttccgc atattgtcca aatgt 35
<210> 96
   <211> 35
   <212> DNA
   <213> artificial sequence
<220>
   <223> amorce
   <400> 96
   atttttgcag agttttgtgc atattgtcca aatgt 35
<210> 97
   <211> 35
   <212> DNA
   <213> artificial sequence
<220>
   <223> amorce
   <400> 97
   atttttgcag agtttttggc atattgtcca aatgt 35
<210> 98
   <211> 35
   <212> DNA
   <213> artificial sequence
<220>
   <223> amorce
   <400> 98
   atttttgcag agttttatgc atattgtcca aatgt 35
<210> 99
   <211> 35
   <212> DNA
   <213> artificial sequence
<220>
   <223> amorce
   <400> 99
   tttgcagagt tttgtagcga ttgtccaaat gttca 35
<210> 100
   <211> 35
   <212> DNA
   <213> artificial sequence
<220>
   <223> amorce
   <400> 100
   tttgcagagt tttgtattca ttgtccaaat gttca 35
<210> 101
   <211> 35
   <212> DNA
   <213> artificial sequence
<220>
   <223> amorce
   <400> 101
   tttgcagagt tttgtaatta ttgtccaaat gttca 35
<210> 102
   <211> 35
   <212> DNA
   <213> artificial sequence
<220>
   <223> amorce
   <400> 102
   tttgcagagt tttgtactga ttgtccaaat gttca 35
<210> 103
   <211> 35
   <212> DNA
   <213> artificial sequence
<220>
   <223> amorce
   <400> 103
   tttgcagagt tttgtaccga ttgtccaaat gttca 35
<210> 104
   <211> 35
   <212> DNA
   <213> artificial sequence
<220>
   <223> amorce
   <400> 104
   tttgcagagt tttgtagtga ttgtccaaat gttca 35
<210> 105
   <211> 35
   <212> DNA
   <213> artificial sequence
<220>
   <223> amorce
   <400> 105
   tttgcagagt tttgtatgga ttgtccaaat gttca 35
<210> 106
   <211> 35
   <212> DNA
   <213> artificial sequence
<220>
   <223> amorce
   <400> 106
   tttgcagagt tttgtaatga ttgtccaaat gttca 35
<210> 107
   <211> 35
   <212> DNA
   <213> artificial sequence
<220>
   <223> amorce
   <400> 107
   agttatttca cttgaggcgg gagatgcaag tattc 35
<210> 108
   <211> 35
   <212> DNA
   <213> artificial sequence
<220>
   <223> amorce
   <400> 108
   agttatttca cttgagttcg gagatgcaag tattc 35
<210> 109
   <211> 35
   <212> DNA
   <213> artificial sequence
<220>
   <223> amorce
   <400> 109
   agttatttca cttgagattg gagatgcaag tattc 35
<210> 110
   <211> 35
   <212> DNA
   <213> artificial sequence
<220>
   <223> amorce
   <400> 110
   agttatttca cttgagctgg gagatgcaag tattc 35
<210> 111
   <211> 35
   <212> DNA
   <213> artificial sequence
<220>
   <223> amorce
   <400> 111
   agttatttca cttgagccgg gagatgcaag tattc 35
<210> 112
   <211> 35
   <212> DNA
   <213> artificial sequence
<220>
   <223> amorce
   <400> 112
   agttatttca cttgaggtgg gagatgcaag tattc 35
<210> 113
   <211> 35
   <212> DNA
   <213> artificial sequence
<220>
   <223> amorce
   <400> 113
   agttatttca cttgagtggg gagatgcaag tattc 35
<210> 114
   <211> 35
   <212> DNA
   <213> artificial sequence
<220>
   <223> amorce
   <400> 114
   agttatttca cttgagatgg gagatgcaag tattc 35
<210> 115
   <211> 35
   <212> DNA
   <213> artificial sequence
<220>
   <223> amorce
   <400> 115
   taatgggaat gtaacagcgt ctggatgcaa agaat 35
<210> 116
   <211> 35
   <212> DNA
   <213> artificial sequence
<220>
   <223> amorce
   <400> 116
   taatgggaat gtaacattct ctggatgcaa agaat 35
<210> 117
   <211> 35
   <212> DNA
   <213> artificial sequence

<220>
   <223> amorce
   <400> 117
   taatgggaat gtaacaattt ctggatgcaa agaat 35
<210> 118
   <211> 35
   <212> DNA
   <213> artificial sequence
<220>
   <223> amorce
   <400> 118
   taatgggaat gtaacactgt ctggatgcaa agaat 35
<210> 119
   <211> 35
   <212> DNA
   <213> artificial sequence
<220>
   <223> amorce
   <400> 119
   taatgggaat gtaacaccgt ctggatgcaa agaat 35
<210> 120
   <211> 35
   <212> DNA
   <213> artificial sequence
<220>
   <223> amorce
   <400> 120
   taatgggaat gtaacagtgt ctggatgcaa agaat 35
<210> 121
   <211> 35
   <212> DNA
   <213> artificial sequence
<220>
   <223> amorce
   <400> 121
   taatgggaat gtaacatggt ctggatgcaa agaat 35
<210> 122
   <211> 35
   <212> DNA
   <213> artificial sequence
<220>
   <223> amorce
   <400> 122
   taatgggaat gtaacaatgt ctggatgcaa agaat 35
<210> 123
   <211> 35
   <212> DNA
   <213> artificial sequence
<220>
   <223> amorce
   <400> 123
   ggaggaaaaa aatattgcgg aatttttgca gagtt 35
<210> 124
   <211> 35
   <212> DNA
   <213> artificial sequence
<220>
   <223> amorce
   <400> 124
   ggaggaaaaa aatattttcg aatttttgca gagtt 35
<210> 125
   <211> 35
   <212> DNA
   <213> artificial sequence
<220>
   <223> amorce
   <400> 125
   ggaggaaaaa aatattattg aatttttgca gagtt 35
<210> 126
   <211> 35
   <212> DNA
   <213> artificial sequence
<220>
   <223> amorce
   <400> 126
   ggaggaaaaa aatattctgg aatttttgca gagtt 35
<210> 127
   <211> 35
   <212> DNA
   <213> artificial sequence
<220>
   <223> amorce
   <400> 127
   ggaggaaaaa aatattccgg aatttttgca gagtt 35
<210> 128
   <211> 35
   <212> DNA
   <213> artificial sequence
<220>
   <223> amorce
   <400> 128
   ggaggaaaaa aatattgtgg aatttttgca gagtt 35
<210> 129
   <211> 35
   <212> DNA
   <213> artificial sequence
<220>
   <223> amorce
   <400> 129
   ggaggaaaaa aatatttggg aatttttgca gagtt 35
<210> 130
   <211> 35
   <212> DNA
   <213> artificial sequence
<220>
   <223> amorce
   <400> 130
   ggaggaaaaa aatattatgg aatttttgca gagtt 35
<210> 131
   <211> 35
   <212> DNA
   <213> artificial sequence
<220>
   <223> amorce
   <400> 131
   acaagttatt tcacttgcgt ccggagatgc aagta 35
<210> 132
   <211> 35
   <212> DNA
   <213> artificial sequence
<220>
   <223> amorce
   <400> 132
   acaagttatt tcacttttct ccggagatgc aagta 35
<210> 133
   <211> 35
   <212> DNA
   <213> artificial sequence
<220>
   <223> amorce
   <400> 133
   acaagttatt tcacttattt ccggagatgc aagta 35
<210> 134
   <211> 35
   <212> DNA
   <213> artificial sequence
<220>
   <223> amorce
   <400> 134
   acaagttatt tcacttctgt ccggagatgc aagta 35
<210> 135
   <211> 35
   <212> DNA
   <213> artificial sequence
<220>
   <223> amorce
   <400> 135
   acaagttatt tcacttccgt ccggagatgc aagta 35
<210> 136
   <211> 35
   <212> DNA
   <213> artificial sequence
<220>
   <223> amorce
   <400> 136
   acaagttatt tcacttgtgt ccggagatgc aagta 35
<210> 137
   <211> 35
   <212> DNA
   <213> artificial sequence
<220>
   <223> amorce
   <400> 137
   acaagttatt tcactttggt ccggagatgc aagta 35
<210> 138
   <211> 35
   <212> DNA
   <213> artificial sequence
<220>
   <223> amorce
   <400> 138
   acaagttatt tcacttatgt ccggagatgc aagta 35
<210> 139
   <211> 35
   <212> DNA
   <213> artificial sequence
<220>
   <223> amorce
   <400> 139
   ggccaccatg cgaattgcga aaccacattt gagaa 35
<210> 140
   <211> 35
   <212> DNA
   <213> artificial sequence

<220>
   <223> amorce
   <400> 140
   ggccaccatg cgaattttca aaccacattt gagaa 35
<210> 141
   <211> 35
   <212> DNA
   <213> artificial sequence
<220>
   <223> amorce
   <400> 141
   ggccaccatg cgaattatta aaccacattt gagaa 35
<210> 142
   <211> 35
   <212> DNA
   <213> artificial sequence
<220>
   <223> amorce
   <400> 142
   ggccaccatg cgaattctga aaccacattt gagaa 35
<210> 143
   <211> 35
   <212> DNA
   <213> artificial sequence
<220>
   <223> amorce
   <400> 143
   ggccaccatg cgaattccga aaccacattt gagaa 35
<210> 144
   <211> 35
   <212> DNA
   <213> artificial sequence
<220>
   <223> amorce
   <400> 144
   ggccaccatg cgaattgtga aaccacattt gagaa 35
<210> 145
   <211> 35
   <212> DNA
   <213> artificial sequence
<220>
   <223> amorce
   <400> 145
   ggccaccatg cgaatttgga aaccacattt gagaa 35
<210> 146
   <211> 35
   <212> DNA
   <213> artificial sequence
<220>
   <223> amorce
   <400> 146
   ggccaccatg cgaattatga aaccacattt gagaa 35
<210> 147
   <211> 35
   <212> DNA
   <213> artificial sequence
<220>
   <223> amorce
   <400> 147
   tcctaaaaca gaagccgcgt gggtgaatgt aataa 35
<210> 148
   <211> 35
   <212> DNA
   <213> artificial sequence
<220>
   <223> amorce
   <400> 148
   tcctaaaaca gaagccttct gggtgaatgt aataa 35
<210> 149
   <211> 35
   <212> DNA
   <213> artificial sequence
<220>
   <223> amorce
   <400> 149
   tcctaaaaca gaagccattt gggtgaatgt aataa 35
<210> 150
   <211> 35
   <212> DNA
   <213> artificial sequence
<220>
   <223> amorce
   <400> 150
   tcctaaaaca gaagccctgt gggtgaatgt aataa 35
<210> 151
   <211> 35
   <212> DNA
   <213> artificial sequence
<220>
   <223> amorce
   <400> 151
   tcctaaaaca gaagccccgt gggtgaatgt aataa 35
<210> 152
   <211> 35
   <212> DNA
   <213> artificial sequence
<220>
   <223> amorce
   <400> 152
   tcctaaaaca gaagccgtgt gggtgaatgt aataa 35
<210> 153
   <211> 35
   <212> DNA
   <213> artificial sequence
<220>
   <223> amorce
   <400> 153
   tcctaaaaca gaagcctggt gggtgaatgt aataa 35
<210> 154
   <211> 35
   <212> DNA
   <213> artificial sequence
<220>
   <223> amorce
   <400> 154
   tcctaaaaca gaagccatgt gggtgaatgt aataa 35
<210> 155
   <211> 35
   <212> DNA
   <213> artificial sequence
<220>
   <223> amorce
   <400> 155
   tgtccaaatg ttcatcgcga cttcttgatt gcaat 35
<210> 156
   <211> 35
   <212> DNA
   <213> artificial sequence
<220>
   <223> amorce
   <400> 156
   tgtccaaatg ttcatcttca cttcttgatt gcaat 35
<210> 157
   <211> 35
   <212> DNA
   <213> artificial sequence
<220>
   <223> amorce
   <400> 157
   tgtccaaatg ttcatcatta cttcttgatt gcaat 35
<210> 158
   <211> 35
   <212> DNA
   <213> artificial sequence
<220>
   <223> amorce
   <400> 158
   tgtccaaatg ttcatcctga cttcttgatt gcaat 35
<210> 159
   <211> 35
   <212> DNA
   <213> artificial sequence
<220>
   <223> amorce
   <400> 159
   tgtccaaatg ttcatcccga cttcttgatt gcaat 35
<210> 160
   <211> 35
   <212> DNA
   <213> artificial sequence
<220>
   <223> amorce
   <400> 160
   tgtccaaatg ttcatcgtga cttcttgatt gcaat 35
<210> 161
   <211> 35
   <212> DNA
   <213> artificial sequence
<220>
   <223> amorce
   <400> 161
   tgtccaaatg ttcatctgga cttcttgatt gcaat 35
<210> 162
   <211> 35
   <212> DNA
   <213> artificial sequence
<220>
   <223> amorce
   <400> 162
   tgtccaaatg ttcatcatga cttcttgatt gcaat 35
<210> 163
   <211> 35
   <212> DNA
   <213> artificial sequence

<220>
   <223> amorce
   <400> 163
   aattgaagat cttattgcgt ctatgcatat tgatg 35
<210> 164
   <211> 35
   <212> DNA
   <213> artificial sequence
<220>
   <223> amorce
   <400> 164
   aattgaagat cttattttct ctatgcatat tgatg 35
<210> 165
   <211> 35
   <212> DNA
   <213> artificial sequence
<220>
   <223> amorce
   <400> 165
   aattgaagat cttattattt ctatgcatat tgatg 35
<210> 166
   <211> 35
   <212> DNA
   <213> artificial sequence
<220>
   <223> amorce
   <400> 166
   aattgaagat cttattctgt ctatgcatat tgatg 35
<210> 167
   <211> 35
   <212> DNA
   <213> artificial sequence
<220>
   <223> amorce
   <400> 167
   aattgaagat cttattccgt ctatgcatat tgatg 35
<210> 168
   <211> 35
   <212> DNA
   <213> artificial sequence
<220>
   <223> amorce
   <400> 168
   aattgaagat cttattgtgt ctatgcatat tgatg 35
<210> 169
   <211> 35
   <212> DNA
   <213> artificial sequence
<220>
   <223> amorce
   <400> 169
   aattgaagat cttatttggt ctatgcatat tgatg 35
<210> 170
   <211> 35
   <212> DNA
   <213> artificial sequence
<220>
   <223> amorce
   <400> 170
   aattgaagat cttattatgt ctatgcatat tgatg 35
<210> 171
   <211> 35
   <212> DNA
   <213> artificial sequence
<220>
   <223> amorce
   <400> 171
   tgctacttta tatacggcga gtgatgttca cccca 35
<210> 172
   <211> 35
   <212> DNA
   <213> artificial sequence
<220>
   <223> amorce
   <400> 172
   tgctacttta tatacgttca gtgatgttca cccca 35
<210> 173
   <211> 35
   <212> DNA
   <213> artificial sequence
<220>
   <223> amorce
   <400> 173
   tgctacttta tatacgatta gtgatgttca cccca 35
<210> 174
   <211> 35
   <212> DNA
   <213> artificial sequence
<220>
   <223> amorce
   <400> 174
   tgctacttta tatacgctga gtgatgttca cccca 35
<210> 175
   <211> 35
   <212> DNA
   <213> artificial sequence
<220>
   <223> amorce
   <400> 175
   tgctacttta tatacgccga gtgatgttca cccca 35
<210> 176
   <211> 35
   <212> DNA
   <213> artificial sequence
<220>
   <223> amorce
   <400> 176
   tgctacttta tatacggtga gtgatgttca cccca 35
<210> 177
   <211> 35
   <212> DNA
   <213> artificial sequence
<220>
   <223> amorce
   <400> 177
   tgctacttta tatacgtgga gtgatgttca cccca 35
<210> 178
   <211> 35
   <212> DNA
   <213> artificial sequence
<220>
   <223> amorce
   <400> 178
   tgctacttta tatacgatga gtgatgttca cccca 35
<210> 179
   <211> 35
   <212> DNA
   <213> artificial sequence
<220>
   <223> amorce
   <400> 179
   tcaccccagt tgcaaagcga cagcaatgaa gtgct 35
<210> 180
   <211> 35
   <212> DNA
   <213> artificial sequence
<220>
   <223> amorce
   <400> 180
   tcaccccagt tgcaaattca cagcaatgaa gtgct 35
<210> 181
   <211> 35
   <212> DNA
   <213> artificial sequence
<220>
   <223> amorce
   <400> 181
   tcaccccagt tgcaaaatta cagcaatgaa gtgct 35
<210> 182
   <211> 35
   <212> DNA
   <213> artificial sequence

<220>
   <223> amorce
   <400> 182
   tcaccccagt tgcaaactga cagcaatgaa gtgct 35
<210> 183
   <211> 35
   <212> DNA
   <213> artificial sequence
<220>
   <223> amorce
   <400> 183
   tcaccccagt tgcaaaccga cagcaatgaa gtgct 35
<210> 184
   <211> 35
   <212> DNA
   <213> artificial sequence
<220>
   <223> amorce
   <400> 184
   tcaccccagt tgcaaagtga cagcaatgaa gtgct 35
<210> 185
   <211> 35
   <212> DNA
   <213> artificial sequence
<220>
   <223> amorce
   <400> 185
   tcaccccagt tgcaaatgga cagcaatgaa gtgct 35
<210> 186
   <211> 35
   <212> DNA
   <213> artificial sequence
<220>
   <223> amorce
   <400> 186
   tcaccccagt tgcaaaatga cagcaatgaa gtgct 35
<210> 187
   <211> 35
   <212> DNA
   <213> artificial sequence
<220>
   <223> amorce
   <400> 187
   gaagtgcttt ctcttggcgt tacaagttat ttcac 35
<210> 188
   <211> 35
   <212> DNA
   <213> artificial sequence
<220>
   <223> amorce
   <400> 188
   gaagtgcttt ctcttgttct tacaagttat ttcac 35
<210> 189
   <211> 35
   <212> DNA
   <213> artificial sequence
<220>
   <223> amorce
   <400> 189
   gaagtgcttt ctcttgattt tacaagttat ttcac 35
<210> 190
   <211> 35
   <212> DNA
   <213> artificial sequence
<220>
   <223> amorce
   <400> 190
   gaagtgcttt ctcttgctgt tacaagttat ttcac 35
<210> 191
   <211> 35
   <212> DNA
   <213> artificial sequence
<220>
   <223> amorce
   <400> 191
   gaagtgcttt ctcttgccgt tacaagttat ttcac 35
<210> 192
   <211> 35
   <212> DNA
   <213> artificial sequence
<220>
   <223> amorce
   <400> 192
   gaagtgcttt ctcttggtgt tacaagttat ttcac 35
<210> 193
   <211> 35
   <212> DNA
   <213> artificial sequence
<220>
   <223> amorce
   <400> 193
   gaagtgcttt ctcttgtggt tacaagttat ttcac 35
<210> 194
   <211> 35
   <212> DNA
   <213> artificial sequence
<220>
   <223> amorce
   <400> 194
   gaagtgcttt ctcttgatgt tacaagttat ttcac 35
<210> 195
   <211> 35
   <212> DNA
   <213> artificial sequence
<220>
   <223> amorce
   <400> 195
   ctgggtgaat gtaatagcgg atttgaaaaa aattg 35
<210> 196
   <211> 35
   <212> DNA
   <213> artificial sequence
<220>
   <223> amorce
   <400> 196
   ctgggtgaat gtaatattcg atttgaaaaa aattg 35
<210> 197
   <211> 35
   <212> DNA
   <213> artificial sequence
<220>
   <223> amorce
   <400> 197
   ctgggtgaat gtaataattg atttgaaaaa aattg 35
<210> 198
   <211> 35
   <212> DNA
   <213> artificial sequence
<220>
   <223> amorce
   <400> 198
   ctgggtgaat gtaatactgg atttgaaaaa aattg 35
<210> 199
   <211> 35
   <212> DNA
   <213> artificial sequence
<220>
   <223> amorce
   <400> 199
   ctgggtgaat gtaataccgg atttgaaaaa aattg 35
<210> 200
   <211> 35
   <212> DNA
   <213> artificial sequence
<220>
   <223> amorce
   <400> 200
   ctgggtgaat gtaatagtgg atttgaaaaa aattg 35
<210> 201
   <211> 35
   <212> DNA
   <213> artificial sequence
<220>
   <223> amorce
   <400> 201
   ctgggtgaat gtaatatggg atttgaaaaa aattg 35
<210> 202
   <211> 35
   <212> DNA
   <213> artificial sequence
<220>
   <223> amorce
   <400> 202
   ctgggtgaat gtaataatgg atttgaaaaa aattg 35
<210> 203
   <211> 35
   <212> DNA
   <213> artificial sequence
<220>
   <223> amorce
   <400> 203
   tatgcatatt gatgctgcgt tatatacgga aagtg 35
<210> 204
   <211> 35
   <212> DNA
   <213> artificial sequence
<220>
   <223> amorce
   <400> 204
   tatgcatatt gatgctttct tatatacgga aagtg 35
<210> 205
   <211> 35
   <212> DNA
   <213> artificial sequence
<220>
   <223> amorce
   <400> 205
   tatgcatatt gatgctattt tatatacgga aagtg 35
<210> 206
   <211> 35
   <212> DNA
   <213> artificial sequence
<220>
   <223> amorce
   <400> 206
   tatgcatatt gatgctctgt tatatacgga aagtg 35
<210> 207
   <211> 35
   <212> DNA
   <213> artificial sequence
<220>
   <223> amorce
   <400> 207
   tatgcatatt gatgctccgt tatatacgga aagtg 35
<210> 208
   <211> 35
   <212> DNA
   <213> artificial sequence
<220>
   <223> amorce
   <400> 208
   tatgcatatt gatgctgtgt tatatacgga aagtg 35
<210> 209
   <211> 35
   <212> DNA
   <213> artificial sequence
<220>
   <223> amorce
   <400> 209
   tatgcatatt gatgcttggt tatatacgga aagtg 35
<210> 210
   <211> 35
   <212> DNA
   <213> artificial sequence
<220>
   <223> amorce
   <400> 210
   tatgcatatt gatgctatgt tatatacgga aagtg 35
<210> 211
   <211> 35
   <212> DNA
   <213> artificial sequence
<220>
   <223> amorce
   <400> 211
   tacggaaagt gatgttgcgc ccagttgcaa agtaa 35
<210> 212
   <211> 35
   <212> DNA
   <213> artificial sequence
<220>
   <223> amorce
   <400> 212
   tacggaaagt gatgttttcc ccagttgcaa agtaa 35
<210> 213
   <211> 35
   <212> DNA
   <213> artificial sequence
<220>
   <223> amorce
   <400> 213
   tacggaaagt gatgttattc ccagttgcaa agtaa 35
<210> 214
   <211> 35
   <212> DNA
   <213> artificial sequence
<220>
   <223> amorce
   <400> 214
   tacggaaagt gatgttctgc ccagttgcaa agtaa 35
<210> 215
   <211> 35
   <212> DNA
   <213> artificial sequence
<220>
   <223> amorce
   <400> 215
   tacggaaagt gatgttccgc ccagttgcaa agtaa 35
<210> 216
   <211> 35
   <212> DNA
   <213> artificial sequence
<220>
   <223> amorce
   <400> 216
   tacggaaagt gatgttgtgc ccagttgcaa agtaa 35
<210> 217
   <211> 35
   <212> DNA
   <213> artificial sequence
<220>
   <223> amorce
   <400> 217
   tacggaaagt gatgtttggc ccagttgcaa agtaa 35
<210> 218
   <211> 35
   <212> DNA
   <213> artificial sequence
<220>
   <223> amorce
   <400> 218
   tacggaaagt gatgttatgc ccagttgcaa agtaa 35
<210> 219
   <211> 35
   <212> DNA
   <213> artificial sequence
<220>
   <223> amorce
   <400> 219
   agtaacagca atgaaggcgt ttctcttgga gttac 35
<210> 220
   <211> 35
   <212> DNA
   <213> artificial sequence
<220>
   <223> amorce
   <400> 220
   agtaacagca atgaagttct ttctcttgga gttac 35

<210> 221
   <211> 35
   <212> DNA
   <213> artificial sequence
<220>
   <223> amorce
   <400> 221
   agtaacagca atgaagattt ttctcttgga gttac 35
<210> 222
   <211> 35
   <212> DNA
   <213> artificial sequence
<220>
   <223> amorce
   <400> 222
   agtaacagca atgaagctgt ttctcttgga gttac 35
<210> 223
   <211> 35
   <212> DNA
   <213> artificial sequence
<220>
   <223> amorce
   <400> 223
   agtaacagca atgaagccgt ttctcttgga gttac 35
<210> 224
   <211> 35
   <212> DNA
   <213> artificial sequence
<220>
   <223> amorce
   <400> 224
   agtaacagca atgaaggtgt ttctcttgga gttac 35
<210> 225
   <211> 35
   <212> DNA
   <213> artificial sequence
<220>
   <223> amorce
   <400> 225
   agtaacagca atgaagtggt ttctcttgga gttac 35
<210> 226
   <211> 35
   <212> DNA
   <213> artificial sequence
<220>
   <223> amorce
   <400> 226
   agtaacagca atgaagatgt ttctcttgga gttac 35
<210> 227
   <211> 35
   <212> DNA
   <213> artificial sequence
<220>
   <223> amorce
   <400> 227
   tctcttggag ttacaagcga tttcacttga gtccg 35
<210> 228
   <211> 35
   <212> DNA
   <213> artificial sequence
<220>
   <223> amorce
   <400> 228
   tctcttggag ttacaattca tttcacttga gtccg 35
<210> 229
   <211> 35
   <212> DNA
   <213> artificial sequence
<220>
   <223> amorce
   <400> 229
   tctcttggag ttacaaatta tttcacttga gtccg 35
<210> 230
   <211> 35
   <212> DNA
   <213> artificial sequence
<220>
   <223> amorce
   <400> 230
   tctcttggag ttacaactga tttcacttga gtccg 35
<210> 231
   <211> 35
   <212> DNA
   <213> artificial sequence
<220>
   <223> amorce
   <400> 231
   tctcttggag ttacaaccga tttcacttga gtccg 35
<210> 232
   <211> 35
   <212> DNA
   <213> artificial sequence

<220>
   <223> amorce
   <400> 232
   tctcttggag ttacaagtga tttcacttga gtccg 35
<210> 233
   <211> 35
   <212> DNA
   <213> artificial sequence
<220>
   <223> amorce
   <400> 233
   tctcttggag ttacaatgga tttcacttga gtccg 35
<210> 234
   <211> 35
   <212> DNA
   <213> artificial sequence
<220>
   <223> amorce
   <400> 234
   tctcttggag ttacaaatga tttcacttga gtccg 35
<210> 235
   <211> 35
   <212> DNA
   <213> artificial sequence
<220>
   <223> amorce
   <400> 235
   acttgagtcc ggagatgcga gtattcatga tacag 35
<210> 236
   <211> 35
   <212> DNA
   <213> artificial sequence
<220>
   <223> amorce
   <400> 236
   acttgagtcc ggagatttca gtattcatga tacag 35
<210> 237
   <211> 35
   <212> DNA
   <213> artificial sequence
<220>
   <223> amorce
   <400> 237
   acttgagtcc ggagatatta gtattcatga tacag 35
<210> 238
   <211> 35
   <212> DNA
   <213> artificial sequence
<220>
   <223> amorce
   <400> 238
   acttgagtcc ggagatctga gtattcatga tacag 35
<210> 239
   <211> 35
   <212> DNA
   <213> artificial sequence
<220>
   <223> amorce
   <400> 239
   acttgagtcc ggagatccga gtattcatga tacag 35
<210> 240
   <211> 35
   <212> DNA
   <213> artificial sequence
<220>
   <223> amorce
   <400> 240
   acttgagtcc ggagatgtga gtattcatga tacag 35
<210> 241
   <211> 35
   <212> DNA
   <213> artificial sequence
<220>
   <223> amorce
   <400> 241
   acttgagtcc ggagattgga gtattcatga tacag 35
<210> 242
   <211> 35
   <212> DNA
   <213> artificial sequence
<220>
   <223> amorce
   <400> 242
   acttgagtcc ggagatatga gtattcatga tacag 35
<210> 243
   <211> 35
   <212> DNA
   <213> artificial sequence
<220>
   <223> amorce
   <400> 243
   tattcatgat acagtagcga atctgatcat cctag 35
<210> 244
   <211> 35
   <212> DNA
   <213> artificial sequence
<220>
   <223> amorce
   <400> 244
   tattcatgat acagtattca atctgatcat cctag 35
<210> 245
   <211> 35
   <212> DNA
   <213> artificial sequence
<220>
   <223> amorce
   <400> 245
   tattcatgat acagtaatta atctgatcat cctag 35
<210> 246
   <211> 35
   <212> DNA
   <213> artificial sequence
<220>
   <223> amorce
   <400> 246
   tattcatgat acagtactga atctgatcat cctag 35
<210> 247
   <211> 35
   <212> DNA
   <213> artificial sequence
<220>
   <223> amorce
   <400> 247
   tattcatgat acagtaccga atctgatcat cctag 35
<210> 248
   <211> 35
   <212> DNA
   <213> artificial sequence
<220>
   <223> amorce
   <400> 248
   tattcatgat acagtagtga atctgatcat cctag 35
<210> 249
   <211> 35
   <212> DNA
   <213> artificial sequence
<220>
   <223> amorce
   <400> 249
   tattcatgat acagtatgga atctgatcat cctag 35
<210> 250
   <211> 35
   <212> DNA
   <213> artificial sequence
<220>
   <223> amorce
   <400> 250
   tattcatgat acagtaatga atctgatcat cctag 35
<210> 251
   <211> 35
   <212> DNA
   <213> artificial sequence

<220>
   <223> amorce
   <400> 251
   gatcatccta gcaaacgcga gtttgtcttc taatg 35
<210> 252
   <211> 35
   <212> DNA
   <213> artificial sequence
<220>
   <223> amorce
   <400> 252
   gatcatccta gcaaacttca gtttgtcttc taatg 35
<210> 253
   <211> 35
   <212> DNA
   <213> artificial sequence
<220>
   <223> amorce
   <400> 253
   gatcatccta gcaaacatta gtttgtcttc taatg 35
<210> 254
   <211> 35
   <212> DNA
   <213> artificial sequence
<220>
   <223> amorce
   <400> 254
   gatcatccta gcaaacctga gtttgtcttc taatg 35
<210> 255
   <211> 35
   <212> DNA
   <213> artificial sequence
<220>
   <223> amorce
   <400> 255
   gatcatccta gcaaacccga gtttgtcttc taatg 35
<210> 256
   <211> 35
   <212> DNA
   <213> artificial sequence
<220>
   <223> amorce
   <400> 256
   gatcatccta gcaaacgtga gtttgtcttc taatg 35
<210> 257
   <211> 35
   <212> DNA
   <213> artificial sequence
<220>
   <223> amorce
   <400> 257
   gatcatccta gcaaactgga gtttgtcttc taatg 35
<210> 258
   <211> 35
   <212> DNA
   <213> artificial sequence
<220>
   <223> amorce
   <400> 258
   gatcatccta gcaaacatga gtttgtcttc taatg 35
<210> 259
   <211> 35
   <212> DNA
   <213> artificial sequence
<220>
   <223> amorce
   <400> 259
   tttgtcttct aatggggcgg taacagaatc tggat 35
<210> 260
   <211> 35
   <212> DNA
   <213> artificial sequence
<220>
   <223> amorce
   <400> 260
   tttgtcttct aatgggttcg taacagaatc tggat 35
<210> 261
   <211> 35
   <212> DNA
   <213> artificial sequence
<220>
   <223> amorce
   <400> 261
   tttgtcttct aatgggattg taacagaatc tggat 35
<210> 262
   <211> 35
   <212> DNA
   <213> artificial sequence
<220>
   <223> amorce
   <400> 262
   tttgtcttct aatgggctgg taacagaatc tggat 35
<210> 263
   <211> 35
   <212> DNA
   <213> artificial sequence
<220>
   <223> amorce
   <400> 263
   tttgtcttct aatgggccgg taacagaatc tggat 35
<210> 264
   <211> 35
   <212> DNA
   <213> artificial sequence
<220>
   <223> amorce
   <400> 264
   tttgtcttct aatggggtgg taacagaatc tggat 35
<210> 265
   <211> 35
   <212> DNA
   <213> artificial sequence
<220>
   <223> amorce
   <400> 265
   tttgtcttct aatgggtggg taacagaatc tggat 35
<210> 266
   <211> 35
   <212> DNA
   <213> artificial sequence
<220>
   <223> amorce
   <400> 266
   tttgtcttct aatgggatgg taacagaatc tggat 35
<210> 267
   <211> 35
   <212> DNA
   <213> artificial sequence
<220>
   <223> amorce
   <400> 267
   agaatctgga tgcaaagcgt gtgaggaact ggagg 35
<210> 268
   <211> 35
   <212> DNA
   <213> artificial sequence
<220>
   <223> amorce
   <400> 268
   agaatctgga tgcaaattct gtgaggaact ggagg 35
<210> 269
   <211> 35
   <212> DNA
   <213> artificial sequence
<220>
   <223> amorce
   <400> 269
   agaatctgga tgcaaaattt gtgaggaact ggagg 35
<210> 270
   <211> 35
   <212> DNA
   <213> artificial sequence
<220>
   <223> amorce
   <400> 270
   agaatctgga tgcaaactgt gtgaggaact ggagg 35
<210> 271
   <211> 35
   <212> DNA
   <213> artificial sequence
<220>
   <223> amorce
   <400> 271
   agaatctgga tgcaaaccgt gtgaggaact ggagg 35
<210> 272
   <211> 35
   <212> DNA
   <213> artificial sequence
<220>
   <223> amorce
   <400> 272
   agaatctgga tgcaaagtgt gtgaggaact ggagg 35
<210> 273
   <211> 35
   <212> DNA
   <213> artificial sequence
<220>
   <223> amorce
   <400> 273
   agaatctgga tgcaaatggt gtgaggaact ggagg 35
<210> 274
   <211> 35
   <212> DNA
   <213> artificial sequence
<220>
   <223> amorce
   <400> 274
   agaatctgga tgcaaaatgt gtgaggaact ggagg 35
<210> 275
   <211> 35
   <212> DNA
   <213> artificial sequence
<220>
   <223> amorce
   <400> 275
   atgtgaggaa ctggaggcga aaaatattaa agaat 35
<210> 276
   <211> 35
   <212> DNA
   <213> artificial sequence
<220>
   <223> amorce
   <400> 276
   atgtgaggaa ctggagttca aaaatattaa agaat 35
<210> 277
   <211> 35
   <212> DNA
   <213> artificial sequence
<220>
   <223> amorce
   <400> 277
   atgtgaggaa ctggagatta aaaatattaa agaat 35
<210> 278
   <211> 35
   <212> DNA
   <213> artificial sequence

<220>
   <223> amorce
   <400> 278
   atgtgaggaa ctggagctga aaaatattaa agaat 35
<210> 279
   <211> 35
   <212> DNA
   <213> artificial sequence
<220>
   <223> amorce
   <400> 279
   atgtgaggaa ctggagccga aaaatattaa agaat 35
<210> 280
   <211> 35
   <212> DNA
   <213> artificial sequence
<220>
   <223> amorce
   <400> 280
   atgtgaggaa ctggaggtga aaaatattaa agaat 35
<210> 281
   <211> 35
   <212> DNA
   <213> artificial sequence
<220>
   <223> amorce
   <400> 281
   atgtgaggaa ctggagtgga aaaatattaa agaat 35
<210> 282
   <211> 35
   <212> DNA
   <213> artificial sequence
<220>
   <223> amorce
   <400> 282
   atgtgaggaa ctggagatga aaaatattaa agaat 35
<210> 283
   <211> 35
   <212> DNA
   <213> artificial sequence
<220>
   <223> amorce
   <400> 283
   tattaaagaa tttttggcga gttttgtaca tattg 35
<210> 284
   <211> 35
   <212> DNA
   <213> artificial sequence
<220>
   <223> amorce
   <400> 284
   tattaaagaa tttttgttca gttttgtaca tattg 35
<210> 285
   <211> 35
   <212> DNA
   <213> artificial sequence
<220>
   <223> amorce
   <400> 285
   tattaaagaa tttttgatta gttttgtaca tattg 35
<210> 286
   <211> 35
   <212> DNA
   <213> artificial sequence
<220>
   <223> amorce
   <400> 286
   tattaaagaa tttttgctga gttttgtaca tattg 35
<210> 287
   <211> 35
   <212> DNA
   <213> artificial sequence
<220>
   <223> amorce
   <400> 287
   tattaaagaa tttttgccga gttttgtaca tattg 35
<210> 288
   <211> 35
   <212> DNA
   <213> artificial sequence
<220>
   <223> amorce
   <400> 288
   tattaaagaa tttttggtga gttttgtaca tattg 35
<210> 289
   <211> 35
   <212> DNA
   <213> artificial sequence
<220>
   <223> amorce
   <400> 289
   tattaaagaa tttttgtgga gttttgtaca tattg 35
<210> 290
   <211> 35
   <212> DNA
   <213> artificial sequence
<220>
   <223> amorce
   <400> 290
   tattaaagaa tttttgatga gttttgtaca tattg 35
<210> 291
   <211> 35
   <212> DNA
   <213> artificial sequence
<220>
   <223> amorce
   <400> 291
   ttttgtacat attgtcgcga tgttcatcaa cactt 35
<210> 292
   <211> 35
   <212> DNA
   <213> artificial sequence
<220>
   <223> amorce
   <400> 292
   ttttgtacat attgtcttca tgttcatcaa cactt 35
<210> 293
   <211> 35
   <212> DNA
   <213> artificial sequence
<220>
   <223> amorce
   <400> 293
   ttttgtacat attgtcatta tgttcatcaa cactt 35
<210> 294
   <211> 35
   <212> DNA
   <213> artificial sequence
<220>
   <223> amorce
   <400> 294
   ttttgtacat attgtcctga tgttcatcaa cactt 35
<210> 295
   <211> 35
   <212> DNA
   <213> artificial sequence
<220>
   <223> amorce
   <400> 295
   ttttgtacat attgtcccga tgttcatcaa cactt 35
<210> 296
   <211> 35
   <212> DNA
   <213> artificial sequence
<220>
   <223> amorce
   <400> 296
   ttttgtacat attgtcgtga tgttcatcaa cactt 35
<210> 297
   <211> 35
   <212> DNA
   <213> artificial sequence
<220>
   <223> amorce
   <400> 297
   ttttgtacat attgtctgga tgttcatcaa cactt 35
<210> 298
   <211> 35
   <212> DNA
   <213> artificial sequence
<220>
   <223> amorce
   <400> 298
   ttttgtacat attgtcatga tgttcatcaa cactt 35
<210> 299
   <211> 767
   <212> DNA
   <213> artificial sequence
<220>
   <223> Clone 1
   <400> 299
   aggagggcca ccatgcgaat ttcgaaacca catttgagaa gtatttccat ccagtgctac     60
   ttgtgtttac ttctaaacag tcattttcta actgaagctg gcattcatgt cttcattttg    120
   ggctgtttca gtgcagggct tcctaaaaca gaagccaact gggtgaatgt aataagtgat    180
   ttgaaaaaaa ttgaagatct tattcaatct atgcatattg atgctacttt atatacggaa    240
   agtgatgttc accccagttg caaagtaaca gcaatgaagt gctttctctt ggagttacaa    300
   gttatttcac ttgagtccgg agatgcaagt attcatgata cagtagaaaa tctgatcatc    360
   ctagcaaaca acagtttgtc ttctaatggg aatgtaacag aatctggatg caaagaatgt    420
   gaggaactgg aggaaaaaaa tattaaagaa tttttgcaga gttttttcca tattgtccaa    480
   atgttcatca acacttcttg attgcaattg agctagcatt atccctaata cctgccaccc    540
   cactcttaat cagtggtgga agaacggtct cagaactgtt tgtttcaatt ggccatttaa    600
   gtttagtagt aaaagactgg ttaatgataa caatgcatcg taaaagcttt cagaaggaaa    660
   ggagaatgtt ttgtgatcta ctttggtttt cttttttgcg tgtggcagtt ttaagttatt    720
   agtttttaaa atcagtactt tttaatggaa acaacttgac caaaaat                  767
<210> 300
   <211> 767
   <212> DNA
   <213> artificial sequence
<220>
   <223> clone 2
   <400> 300
   aggagggcca ccatgcgaat ttcgaaacca catttgagaa gtatttccat ccagtgctac     60
   ttgtgtttac ttctaaacag tcattttcta actgaagctg gcattcatgt cttcattttg    120
   ggctgtttca gtgcagggct tcctaaaaca gaagccaact gggtgaatgt aataagtgat    180
   ttgaaaaaaa ttgaagcgct tattcaatct atgcatattg atgctacttt atatacggaa    240
   agtgatgttc accccagttg caaagtaaca gcaatgaagt gctttctctt ggagttacaa    300
   gttatttcac ttgagtccgg agatgcaagt attcatgata cagtagaaaa tctgatcatc    360
   ctagcaaaca acagtttgtc ttctaatggg aatgtaacag aatctggatg caaagaatgt    420
   gaggaactgg aggaaaaaaa tattaaagaa tttttgcaga gttttgtaca tattgtccaa    480
   atgttcatca acacttcttg attgcaattg agctagcatt atccctaata cctgccaccc    540
   cactcttaat cagtggtgga agaacggtct cagaactgtt tgtttcaatt ggccatttaa    600
   gtttagtagt aaaagactgg ttaatgataa caatgcatcg taaaagcttt cagaaggaaa    660
   ggagaatgtt ttgtgatcta ctttggtttt cttttttgcg tgtggcagtt ttaagttatt     720
   agtttttaaa atcagtactt tttaatggaa acaacttgac caaaaat                  767
<210> 301
   <211> 767
   <212> DNA
   <213> artificial sequence
<220>
   <223> clone 3
   <400> 301
   aggagggcca ccatgcgaat ttcgaaacca catttgagaa gtatttccat ccagtgctac     60
   ttgtgtttac ttctaaacag tcattttcta actgaagctg gcattcatgt cttcattttg    120
   ggctgtttca gtgcagggct tcctaaaaca gaagccaact gggtgaatgt aataagtgat    180
   ttgaaaaaaa ttgaagatct tattcaatct atgcatattg atgctacttt atatacggaa    240
   agtgatgttc accccagttg caaagtaaca gcaatgaagt gctttctctt ggagttacaa    300
   gttatttcac ttgagtccgg agatgcaagt attcatgata cagtagaaaa tctgatcatc    360
   ctagcaaaca acagtttgtc ttctaatggg aatgtaacag aatctggatg caaagaatgt    420
   gaggaactgg aggaaaaaaa tattaaagaa tttttgcaga gttttccgca tattgtccaa    480
   atgttcatca acacttcttg attgcaattg agctagcatt atccctaata cctgccaccc    540
   cactcttaat cagtggtgga agaacggtct cagaactgtt tgtttcaatt ggccatttaa    600
   gtttagtagt aaaagactgg ttaatgataa caatgcatcg taaaagcttt cagaaggaaa    660
   ggagaatgtt ttgtgatcta ctttggtttt cttttttgcg tgtggcagtt ttaagttatt     720
   agtttttaaa atcagtactt tttaatggaa acaacttgac caaaaat                  767
<210> 302
   <211> 767
   <212> DNA
   <213> artificial sequence
<220>
   <223> clone 4
   <400> 302
   aggagggcca ccatgcgaat ttcgaaacca catttgagaa gtatttccat ccagtgctac     60
   ttgtgtttac ttctaaacag tcattttcta actgaagctg gcattcatgt cttcattttg    120
   ggctgtttca gtgcagggct tcctaaaaca gaagccaact gggtgaatgt aataagtgat    180
   ttgaaaaaaa ttgaagatct tattcaatct atgcatattg atgctacttt atatacggaa    240
   agtgatgttc accccagttg caaagtaaca gcaatgaagt gctttctctt ggagttacaa    300
   gttatttcac ttgagtccgg agatgcaagt attcatgata cagtagaaaa tctgatcatc    360
   ctagcaaaca acagtttgtc ttctaatggg aatgtaacag aatctggatg caaagaatgt    420
   gaggaactgg aggaaaaaaa tattaaagaa tttttgcaga gttttgtacc gattgtccaa    480
   atgttcatca acacttcttg attgcaattg agctagcatt atccctaata cctgccaccc    540
   cactcttaat cagtggtgga agaacggtct cagaactgtt tgtttcaatt ggccatttaa    600
   gtttagtagt aaaagactgg ttaatgataa caatgcatcg taaaagcttt cagaaggaaa    660
   ggagaatgtt ttgtgatcta ctttggtttt cttttttgcg tgtggcagtt ttaagttatt    720
   agtttttaaa atcagtactt tttaatggaa acaacttgac caaaaat                 767
<210> 303
   <211> 767
   <212> DNA
   <213> artificial sequence
<220>
   <223> clone 5
   <400> 303
   aggagggcca ccatgcgaat ttcgaaacca catttgagaa gtatttccat ccagtgctac     60
   ttgtgtttac ttctaaacag tcattttcta actgaagctg gcattcatgt cttcattttg    120
   ggctgtttca gtgcagggct tcctaaaaca gaagccaact gggtgaatgt aataagtgat    180
   ttgaaaaaaa ttgaagatct tattcaatct atgcatattg atgctacttt atatacggaa    240
   agtgatgttc accccagttg caaagtaaca gcaatgaagt gctttctctt ggagttacaa    300
   gttatttcac ttgagtccgg agatgcaagt attcatgata cagtagaaaa tctgatcatc    360
   ctagcaaaca acagtttgtc ttctaatggg aatgtaacag aatctggatg caaagaatgt    420
   gaggaactgg aggaaaaaaa tattaaagaa tttttgcaga gttttgtacc gattgtccaa    480
   atgttcatca acacttcttg attgcaattg agctagcatt atccctaata cctgccaccc    540
   cactcttaat cagtggtgga agaacggtct cagaactgtt tgtttcaatt ggccatttaa    600
   gtttagtagt aaaagactgg ttaatgataa caatgcatcg taaaagcttt cagaaggaaa    660
   ggagaatgtt ttgtgatcta ctttggtttt cttttttgcg tgtggcagtt ttaagttatt    720
   agtttttaaa atcagtactt tttaatggaa acaacttgac caaaaat                  767
<210> 304
   <211> 767
   <212> DNA
   <213> artificial sequence
<220>
   <223> clone 6
   <400> 304
   aggagggcca ccatgcgaat ttcgaaacca catttgagaa gtatttccat ccagtgctac     60
   ttgtgtttac ttctaaacag tcattttcta actgaagctg gcattcatgt cttcattttg    120
   ggctgtttca gtgcagggct tcctaaaaca gaagccaact gggtgaatgt aataagtgat    180
   ttgaaaaaaa ttgaagatct tattcaatct atgcatattg atgctacttt atatacggaa    240
   agtgatgttt tccccagttg caaagtaaca gcaatgaagt gctttctctt ggagttacaa    300
   gttatttcac ttgagtccgg agatgcaagt attcatgata cagtagaaaa tctgatcatc    360
   ctagcaaaca acagtttgtc ttctaatggg aatgtaacat ggtctggatg caaagaatgt    420
   gaggaactgg aggaaaaaaa tattaaagaa tttttgcaga gttttgtacc gattgtccaa    480
   atgttcatca acacttcttg attgcaattg agctagcatt atccctaata cctgccaccc    540
   cactcttaat cagtggtgga agaacggtct cagaactgtt tgtttcaatt ggccatttaa    600
   gtttagtagt aaaagactgg ttaatgataa caatgcatcg taaaagcttt cagaaggaaa    660
   ggagaatgtt ttgtgatcta ctttggtttt cttttttgcg tgtggcagtt ttaagttatt    720
   agtttttaaa atcagtactt tttaatggaa acaacttgac caaaaat                  767
<210> 305
   <211> 767
   <212> DNA
   <213> artificial sequence
<220>
   <223> clone 7
   <400> 305
   aggagggcca ccatgcgaat ttcgaaacca catttgagaa gtatttccat ccagtgctac 60
   ttgtgtttac ttctaaacag tcattttcta actgaagctg gcattcatgt cttcattttg 120
   ggctgtttca gtgcagggct tcctaaaaca gaagccaact gggtgaatgt aataagtgat 180
   ttgaaaaaaa ttgaagatct tattcaatct atgcatattg atgctacttt atatacggaa 240
   agtgatgttc accccagttg caaagtaaca gcaatgaagt gctttctctt ggagttacaa 300
   gttatttcac ttgagtccgg agatgcaagt attcatgata cagtagaaaa tctgatcatc 360
   ctagcaaaca acagtttgtc ttctaatggg aatgtaacag aatctggatg caaagaatgt 420
   gaggaactgg aggaaaaaaa tattaaagaa tttttgcaga gttttgtaca tattgtccaa 480
   atgttcatca acacttcttg attgcaattg agctagcatt atccctaata cctgccaccc 540
   cactcttaat cagtggtgga agaacggtct cagaactgtt tgtttcaatt ggccatttaa 600
   gtttagtagt aaaagactgg ttaatgataa caatgcatcg taaaagcttt cagaaggaaa 660
   ggagaatgtt ttgtgatcta ctttggtttt cttttttgcg tgtggcagtt ttaagttatt 720
   agtttttaaa atcagtactt tttaatggaa acaacttgac caaaaat 767
<210> 306
   <211> 767
   <212> DNA
   <213> artificial sequence
<220>
   <223> clone 8
   <400> 306
   aggagggcca ccatgcgaat ttcgaaacca catttgagaa gtatttccat ccagtgctac 60
   ttgtgtttac ttctaaacag tcattttcta actgaagctg gcattcatgt cttcattttg 120
   ggctgtttca gtgcagggct tcctaaaaca gaagccaact gggtgaatgt aataagtgat 180
   ttgaaaaaaa ttgaagatct tattcaatct atgcatattg atgctacttt atatacggaa 240
   agtgatgttc accccagttg caaagtaaca gcaatgaagt gctttctctt ggagttacaa 300
   gttatttcac ttgagtccgg agatgcaagt attcatgata cagtagaaaa tctgatcatc 360
   ctagcaaaca acagtttgtc ttctaatggg aatgtaacag aatctggatg caaagaatgt 420
   gaggaactgg aggaaaaaaa tattaaagaa tttttgcaga gttttgtaca tattgtccaa 480
   atgttcatca acacttcttg attgcaattg agctagcatt atccctaata cctgccaccc 540
   cactcttaat cagtggtgga agaacggtct cagaactgtt tgtttcaatt ggccatttaa 600
   gtttagtagt aaaagactgg ttaatgataa caatgcatcg taaaagcttt cagaaggaaa 660
   ggagaatgtt ttgtgatcta ctttggtttt cttttttgcg tgtggcagtt ttaagttatt 720
   agtttttaaa atcagtactt tttaatggaa acaacttgac caaaaat 767
<210> 307
   <211> 767
   <212> DNA
   <213> artificial sequence
<220>
   <223> clone 9
   <400> 307
   aggagggcca ccatgcgaat ttcgaaacca catttgagaa gtatttccat ccagtgctac 60
   ttgtgtttac ttctaaacag tcattttcta actgaagctg gcattcatgt cttcattttg 120
   ggctgtttca gtgcagggct tcctaaaaca gaagccaact gggtgaatgt aataagtgat 180
   ttgaaaaaaa ttgaagatct tattcaatct atgcatattg atgctacttt atatacggaa 240
   agtgatgttc accccagttg caaagtaaca gcaatgaagt gctttctctt ggagttacaa 300
   gttatttcac ttgagtccgg agatgcaagt attcatgata cagtagaaaa tctgatcatc 360
   ctagcaaaca acagtttgtc ttctaatggg aatgtaacag aatctggatg caaagaatgt 420
   gaggaactgg aggaaaaaaa tattaaagaa tttttgcaga gttttgtacc gattgtccaa 480
   atgttcatca acacttcttg attgcaattg agctagcatt atccctaata cctgccaccc 540
   cactcttaat cagtggtgga agaacggtct cagaactgtt tgtttcaatt ggccatttaa 600
   gtttagtagt aaaagactgg ttaatgataa caatgcatcg taaaagcttt cagaaggaaa 660
   ggagaatgtt ttgtgatcta ctttggtttt cttttttgcg tgtggcagtt ttaagttatt 720
   agtttttaaa atcagtactt tttaatggaa acaacttgac caaaaat 767
<210> 308
   <211> 767
   <212> DNA
   <213> artificial sequence
<220>
   <223> clone 10
   <400> 308
   aggagggcca ccatgcgaat ttcgaaacca catttgagaa gtatttccat ccagtgctac 60
   ttgtgtttac ttctaaacag tcattttcta actgaagctg gcattcatgt cttcattttg 120
   ggctgtttca gtgcagggct tcctaaaaca gaagccaact gggtgaatgt aataagtgat 180
   ttgaaaaaaa ttgaactgct tattcaatct atgcatattg atgctacttt atatacggaa 240
   agtgatgttc accccagttg caaagtaaca gcaatgaagt gctttctctt ggagttacaa 300
   gttatttcac ttgagtccgg agatgcaagt attcatgata cagtagaaaa tctgatcatc 360
   ctagcaaaca acagtttgtc ttctaatggg aatgtaacag aatctggatg caaagaatgt 420
   gaggaactgg aggaaaaaaa tattaaagaa tttttgcaga gttttgtaca tattgtccaa 480
   atgttcatca acacttcttg attgcaattg agctagcatt atccctaata cctgccaccc 540
   cactcttaat cagtggtgga agaacggtct cagaactgtt tgtttcaatt ggccatttaa 600
   gtttagtagt aaaagactgg ttaatgataa caatgcatcg taaaagcttt cagaaggaaa 660
   ggagaatgtt ttgtgatcta ctttggtttt cttttttgcg tgtggcagtt ttaagttatt 720
   agtttttaaa atcagtactt tttaatggaa acaacttgac caaaaat 767
<210> 309
   <211> 767
   <212> DNA
   <213> artificial sequence
<220>
   <223> clone 11
   <400> 309
   aggagggcca ccatgcgaat ttcgaaacca catttgagaa gtatttccat ccagtgctac 60
   ttgtgtttac ttctaaacag tcattttcta actgaagctg gcattcatgt cttcattttg 120
   ggctgtttca gtgcagggct tcctaaaaca gaagccaact gggtgaatgt aataagtgat 180
   ttgaaaaaaa ttgaagcgct tattcaatct atgcatattg atgctacttt atatacggaa 240
   agtgatgttc accccagttg caaagtaaca gcaatgaagt gctttctctt ggagttacaa 300
   gttatttcac ttgagtccgg agatgcaagt attcatgata cagtagaaaa tctgatcatc 360
   ctagcaaaca acagtttgtc ttctaatggg aatgtaacag aatctggatg caaagaatgt 420
   gaggaactgg aggaaaaaaa tattaaagaa tttttgcaga gttttgtaca tattgtccaa 480
   atgttcatca acacttcttg attgcaattg agctagcatt atccctaata cctgccaccc 540
   cactcttaat cagtggtgga agaacggtct cagaactgtt tgtttcaatt ggccatttaa 600
   gtttagtagt aaaagactgg ttaatgataa caatgcatcg taaaagcttt cagaaggaaa 660
   ggagaatgtt ttgtgatcta ctttggtttt cttttttgcg tgtggcagtt ttaagttatt 720
   agtttttaaa atcagtactt tttaatggaa acaacttgac caaaaat 767
<210> 310
   <211> 767
   <212> DNA
   <213> artificial sequence
<220>
   <223> clone 12
   <400> 310
   aggagggcca ccatgcgaat ttcgaaacca catttgagaa gtatttccat ccagtgctac     60
   ttgtgtttac ttctaaacag tcattttcta actgaagctg gcattcatgt cttcattttg    120
   ggctgtttca gtgcagggct tcctaaaaca gaagccaact gggtgaatgt aataagtgat    180
   ttgaaaaaaa ttgaagatct tattcaatct atgcatattg atgctacttt atatacggaa    240
   agtgatgttc accccagttg caaagtaaca gcaatgaagt gctttctctt ggagttacaa    300
   gttatttcac ttgagtccgg agatgcaagt attcatgata cagtagaaaa tctgatcatc    360
   ctagcaaaca acagtttgtc ttctaatggg aatgtaacag aatctggatg caaagaatgt    420
   gaggaactgg aggaaaaaaa tattaaagaa tttttgcaga gttttgtaca tattgtccaa    480
   atgttcatca acacttcttg attgcaattg agctagcatt atccctaata cctgccaccc    540
   cactcttaat cagtggtgga agaacggtct cagaactgtt tgtttcaatt ggccatttaa    600
   gtttagtagt aaaagactgg ttaatgataa caatgcatcg taaaagcttt cagaaggaaa    660
   ggagaatgtt ttgtgatcta ctttggtttt cttttttgcg tgtggcagtt ttaagttatt    720
   agtttttaaa atcagtactt tttaatggaa acaacttgac caaaaat                  767
<210> 311
   <211> 767
   <212> DNA
   <213> artificial sequence
<220>
   <223> clone 13
   <400> 311
   aggagggcca ccatgcgaat ttcgaaacca catttgagaa gtatttccat ccagtgctac     60
   ttgtgtttac ttctaaacag tcattttcta actgaagctg gcattcatgt cttcattttg    120
   ggctgtttca gtgcagggct tcctaaaaca gaagccaact gggtgaatgt aataagtgat    180
   ttgaaaaaaa ttgaagatct tattcaatct atgcatattg atgctacttt atatacgatg    240
   agtgatgttc accccagttg caaagtaaca gtgatgaagt gctttctctt ggagttacaa    300
   gttatttcac ttgagtccgg agatgcaagt attcatgata cagtagaaaa tctgatcatc    360
   ctagcaaaca acagtttgtc ttctaatggg aatgtaacag aatctggatg caaagaatgt    420
   gaggaactgg aggaaaaaaa tattaaagaa tttttgcaga gttttgtacc gattgtccaa    480
   atgttcatca acacttcttg attgcaattg agctagcatt atccctaata cctgccaccc    540
   cactcttaat cagtggtgga agaacggtct cagaactgtt tgtttcaatt ggccatttaa    600
   gtttagtagt aaaagactgg ttaatgataa caatgcatcg taaaagcttt cagaaggaaa    660
   ggagaatgtt ttgtgatcta ctttggtttt cttttttgcg tgtggcagtt ttaagttatt     720
   agtttttaaa atcagtactt tttaatggaa acaacttgac caaaaat                  767
<210> 312
   <211> 767
   <212> DNA
   <213> artificial sequence
<220>
   <223> clone 14
   <400> 312
   aggagggcca ccatgcgaat ttcgaaacca catttgagaa gtatttccat ccagtgctac     60
   ttgtgtttac ttctaaacag tcattttcta actgaagctg gcattcatgt cttcattttg    120
   ggctgtttca gtgcagggct tcctaaaaca gaagccaact gggtgaatgt aataagtgat    180
   ttgaaaaaaa ttgaaatgct tattcaatct atgcatattg atgctacttt atatacggaa    240
   agtgatgttc accccagttg caaagtaaca gcaatgaagt gctttctctt ggagttacaa    300
   gttatttcac ttgagtccgg agatgcaagt attcatgata cagtagaaaa tctgatcatc    360
   ctagcaaaca acagtttgtc ttctaatggg aatgtaacag aatctggatg caaagaatgt    420
   gaggaactgg aggaaaaaaa tattaaagaa tttttgcaga gttttgtaca tattgtccaa    480
   atgttcatca acacttcttg attgcaattg agctagcatt atccctaata cctgccaccc    540
   cactcttaat cagtggtgga agaacggtct cagaactgtt tgtttcaatt ggccatttaa    600
   gtttagtagt aaaagactgg ttaatgataa caatgcatcg taaaagcttt cagaaggaaa    660
   ggagaatgtt ttgtgatcta ctttggtttt cttttttgcg tgtggcagtt ttaagttatt     720
   agtttttaaa atcagtactt tttaatggaa acaacttgac caaaaat                  767
<210> 313
   <211> 767
   <212> DNA
   <213> artificial sequence
<220>
   <223> clone 15
   <400> 313
   aggagggcca ccatgcgaat ttcgaaacca catttgagaa gtatttccat ccagtgctac 60
   ttgtgtttac ttctaaacag tcattttcta actgaagctg gcattcatgt cttcattttg 120
   ggctgtttca gtgcagggct tcctaaaaca gaagccaact gggtgaatgt aataagtgat 180
   ttgaaaaaaa ttgaagatct tattcaatct atgcatattg atgctacttt atatacggaa 240
   agtgatgttc accccagttg caaagtaaca gcaatgaagt gctttctctt ggagttacaa 300
   gttatttcac ttgtgtccgg agatgcaagt attattgata cagtagaaaa tctgatcatc 360
   ctagcaaaca acagtttgtc ttctaatggg aatgtaacag aatctggatg caaagaatgt 420
   gaggaactgg aggaaaaaaa tattaaagaa tttttgcaga gttttgtaca tattgtccaa 480
   atgttcatca acacttcttg attgcaattg agctagcatt atccctaata cctgccaccc 540
   cactcttaat cagtggtgga agaacggtct cagaactgtt tgtttcaatt ggccatttaa 600
   gtttagtagt aaaagactgg ttaatgataa caatgcatcg taaaagcttt cagaaggaaa 660
   ggagaatgtt ttgtgatcta ctttggtttt cttttttgcg tgtggcagtt ttaagttatt 720
   agtttttaaa atcagtactt tttaatggaa acaacttgac caaaaat 767
<210> 314
   <211> 767
   <212> DNA
   <213> artificial sequence
<220>
   <223> clone 16
   <400> 314
   aggagggcca ccatgcgaat ttcgaaacca catttgagaa gtatttccat ccagtgctac 60
   ttgtgtttac ttctaaacag tcattttcta actgaagctg gcattcatgt cttcattttg 120
   ggctgtttca gtgcagggct tcctaaaaca gaagccaact gggtgaatgt aataagtgat 180
   ttgaaaaaaa ttgaagatct tattcaatct atgcatattg atgctacttt atatacggaa 240
   agtgatgttc accccagttg caaagtaaca gcaatgaagt gctttctctt ggagttacaa 300
   gttatttcac ttgagtccgg agatgcaagt attcatgata cagtagaaaa tctgatcatc 360
   ctagcaaaca acagtttgtc ttctaatggg aatgtaacag aatctggatg caaagaatgt 420
   gaggaactgg aggaaaaatg gattaaagaa tttttgcaga gttttgtaca tattgtccaa 480
   atgttcatca acacttcttg attgcaattg agctagcatt atccctaata cctgccaccc 540
   cactcttaat cagtggtgga agaacggtct cagaactgtt tgtttcaatt ggccatttaa 600
   gtttagtagt aaaagactgg ttaatgataa caatgcatcg taaaagcttt cagaaggaaa 660
   ggagaatgtt ttgtgatcta ctttggtttt cttttttgcg tgtggcagtt ttaagttatt 720
   agtttttaaa atcagtactt tttaatggaa acaacttgac caaaaat 767
<210> 315
   <211> 767
   <212> DNA
   <213> artificial sequence
<220>
   <223> clone 17
   <400> 315
   aggagggcca ccatgcgaat ttcgaaacca catttgagaa gtatttccat ccagtgctac      60
   ttgtgtttac ttctaaacag tcattttcta actgaagctg gcattcatgt cttcattttg     120
   ggctgtttca gtgcagggct tcctaaaaca gaagccaact gggtgaatgt aataagtgat     180
   ttgaaaaaaa ttgaagatct tattcaatct atgcatattg atgctacttt atatacggaa     240
   agtgatgttc accccagttg caaagtaaca gcaatgaagt gctttctctt ggagttacaa     300
   gttatttcac ttgagtccgg agatgcaagt attcatgata cagtagaaaa tctgatcatc     360
   ctagcaaaca acagtttgtc ttctaatggg aatgtaacag aatctggatg caaagaatgt     420
   gaggaactgg aggaaaaaat tattaaagaa tttttgcaga gttttgtaca tattgtccaa     480
   atgttcatca acacttcttg attgcaattg agctagcatt atccctaata cctgccaccc     540
   cactcttaat cagtggtgga agaacggtct cagaactgtt tgtttcaatt ggccatttaa     600
   gtttagtagt aaaagactgg ttaatgataa caatgcatcg taaaagcttt cagaaggaaa     660
   ggagaatgtt ttgtgatcta ctttggtttt cttttttgcg tgtggcagtt ttaagttatt      720
   agtttttaaa atcagtactt tttaatggaa acaacttgac caaaaat                   767
<210> 316
   <211> 767
   <212> DNA
   <213> artificial sequence
<220>
   <223> clone 18
   <400> 316
   aggagggcca ccatgcgaat ttcgaaacca catttgagaa gtatttccat ccagtgctac      60
   ttgtgtttac ttctaaacag tcattttcta actgaagctg gcattcatgt cttcattttg     120
   ggctgtttca gtgcagggct tcctaaaaca gaagccaact gggtgaatgt aataagtgat 180
   ttgaaaaaaa ttgaagatct tattcaatct atgcatattg atgctacttt atatacggaa 240
   agtgatgttc accccagttg caaagtaaca gcaatgaagt gctttctctt ggagttacaa 300
   gttatttcac ttgagtccgg agatgcaagt attcatgata cagtagaaaa tctgatcatc 360
   ctagcaaaca acagtttgtc ttctaatggg aatgtaacag aatctggatg caaagaatgt 420
   gaggaactgg aggaaaaaat tattaaagaa tttttgcaga gttttgtaca tattgtccaa 480
   atgttcatca acacttcttg attgcaattg agctagcatt atccctaata cctgccaccc 540
   cactcttaat cagtggtgga agaacggtct cagaactgtt tgtttcaatt ggccatttaa 600
   gtttagtagt aaaagactgg ttaatgataa caatgcatcg taaaagcttt cagaaggaaa 660
   ggagaatgtt ttgtgatcta ctttggtttt cttttttgcg tgtggcagtt ttaagttatt 720
   agtttttaaa atcagtactt tttaatggaa acaacttgac caaaaat 767
<210> 317
   <211> 767
   <212> DNA
   <213> artificial sequence
<220>
   <223> clone 19
   <400> 317
   aggagggcca ccatgcgaat ttcgaaacca catttgagaa gtatttccat ccagtgctac 60
   ttgtgtttac ttctaaacag tcattttcta actgaagctg gcattcatgt cttcattttg 120
   ggctgtttca gtgcagggct tcctaaaaca gaagccaact gggtgaatgt aataagtgat 180
   ttgaaaaaaa ttgaagatct tattcaatct atgcatattg atgctacttt atatacggaa 240
   agtgatgttc accccagttg caaagtaaca gcaatgaagt gctttctctt ggagttacaa 300
   gttatttcac ttgagtccgg agatgcaagt attcatgata cagtagaaaa tctgatcatc 360
   ctggcaaaca acagtttgtc ttctaatggg aatgtaacac cgtctggatg caaagaatgt 420
   gaggaactgg aggaaaaact gattaaagaa tttttgcaga gttttgtaca tattgtccaa 480
   atgttcatca acacttcttg attgcaattg agctagcatt atccctaata cctgccaccc 540
   cactcttaat cagtggtgga agaacggtct cagaactgtt tgtttcaatt ggccatttaa 600
   gtttagtagt aaaagactgg ttaatgataa caatgcatcg taaaagcttt cagaaggaaa 660
   ggagaatgtt ttgtgatcta ctttggtttt cttttttgcg tgtggcagtt ttaagttatt 720
   agtttttaaa atcagtactt tttaatggaa acaacttgac caaaaat 767
<210> 318
   <211> 767
   <212> DNA
   <213> artificial sequence
<220>
   <223> clone 20
   <400> 318
   aggagggcca ccatgcgaat ttcgaaacca catttgagaa gtatttccat ccagtgctac      60
   ttgtgtttac ttctaaacag tcattttcta actgaagctg gcattcatgt cttcattttg     120
   ggctgtttca gtgcagggct tcctaaaaca gaagccaact gggtgaatgt aataagtgat     180
   ttgaaaaaaa ttgaactgct tattcaatct atgcatattg atgctacttt atatacggaa     240
   agtgatgttc accccagttg caaagtaaca gcaatgaagt gctttctctt ggagttacaa     300
   gttatttcac ttgagtccgg agatgcaagt attcatgata cagtagaaaa tctgatcatc     360
   ctagcaaaca acagtttgtc ttctaatggg aatgtaacag aatctggatg caaagaatgt     420
   gaggaactgg aggaaaaaaa tattaaagaa tttttgcaga gttttgtaca tattgtccaa     480
   atgttcatca acacttcttg attgcaattg agctagcatt atccctaata cctgccaccc     540
   cactcttaat cagtggtgga agaacggtct cagaactgtt tgtttcaatt ggccatttaa     600
   gtttagtagt aaaagactgg ttaatgataa caatgcatcg taaaagcttt cagaaggaaa     660
   ggagaatgtt ttgtgatcta ctttggtttt cttttttgcg tgtggcagtt ttaagttatt     720
   agtttttaaa atcagtactt tttaatggaa acaacttgac caaaaat                   767
<210> 319
   <211> 767
   <212> DNA
   <213> artificial sequence
<220>
   <223> clone 21
   <400> 319
   aggagggcca ccatgcgaat ttcgaaacca catttgagaa gtatttccat ccagtgctac      60
   ttgtgtttac ttctaaacag tcattttcta actgaagctg gcattcatgt cttcattttg     120
   ggctgtttca gtgcagggct tcctaaaaca gaagccaact gggtgaatgt aataagtgat     180
   ttgaaaaaaa ttgaagtgct tattcaatct atgcatattg atgctacttt atatacggaa     240
   agtgatgttc accccagttg caaagtaaca gcaatgaagt gctttctctt ggagttacaa     300
   gttatttcac ttgagtccgg agatgcaagt attcatgata cagtagaaaa tctgatcatc     360
   ctagcaaaca acagtttgtc ttctaatggg aatgtaacag aatctggatg caaagaatgt     420
   gaggaactgg aggaaaaaaa tattaaagaa tttttgcaga gttttgtaca tattgtccaa     480
   atgttcatca acacttcttg attgcaattg agctagcatt atccctaata cctgccaccc     540
   cactcttaat cagtggtgga agaacggtct cagaactgtt tgtttcaatt ggccatttaa     600
   gtttagtagt aaaagactgg ttaatgataa caatgcatcg taaaagcttt cagaaggaaa     660
   ggagaatgtt ttgtgatcta ctttggtttt cttttttgcg tgtggcagtt ttaagttatt     720
   agtttttaaa atcagtactt tttaatggaa acaacttgac caaaaat                   767
<210> 320
   <211> 767
   <212> DNA
   <213> artificial sequence
<220>
   <223> clone 22
   <400> 320
   aggagggcca ccatgcgaat ttcgaaacca catttgagaa gtatttccat ccagtgctac      60
   ttgtgtttac ttctaaacag tcattttcta actgaatggg gcattcatgt cttcattttg     120
   ggctgtttca gtgcagggct tcctaaaaca gaagccaact gggtgaatgt aataagtgat     180
   ttgaaaaaaa ttgaaattct tattcaatct atgcatattg atgctacttt atatacggaa     240
   agtgatgttc accccagttg caaagtaaca gcaatgaagt gctttctctt ggagttacaa     300
   gttatttcac ttgagtccgg agatgcaagt attcatgata cagtagaaaa tctgatcatc     360
   ctagcaaaca acagtttgtc ttctaatggg aatgtaacag aatctggatg caaagaatgt     420
   gaggaactgg aggaaaaaaa tattaaagaa tttttgcaga gttttgtaca tattgtccaa     480
   atgttcatca acacttcttg attgcaattg agctagcatt atccctaata cctgccaccc     540
   cactcttaat cagtggtgga agaacggtct cagaactgtt tgtttcaatt ggccatttaa     600
   gtttagtagt aaaagactgg ttaatgataa caatgcatcg taaaagcttt cagaaggaaa     660
   ggagaatgtt ttgtgatcta ctttggtttt cttttttgcg tgtggcagtt ttaagttatt     720
   agtttttaaa atcagtactt tttaatggaa acaacttgac caaaaat                   767
<210> 321
   <211> 767
   <212> DNA
   <213> artificial sequence
<220>
   <223> clone 23
   <400> 321
   aggagggcca ccatgcgaat ttcgaaacca catttgagaa gtatttccat ccagtgctac      60
   ttgtgtttac ttctaaacag tcattttcta actgaagctg gcattcatgt cttcattttg 120
   ggctgtttca gtgcagggct tcctaaaaca gaagccaact gggtgaatgt aataagtgat 180
   ttgaaaaaaa ttgaagatct tattcaatct atgcatattg atgctacttt atatacggaa 240
   agtgatgttc accccagttg caaagtaaca gcaatgaagt gctttctctt ggagttacaa 300
   gttatttcac ttgagtccgg agatgcaagt attcatgata cagtagaaaa tctgatcatc 360
   ctagcaaaca acagtttgtc ttctaatggg aatgtaacat ggtctggatg caaagaatgt 420
   gaggaactgg aggaaaaaaa tattaaagaa tttttgcaga gttttgtacc gattgtccaa 480
   atgttcatca acacttcttg attgcaattg agctagcatt atccctaata cctgccaccc 540
   cactcttaat cagtggtgga agaacggtct cagaactgtt tgtttcaatt ggccatttaa 600
   gtttagtagt aaaagactgg ttaatgataa caatgcatcg taaaagcttt cagaaggaaa 660
   ggagaatgtt ttgtgatcta ctttggtttt cttttttgcg tgtggcagtt ttaagttatt 720
   agtttttaaa atcagtactt tttaatggaa acaacttgac caaaaat 767
<210> 322
   <211> 767
   <212> DNA
   <213> artificial sequence
<220>
   <223> clone 24
   <400> 322
   aggagggcca ccatgcgaat ttcgaaacca catttgagaa gtatttccat ccagtgctac 60
   ttgtgtttac ttctaaacag tcattttcta actgaagctg gcattcatgt cttcattttg 120
   ggctgtttca gtgcagggct tcctaaaaca gaagccaact gggtgaatgt aataagtgat 180
   ttgaaaaaaa ttgaagatct tattcaatct atgcatattg atgctacttt atatacggaa 240
   agtgatgttc accccagttg caaagtaaca gcaatgaagt gctttctctt ggagttacaa 300
   gttatttcac ttgagtccgg agatgcaagt attcatgata cagtagaaaa tctgatcatc 360
   ctagcaaaca acagtttgtc ttctaatggg aatgtaacag aatctggatg caaagaatgt 420
   gaggaactgg aggaaaaaaa tattaaagaa tttttgcaga gttttgtaca tattgtccgc 480
   gtgttcatca acacttcttg attgcaattg agctagcatt atccctaata cctgccaccc 540
   cactcttaat cagtggtgga agaacggtct cagaactgtt tgtttcaatt ggccatttaa 600
   gtttagtagt aaaagactgg ttaatgataa caatgcatcg taaaagcttt cagaaggaaa 660
   ggagaatgtt ttgtgatcta ctttggtttt cttttttgcg tgtggcagtt ttaagttatt 720
   agtttttaaa atcagtactt tttaatggaa acaacttgac caaaaat 767
<210> 323
   <211> 767
   <212> DNA
   <213> artificial sequence
<220>
   <223> clone 25
   <400> 323
   aggagggcca ccatgcgaat ttcgaaacca catttgagaa gtatttccat ccagtgctac    60
   ttgtgtttac ttctaaacag tcattttcta actgaagctg gcattcatgt cttcattttg   120
   ggctgtttca gtgcagggct tcctaaaaca gaagccaact gggtgaatgt aataagtgat   180
   ttgaaaaaaa ttgaagatct tattcaatct atgcatattg atgctacttt atatacggaa   240
   agtgatgttc accccagttg caaagtaaca gcaatgaagt gctttctctt ggagttacaa   300
   gttatttcac ttgagtccgg agatgcaagt attattgata cagtagaaaa tctgatcatc   360
   ctagcaaaca acagtttgtc ttctaatggg aatgtaacag aatctggatg caaagaatgt   420
   gaggaactgg aggaaaaaaa tattaaagaa tttttgcaga gttttgtaca tattgtccaa   480
   atgttcatca acacttcttg attgcaattg agctagcatt atccctaata cctgccaccc   540
   cactcttaat cagtggtgga agaacggtct cagaactgtt tgtttcaatt ggccatttaa   600
   gtttagtagt aaaagactgg ttaatgataa caatgcatcg taaaagcttt cagaaggaaa   660
   ggagaatgtt ttgtgatcta ctttggtttt cttttttgcg tgtggcagtt ttaagttatt   720
   agtttttaaa atcagtactt tttaatggaa acaacttgac caaaaat                  767
<210> 324
   <211> 767
   <212> DNA
   <213> artificial sequence
<220>
   <223> clone 26
   <400> 324
   aggagggcca ccatgcgaat ttcgaaacca catttgagaa gtatttccat ccagtgctac    60
   ttgtgtttac ttctaaacag tcattttcta actgaagctg gcattcatgt cttcattttg   120
   ggctgtttca gtgcagggct tcctaaaaca gaagccaact gggtgaatgt aataagtgat   180
   ttgaaaaaaa ttgaagatct tattcaatct atgcatattg atgctacttt atatacggaa   240
   agtgatgttc accccagttg caaagtaaca gcaatgaagt gctttctctt gttcttacaa   300
   gttatttcac ttgagtccgg agatgcaagt attcatgata cagtagaaaa tctgatcatc   360
   ctagcaaaca acagtttgtc ttctaatggg aatgtaacag aatctggatg caaagaatgt   420
   gaggaactgg aggaaaaaaa tattaaagaa tttttgcaga gttttgtaca tattgtccaa 480
   atgttcatca acacttcttg attgcaattg agctagcatt atccctaata cctgccaccc 540
   cactcttaat cagtggtgga agaacggtct cagaactgtt tgtttcaatt ggccatttaa 600
   gtttagtagt aaaagactgg ttaatgataa caatgcatcg taaaagcttt cagaaggaaa 660
   ggagaatgtt ttgtgatcta ctttggtttt cttttttgcg tgtggcagtt ttaagttatt 720
   agtttttaaa atcagtactt tttaatggaa acaacttgac caaaaat 767
<210> 325
   <211> 767
   <212> DNA
   <213> artificial sequence
<220>
   <223> clone 27
   <400> 325
   aggagggcca ccatgcgaat ttcgaaacca catttgagaa gtatttccat ccagtgctac 60
   ttgtgtttac ttctaaacag tcattttcta actgaagctg gcattcatgt cttcattttg 120
   ggctgtttca gtgcagggct tcctaaaaca gaagccaact gggtgaatgt aataagtgat 180
   ttgaaaaaaa ttgaagatct tattcaatct atgcatattg atgctacttt atatacggaa 240
   agtgatgttc accccagttg caaagtaaca gcaatgaagt gctttctctt ggagttacaa 300
   gttatttcac ttgagtccgg agatgcaagt attcatgata cagtagaaaa tctgatcatc 360
   ctggcaaaca acagtttgtc ttctaatggg aatgtaacag aatctggatg caaagaatgt 420
   gaggaactgg aggaaaaaaa tattaaagaa tttttgcaga gttttgtaca tattgtccaa 480
   atgttcatca acacttcttg attgcaattg agctagcatt atccctaata cctgccaccc 540
   cactcttaat cagtggtgga agaacggtct cagaactgtt tgtttcaatt ggccatttaa 600
   gtttagtagt aaaagactgg ttaatgataa caatgcatcg taaaagcttt cagaaggaaa 660
   ggagaatgtt ttgtgatcta ctttggtttt cttttttgcg tgtggcagtt ttaagttatt 720
   agtttttaaa atcagtactt tttaatggaa acaacttgac caaaaat 767